(19) Europäisches Patentamt
European Patent Office
Office européen des brevets

(11) EP 1 877 098 B1

(12) **EUROPEAN PATENT SPECIFICATION**

(45) Date of publication and mention of the grant of the patent:
**24.04.2013 Bulletin 2013/17**

(21) Application number: **06727008.2**

(22) Date of filing: **04.05.2006**

(51) Int Cl.:
***A61K 47/48*** (2006.01)

(86) International application number:
**PCT/GB2006/001635**

(87) International publication number:
**WO 2006/117567 (09.11.2006 Gazette 2006/45)**

(54) **ALPHA AMINOACID ESTER-DRUG CONJUGATES HYDROLYSABLE BY CARBOXYLESTERASE**

DURCH CARBOXYLESTERASE HYDROLYSIERBARE ALPHA-AMINOSÄUREESTER-ARZNEIKONJUGATE

CONJUGUÉS D'UN ESTER D'AMINOACIDE ALPHA ET D'UN MÉDICAMENT HYDROLYSABLES PAR LA CARBOXYLESTÉRASE

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.05.2005 GB 0509226**
**13.05.2005 US 680542 P**

(43) Date of publication of application:
**16.01.2008 Bulletin 2008/03**

(60) Divisional application:
**08075300.7 / 1 964 577**
**08075301.5 / 1 964 578**

(73) Proprietor: **Chroma Therapeutics Limited**
**Abingdon,**
**Oxfordshire OX14 4RY (GB)**

(72) Inventors:
- **DAVIDSON, Alan Hornsby**
**Abingdon,**
**Oxfordshire OX14 4RY (GB)**
- **DRUMMOND, Alan Hastings**
**Abingdon,**
**Oxfordshire OX14 4RY (GB)**
- **NEEDHAM, Lindsey Ann**
**Abingdon,**
**Oxfordshire OX14 4RY (GB)**

(74) Representative: **Simons, Amanda Louise et al**
**J A Kemp**
**14 South Square**
**Gray's Inn**
**London WC1R 5JJ (GB)**

(56) References cited:
**WO-A-01/18171**
**WO-A-2005/097747**
**WO-A-2006/117548**
**WO-A-2006/117549**
**WO-A1-2005/037272**
**US-A1- 2004 092 558**

- **LEE W A ET AL: "SELECTIVE INTRACELLULAR ACTIVATION OF A NOVEL PRODRUG OF THE HUMAN IMMUNODEFICIENCY VIRUS REVERSE TRANSCRIPTASE INHIBITOR TENOFOVIR LEADS TO PREFERENTIAL DISTRIBUTION AND ACCUMULATION IN LYMPHATIC TISSUE" ANTIMICROBIAL AGENTS AND CHEMOTHERAPY, AMERICAN SOCIETY FOR MICROBIOLOGY, WASHINGTON, DC, US, vol. 49, no. 5, May 2005 (2005-05), pages 1898-1906, XP008065032 ISSN: 0066-4804**
- **CAHARD D ET AL: "Aryloxy phosphoramidate triesters as pro-tides" MINI-REVIEWS IN MEDICINAL CHEMISTRY 2004 NETHERLANDS, vol. 4, no. 4, 2004, pages 371-381, XP008076430 ISSN: 1389-5575**
- **WITTICH S. ET AL: JOURNAL OF MEDICINAL CHEMISTRY, vol. 45, 2002, pages 3296-3309,**
- **JUNG M. ET AL: BIOORGANIC AND MEDICINAL CHEMISTRY LETTERS, vol. 7, no. 13, 1997, pages 1655-1658, XP004136274,**
- **DUTTA A.S. ET AL: JOURNAL OF MEDICINAL CHEMISTRY, vol. 33, 1990, pages 2560-2568,**
- **JENSEN K.J. ET AL: JOURNAL OF THE AMERICAN CHEMICALY SOCIETY, vol. 120, 1998, pages 5441-5452, XP001084160,**
- **BLANCHARD F. ET AL: DRUG DISCOVERY TODAY, vol. 10, no. 3, February 2005 (2005-02), pages 197-204,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

This invention relates to increasing or prolonging the activity of a compound which inhibits the activity of the target intracellular enzyme histone deacetylase by the covalent conjugation of an alpha amino acid ester motif to the modulator, more specifically to inhibitors to which an alpha amino acid ester motif has been covalently conjugated, for use in a method of treatment of the human or animal body by therapy. The invention also relates to a method for the identification of such conjugates having superior properties relative to the parent non-conjugated inhibitor. The invention further relates to the use of modulators containing amino acid ester motifs that allow the selective accumulation of amino acid conjugates inside cells of the monocyte-macrophage lineage.

## Background to the invention

Many intracellular enzymes and receptors are targets for pharmaceutically useful drugs which modulate their activities by binding to their active sites. Examples appear in Table 1 below. To reach the target enzymes and receptors, modulator compounds must of course cross the cell membrane from plasma/extracellular fluid. In general, charge neutral modulators cross the cell membrane more easily than charged species. A dynamic equilibrium is then set up whereby the modulator equilibrates between plasma and cell interior. As a result of the equilibrium, the intracellular residence times and concentrations of many modulators of intracellular enzymes and receptors are often very low, especially in cases where the modulator is rapidly cleared from the plasma. The potencies of the modulators are therefore poor despite their high binding affinities for the target enzyme or receptor.

It would therefore be desirable if a method were available for increasing the intracellular concentration of a given modulator of an intracellular enzyme or receptor. This would result in increased potency, and by prolonging the residency of the modulator inside the cell would result in improved pharmacokinetic and pharmacodynamic properties. More consistent exposure and reduced dosing frequencies would be achieved. A further benefit could be obtained if the drug could be targeted to the specific target cells responsible for its therapeutic action, reducing systemic exposure and hence side effects.

## Brief description of the invention

This invention describes such a method, and describes improved modulators incorporating the structural principles on which the method is based. It takes advantage of the fact that lipophilic (low polarity or charge neutral) molecules pass through the cell membrane and enter cells relatively easily, and hydrophilic (higher polarity, charged) molecules do not. Hence, if a lipophilic motif is attached to a given modulator, allowing the modulator to enter the cell, and if that motif is converted in the cell to one of higher polarity, it is to be expected that the modulator with the higher polarity motif attached would accumulate within the cell. Providing such a motif is attached to the modulator in a way which does not alter its binding mode with the target enzyme or receptor, the accumulation of modulator with the higher polarity motif attached is therefore expected to result in prolonged and/or increased activity. The present invention is related to modulators which are inhibitors of histone deacetylase.

The present invention makes use of the fact that there are carboxylesterase enzymes within cells, which may be utilised to hydrolyse an alpha amino acid ester motif attached to a given modulator to the parent acid. Therefore, a modulator may be administered as a covalent conjugate with an alpha amino acid ester, in which form it readily enters the cell where it is hydrolysed efficiently by one or more intracellular carboxylesterases, and the resultant alpha amino acid-modulator conjugate accumulates within the cell, increasing overall potency and/or active residence time. It has also been found that by modification of the alpha amino acid motif or the way in which it is conjugated, modulators can be targeted to monocytes and macrophages. Herein, unless "monocyte" or "monocytes" is specified, the term macrophage or macrophages will be used to denote macrophages (including tumour associated macrophages) and/or monocytes.

## Detailed description of the invention

Hence in one broad aspect the present invention provides a covalent conjugate of an alpha amino acid ester and an inhibitor of the activity of the target intracellular enzyme histone deacetylase for use in a method of treatment of the human or animal body by therapy, wherein: the ester group of the conjugate is hydrolysable by one or more intracellular carboxylesterase enzymes to the corresponding acid; the nitrogen of the amino group of the amino acid ester is not linked directly to a carbonyl moiety, or is left unsubstituted; and the alpha amino acid ester is covalently attached to the inhibitor at a position remote from the binding interface between the inhibitor and the histone deacetylase enzyme, and/or is conjugated to the inhibitor such that the binding mode of the conjugated inhibitor and the said corresponding acid to the histone deacetylase enzyme is the same as that of the unconjugated inhibitor; wherein the position of conjugation is remote/the binding mode is the same when the conjugate has a potency in a cellular activity assay at least as high

as that of the unconjugated inhibitor in the same assay, which cellular activity assay is a cell proliferation inhibition assay carried out in U937 cancer cells.

As stated, the invention is concerned with modification of inhibitors of histone deacetylase. Although the principle of the invention is of general application, not restricted by the chemical identity of the inhibitor, it is strongly preferred that the inhibitor be one that exerts its effect by reversible binding to the target histone deacetylase, as opposed to those whose effect is due to covalent binding to the target histone deacetylase.

Since for practical utility the carboxylesterase-hydrolysed conjugate is required to retain the intracellular binding activity of the parent modulator with its target histone deacetylase enzyme, attachment of the ester motif must take account of that requirement, which will be fulfilled if the alpha amino acid carboxylesterase ester motif is attached to the modulator such that the binding mode of the corresponding carboxylesterase hydrolysis product (ie the corresponding acid) to the target is essentially the same as the unconjugated modulator. In general this is achieved by covalent attachment of the carboxylesterase ester motif to the modulator at a position remote from the binding interface between the modulator and the target histone deacetylase enzyme. In this way, the motif is arranged to extend into solvent, rather than potentially interfering with the binding mode.

In addition, the amino acid carboxylesterase motif obviously must be a substrate for the carboxylesterase if the former is to be hydrolysed by the latter within the cell. Intracellular carboxylesterases are rather promiscuous in general, in that their ability to hydrolyse does not depend on very strict structural requirements of the amino acid ester substrate. Hence most modes of covalent conjugation of the amino acid carboxylesterase motif to a modulator will allow hydrolysis. Attachment by a flexible linker chain will usually be how this is achieved.

It will be appreciated that any chemical modification of a drug may subtly alter its binding geometry, and the chemistry strategy for linkage of the carboxylesterase ester motif may introduce additional binding interactions with the target, or may substitute for one or more such interactions. Hence the requirement that the hydrolysed conjugate's binding mode to the target is the same as the unconjugated modulator is to be interpreted as requiring that there is no significant perturbation of the binding mode, in other words that the binding mode is essentially the same as that of the unconjugated modulator. When the requirement is met, the main binding characteristics of the parent modulator are retained, and the modified and unmodified modulators have an overall common set of binding characteristics. The "same binding mode" and "remote attachment" viewpoints are similar because, as stated above, the usual way of achieving the "same binding mode" requirement is to attach the carboxylesterase motif at a point in the modulator molecule which is remote from the binding interface between the inhibitor and the target histone deacetylase enzyme. However, it should be noted that these requirements do not imply that the conjugate and/or its corresponding acid must have the same *in vitro* or *in vivo* modulatory potency as the parent modulator. As used herein, however, the 'same binding mode' and 'remote attachment' features are considered fulfilled when the ester has a potency in a cellular activity assay at least as high as that of the parent modulator in the same assay, the cellular assay being a cell proliferation assay carried out in U937 cancer cells. In general, is preferred that the esterase-hydrolysed carboxylic acid has a potency in an *in vitro* histone deacetylase inhibitory assay no less than one tenth of the potency of the parent modulator in that assay.

Although traditional medicinal chemistry methods of mapping structure-activity relationships are perfectly capable of identifying an attachment strategy to meet the foregoing "same binding mode" and "remote attachment" requirements, modern techniques such as NMR and X-ray crystallography have advanced to the point where it is very common for the binding mode of a known inhibitor of histone deacetylase to be known, or determinable. Such information is in the vast majority of cases in the public domain, or can be modelled using computer based modelling methods, such as ligand docking and homology modelling, based on the known binding modes of structurally similar modulators, or the known structure of the active site of histone deacetylase. With knowledge of the binding mode of the modulator obtained by these techniques, a suitable location for attachment of the carboxylesterase ester motif may be identified, usually (as stated above) at a point on the modulator which is remote from the binding interface between the inhibitor and the target histone deacetylase enzyme.

Intracellular carboxylesterase enzymes capable of hydrolysing the ester group of the conjugated alpha amino acid to the corresponding acid include the three known human carboxylesterase ("hCE") enzyme isotypes hCE-1 (also known as CES-1), hCE-2 (also known as CES-2) and hCE-3 (Drug Disc. Today 2005, 10, 313-325). Although these are considered to be the main enzymes other carboxylester enzymes such as biphenylhydrolase (BPH) may also have a role in hydrolysing the conjugates.

The broken cell assay described below is a simple method of confirming that a given conjugate of modulator and alpha amino acid ester, or a given alpha amino acid ester to be assessed as a possible carboxylesterase ester motif, is hydrolysed as required. These enzymes can also be readily expressed using recombinant techniques, and the recombinant enzymes may be used to determine or confirm that hydrolysis occurs.

It is a feature of the invention that the desired conjugate retains the covalently linked alpha amino acid motif when hydrolysed by the carboxylesterase(s) within the cell, since it is the polar carboxyl group of that motif which prevents or reduces clearance of the hydrolysed conjugate from the cell, and thereby contributes to its accumulation within the cell. Indeed, the cellular potency of the modified modulator is predominantly due to the accumulation of the acid and its

modulation of the activity of the target (although the unhydrolysed ester also exerts its activity on the target for so long as it remains unhydrolysed). Since cells in general contain several types of peptidase enzymes, it is preferable that the conjugate, or more especially the hydrolysed conjugate (the corresponding acid), is not a substrate for such peptidases. In particular, it is strongly preferred that the alpha amino acid ester group should not be the C-terminal element of a dipeptide motif in the conjugate. However, apart from that limitation on the mode of covalent attachment, the alpha amino acid ester group may be covalently attached to the modulator via its amino group or via its alpha carbon. In some cases the modulator will have a convenient point of attachment for the carboxylesterase ester motif, and in other cases a synthetic strategy will have to be devised for its attachment.

It has been found that cells that only express the carboxylesterases hCE-2, and/or hCE-3 and recombinant forms of these enzymes will only hydrolyse amino acid ester conjugates to their resultant acids if the nitrogen of the alpha amino acid group is either unsubstituted or is directly linked to a carbonyl group, whereas cells containing hCE-1, or recombinant hCE-1 can hydrolyse amino acid conjugates with a wide range of groups on the nitrogen. This selectivity requirement of hCE-2 and hCE-3 can be turned to advantage where it is required that the modulator should target enzymes or receptors in certain cell types only. It has been found that the relative amounts of these three carboxylesterase enzymes vary between cell types (see Figure 1 and database at http:/symatlas.gnf.org/SymAtlas (note that in this database hCE3/CES3 is referred to by the symbol FLJ21736)) If the modulator is intended to act only in cell types where hCE-1 is found, attachment of a carboxylesterase ester motif wherein the amino group is directly linked to a group other than carbonyl results in the hydrolysed modulator conjugate accumulating preferentially in cells with effective concentrations of hCE-1. Stated in another way, specific accumulation of the acid derived from the modulator conjugate in hCE-1 expressing cells is achieved by linking the amino acid ester motif to the modulator in such a way that the nitrogen atom of the amino acid ester is not linked directly to a carbonyl, or is left unsubstituted.

Macrophages are known to play a key role in inflammatory disorders through the release of cytokines in particular TNF$\alpha$ and IL-1 (van Roon et al Arthritis and Rheumatism , 2003, 1229-1238). In rheumatoid arthritis they are major contributors to joint inflammation and joint destruction (Conell in N.Eng J. Med. 2004, 350, 2591-2602). Macrophages are also involved in tumour growth and development (Naldini and Carraro in Curr Drug Targets Inflamm Allergy ,2005, 3-8 ). Hence agents that selectively target macrophage cells could be of value in the treatment of cancer, inflammation and autoimmune disease. Targeting specific cell types would be expected to lead to reduced side-effects. The present invention enables a method of targeting modulators to macrophages, which is based on the above observation that the way in which the carboxylesterase ester motif is linked to the modulator determines whether it is hydrolysed by specific carboxylesterases, and hence whether or not the resultant acid accumulates in different cell types. Specifically it has been found that macrophages contain the human carboxylesterase hCE-1 whereas other cell types do not. In the conjugates of the invention, the nitrogen of the ester motif is substituted but not directly bonded to a carbonyl group moiety so that the ester will only be hydrolysed by hCE-1 and hence the esterase-hydrolysed modulator conjugates will only accumulate in macrophages.

There are of course many possible ester groups which may in principle be present in the carboxylesterase ester motif for attachment to the modulator. Likewise, there are many alpha amino acids, both natural and non-natural, differing in the side chain on the alpha carbon, which may be used as esters in the carboxylesterase ester motif. Some alpha amino acid esters are rapidly hydrolysed by one or more of the hCE-1, -2 and -3 isotypes or cells containing these enzymes, while others are more slowly hydrolysed, or hydrolysed only to a very small extent. In general, if the carboxylesterase hydrolyses the free amino acid ester to the parent acid it will, subject to the N-carbonyl dependence of hCE-2 and hCE-3 discussed above, also hydrolyse the ester motif when covalently conjugated to the modulator. Hence, the broken cell assay and/or the isolated carboxylesterase assay described herein provide a straightforward, quick and simple first screen for esters which have the required hydrolysis profile. Ester motifs selected in that way may then be re-assayed in the same carboxylesterase assay when conjugated to the modulator via the chosen conjugation chemistry, to confirm that it is still a carboxylesterase substrate in that background. Suitable types of ester will be discussed below, but at this point it may be mentioned that it has been found that t-butyl esters of alpha amino acids are relatively poor substrates for hCE-1, -2 and -3, whereas cyclopentyl esters are effectively hydrolysed. Suitable alpha amino acids will also be discussed in more detail below, but at this point it may be mentioned that phenylalanine, homophenylalanine, phenylglycine and leucine are generally suitable, and esters of secondary alcohols are preferred.

As stated above, the alpha amino acid ester may be conjugated to the modulator via the amino group of the amino acid ester, or via the alpha carbon (for example through its side chain) of the amino acid ester. A linker radical may be present between the carboxylesterase ester motif and the modulator. For example, the alpha amino acid ester may be conjugated to the modulator as a radical of formula (IA), (IB) or (IC):
wherein

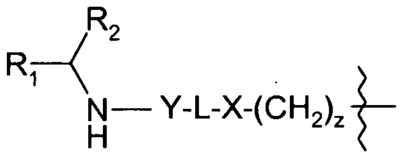

(IA)

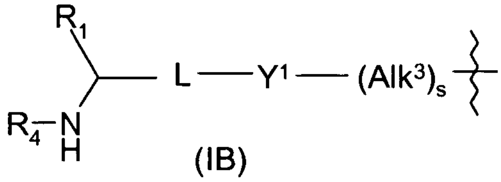

(IB)

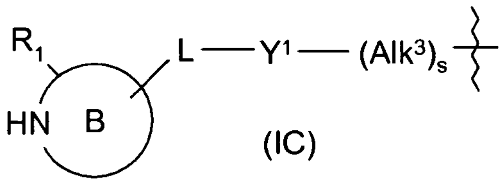

(IC)

**$R_1$** is an ester group which is hydrolysable by one or more intracellular carboxylesterase enzymes to a carboxylic acid group;

**$R_2$** is the side chain of a natural or non-natural alpha amino acid;

**$R_4$** is hydrogen; or optionally substituted $C_1$-$C_6$ alkyl, $C_3$-$C_7$ cycloalkyl, aryl or heteroaryl;

**B** is a monocyclic heterocyclic ring of 5 or 6 ring atoms wherein $R_1$ is linked to a ring carbon adjacent the ring nitrogen shown, and ring B is optionally fused to a second carbocyclic or heterocyclic ring of 5 or 6 ring atoms in which case the bond to L may be from a ring atom in said second ring

Y is a bond, $-C(=S)-NR_3-$, $-C(=NH)NR_3-$ or $-S(=O)_2NR_3-$ wherein $R_3$ is hydrogen or optionally substituted $C_1$-$C_6$ alkyl;

**$Y^1$** is a bond, -(C=O)-, $-S(O_2)-$, -C(=O)O-, -OC(=O)-, $-(C=O)NR_3-$, $-NR_3(C=O)-$, $-S(O_2)NR_3-$, $-NR_3S(O_2)-$, or $-NR_3(C=O)NR_5-$, wherein $R_3$ and $R_5$ are independently hydrogen or optionally substituted $(C_1$-$C_6)$alkyl,

**L** is a divalent radical of formula $-(Alk^1)_m(Q)_n(Alk^2)_p-$ wherein

m, n and p are independently 0 or 1,

**Q** is (i) an optionally substituted divalent mono- or bicyclic carbocyclic or heterocyclic radical having 5 - 13 ring members, or (ii), in the case where both m and p are 0, a divalent radical of formula $-X^2-Q^1-$ or $-Q^1-X^2-$ wherein $X^2$ is -O-, -S- or $NR^A-$ wherein $R^A$ is hydrogen or optionally substituted $C_1$-$C_3$ alkyl, and $Q^1$ is an optionally substituted divalent mono- or bicyclic carbocyclic or heterocyclic radical having 5-13 ring members,

**$Alk^1$** and **$Alk^2$** independently represent optionally substituted divalent $C_3$-$C_7$ cycloalkyl radicals, or optionally substituted straight or branched, $C_1$-$C_6$ alkylene, $C_2$-$C_6$ alkenylene, or $C_2$-$C_6$ alkynylene radicals which may optionally contain or terminate in an ether (-O-), thioether (-S-) or amino $(-NR^A-)$ link wherein $R^A$ is hydrogen or optionally substituted $C_1$-$C_3$ alkyl;

**X** represents a bond, -C(=O)-; $-S(=O)_2-$; $-NR_3C(=O)-$, $-C(=O)NR_3-$,$-NR_3C(=O)NR_5-$, $-NR_3S(=O)_2-$, or $-S(=O)_2NR_3-$ wherein $R_3$ and $R_5$ are independently hydrogen or optionally substituted $C_1$-$C_6$ alkyl;

**z** is 0 or 1;

**s** is 0 or 1; and

**$Alk^3$** represents an optionally substituted divalent $C_3$-$C_7$ cycloalkyl radical, or optionally substituted straight or branched, $C_1$-$C_6$ alkylene, $C_2$-$C_6$ alkenylene ,or $C_2$-$C_6$ alkynylene radical which may optionally contain or terminate in an ether (-O-), thioether (-S-) or amino $(-NR^A-)$ link wherein $R^A$ is hydrogen or optionally substituted $C_1$-$C_3$ alkyl;

wherein the nitrogen atom of the amino group of the amino acid ester is not linked directly to a carbonyl moiety or is left unsubstituted.

The term "ester" or "esterified carboxyl group" means a group $R_9O(C=O)$- in which $R_9$ is the group characterising the ester, notionally derived from the alcohol $R_9OH$.

As used herein, the term "($C_a$-$C_b$)alkyl" wherein a and b are integers refers to a straight or branched chain alkyl radical having from a to b carbon atoms. Thus when a is 1 and b is 6, for example, the term includes methyl, ethyl, n-propyl, isopropyl, n-butyl, isobutyl, sec-butyl, t-butyl, n-pentyl and n-hexyl.

As used herein the term "divalent ($C_a$-$C_b$)alkylene radical" wherein a and b are integers refers to a saturated hydrocarbon chain having from a to b carbon atoms and two unsatisfied valences.

As used herein the term "($C_a$-$C_b$)alkenyl" wherein a and b are integers refers to a straight or branched chain alkenyl moiety having from a to b carbon atoms having at least one double bond of either E or Z stereochemistry where applicable. The term includes, for example, vinyl, allyl, 1- and 2-butenyl and 2-methyl-2-propenyl.

As used herein the term "divalent ($C_a$-$C_b$)alkenylene radical" means a hydrocarbon chain having from a to b carbon atoms, at least one double bond, and two unsatisfied valences.

As used herein the term "$C_a$-$C_b$ alkynyl" wherein a and b are integers refers to straight chain or branched chain hydrocarbon groups having from two to six carbon atoms and having in addition one triple bond. This term would include for example, ethynyl, 1-propynyl, 1- and 2-butynyl, 2-methyl-2-propynyl, 2-pentynyl, 3-pentynyl, 4-pentynyl, 2-hexynyl, 3-hexynyl, 4-hexynyl and 5-hexynyl.

As used herein the term "divalent ($C_a$-$C_b$)alkynylene radical" wherein a and b are integers refers to a divalent hydrocarbon chain having from 2 to 6 carbon atoms, and at least one triple bond.

As used herein the term "carbocyclic" refers to a mono-, bi- or tricyclic radical having up to 16 ring atoms, all of which are carbon, and includes aryl and cycloalkyl.

As used herein the term "cycloalkyl" refers to a monocyclic saturated carbocyclic radical having from 3-8 carbon atoms and includes, for example, cyclopropyl, cyclobutyl, cyclopentyl, cyclohexyl, cycloheptyl and cyclooctyl.

As used herein the unqualified term "aryl" refers to a mono-, bi- or tri-cyclic carbocyclic aromatic radical, and includes radicals having two monocyclic carbocyclic aromatic rings which are directly linked by a covalent bond. Illustrative of such radicals are phenyl, biphenyl and napthyl.

As used herein the unqualified term "heteroaryl" refers to a mono-, bi- or tri-cyclic aromatic radical containing one or more heteroatoms selected from S, N and O, and includes radicals having two such monocyclic rings, or one such monocyclic ring and one monocyclic aryl ring, which are directly linked by a covalent bond. Illustrative of such radicals are thienyl, benzthienyl, furyl, benzfuryl, pyrrolyl, imidazolyl, benzimidazolyl, thiazolyl, benzthiazolyl, isothiazolyl, benzisothiazolyl, pyrazolyl, oxazolyl, benzoxazolyl, isoxazolyl, benzisoxazolyl, isothiazolyl, triazolyl, benztriazolyl, thiadiazolyl, oxadiazolyl, pyridinyl, pyridazinyl, pyrimidinyl, pyrazinyl, triazinyl, indolyl and indazolyl.

As used herein the unqualified term "heterocyclyl" or "heterocyclic" includes "heteroaryl" as defined above, and in its non-aromatic meaning relates to a mono-, bi- or tri-cyclic non-aromatic radical containing one or more heteroatoms selected from S, N and O, and to groups consisting of a monocyclic non-aromatic radical containing one or more such heteroatoms which is covalently linked to another such radical or to a monocyclic carbocyclic radical. Illustrative of such radicals are pyrrolyl, furanyl, thienyl, piperidinyl, imidazolyl, oxazolyl, isoxazolyl, thiazolyl, thiadiazolyl, pyrazolyl, pyridinyl, pyrrolidinyl, pyrimidinyl, morpholinyl, piperazinyl, indolyl, morpholinyl, benzfuranyl, pyranyl, isoxazolyl, benzimidazolyl, methylenedioxyphenyl, ethylenedioxyphenyl, maleimido and succinimido groups.

Unless otherwise specified in the context in which it occurs, the term "substituted" as applied to any moiety herein means substituted with up to four compatible substituents, each of which independently may be, for example, ($C_1$-$C_6$)alkyl, ($C_1$-$C_6$)alkoxy, hydroxy, hydroxy($C_1$-$C_6$)alkyl, mercapto, mercapto($C_1$-$C_6$)alkyl, ($C_1$-$C_6$)alkylthio, phenyl, halo (including fluoro, bromo and chloro), trifluoromethyl, trifluoromethoxy, nitro, nitrile (-CN), oxo, -COOH, $-COOR^A$, $-COR^A$, $-SO_2R^A$,
$-CONH_2$, $-SO_2NH_2$, $-CONHR^A$, $-SO_2NHR^A$, $-CONR^AR^B$, $-SO_2NR^AR^B$, $-NH_2$, $-NHR^A$, $-NR^AR^B$, $-OCONH_2$, $-OCONHR^A$, $-OCONR^AR^B$, $-NHCOR^A$, $-NHCOOR^A$, $-NR^BCOOR^A$, $-NHSO_2OR^A$, $-NR^BSO_2OH$, $-NR^BSO_2OR^A$,$-NHCONH_2$, $-NR^ACONH_2$, $-NHCONHR^B$,$-NR^ACONHR^B$, $-NHCONR^AR^B$, or $-NR^ACONR^AR^B$ wherein $R^A$ and $R^B$ are independently a ($C_1$-$C_6$)alkyl, ($C_3$-$C_6$) cycloalkyl , phenyl or monocyclic heteroaryl having 5 or 6 ring atoms. An "optional substituent" may be one of the foregoing substituent groups.

The term "side chain of a natural or non-natural alpha-amino acid" refers to the group $R^1$ in a natural or non-natural amino acid of formula $NH_2$-$CH(R^1)$-COOH.

Examples of side chains of natural alpha amino acids include those of alanine, arginine, asparagine, aspartic acid, cysteine, cystine, glutamic acid, histidine, 5-hydroxylysine, 4-hydroxyproline, isoleucine, leucine, lysine, methionine, phenylalanine, proline, serine, threonine, tryptophan, tyrosine, valine, $\alpha$-aminoadipic acid, $\alpha$-amino-n-butyric acid, 3,4-dihydroxyphenylalanine, homoserine, $\alpha$-methylserine, ornithine, pipecolic acid, and thyroxine.

Natural alpha-amino acids which contain functional substituents, for example amino, carboxyl, hydroxy, mercapto, guanidyl, imidazolyl, or indolyl groups in their characteristic side chains include arginine, lysine, glutamic acid, aspartic acid, tryptophan, histidine, serine, threonine, tyrosine, and cysteine. When $R_2$ in the compounds of the invention is one of those side chains, the functional substituent may optionally be protected.

The term "protected" when used in relation to a functional substituent in a side chain of a natural alpha-amino acid means a derivative of such a substituent which is substantially non-functional. For example, carboxyl groups may be esterified (for example as a $C_1$-$C_6$ alkyl ester), amino groups may be converted to amides (for example as a $NHCOC_1$-$C_6$ alkyl amide) or carbamates (for example as an $NHC(=O)OC_1$-$C_6$ alkyl or $NHC(=O)OCH_2Ph$ carbamate), hydroxyl groups may be converted to ethers (for example an $OC_1$-$C_6$ alkyl or a $O(C_1$-$C_6$ alkyl)phenyl ether) or esters (for example a $OC(=O)C_1$-$C_6$ alkyl ester) and thiol groups may be converted to thioethers (for example a tert-butyl or benzyl thioether) or thioesters (for example a $SC(=O)C_1$-$C_6$ alkyl thioester).

Examples of side chains of non-natural alpha amino acids include those referred to below in the discussion of suitable $R_2$ groups for use in compounds of the present invention.

*The ester group $R_1$*

In addition to the requirement that the ester group must be hydrolysable by one or more intracellular enzymes, it may be preferable for some applications (for example for systemic administration of the conjugate) that it be resistant to hydrolysis by carboxylester-hydrolysing enzymes in the plasma, since this ensures the conjugated modulator will survive after systemic administration for long enough to penetrate cells as the ester. It is a simple matter to test any given conjugate to measure its plasma half life as the ester, by incubation in plasma. However, it has been found that esters notionally derived from secondary alcohols are more stable to plasma carboxylester-hydrolysing enzymes than those derived from primary alcohols. Furthermore, it has also been found that although esters notionally derived from tertiary alcohols are generally stable to plasma carboxylester-hydrolysing enzymes, they are often also relatively stable to intracellular carboxylesterases. Taking these findings into account, it is presently preferred that $R_1$ in formulae (IA), (IB) and (IC) above, is an ester group of formula $-(C=O)OR_9$ wherein $R_9$ is (i) $R_7R_8CH$- wherein $R_7$ is optionally substituted $(C_1$-$C_3)$alkyl-$(Z^1)_a$-$(C_1$-$C_3)$alkyl- or $(C_2$-$C_3)$alkenyl-$(Z^1)_a$-$(C_1$-$C_3)$alkyl- wherein a is 0 or 1 and $Z^1$ is -O-, -S-, or -NH-, and $R_8$ is hydrogen or $(C_1$-$C_3)$alkyl- or $R_7$ and $R_8$ taken together with the carbon to which they are attached form an optionally substituted $C_3$-$C_7$ cycloalkyl ring or an optionally substituted heterocyclic ring of 5- or 6-ring atoms; or (ii) optionally substituted phenyl or monocyclic heterocyclic ring having 5 or 6 ring atoms. Within these classes, $R_9$ may be, for example, methyl, ethyl, n- or iso-propyl, n- or sec-butyl, cyclohexyl, allyl, phenyl, benzyl, 2-, 3- or 4-pyridylmethyl, N-methylpiperidin-4-yl, tetrahydrofuran-3-yl or methoxyethyl. Currently preferred is where $R_9$ is cyclopentyl.

*The amino acid side chain $R_2$*

Subject to the requirement that the ester group $R_1$ be hydrolysable by intracellular carboxylesterase enzymes, the selection of the side chain group $R_2$ can determine the rate of hydrolysis. For example, when the carbon in $R_2$ adjacent to the alpha amino acid carbon does not contain a branch eg when $R_2$ is ethyl, isobutyl or benzyl the ester is more readily hydrolysed than when $R_2$ is branched eg isopropyl or t-butyl.

Examples of amino acid side chains include

$C_1$-$C_6$ alkyl, phenyl, 2,- 3-, or 4-hydroxyphenyl, 2,- 3-, or 4-methoxyphenyl, 2,-3-, or 4-pyridylmethyl, benzyl, phenylethyl, 2-, 3-, or 4-hydroxybenzyl, 2,- 3-,

or 4-benzyloxybenzyl, 2,- 3-, or 4- $C_1$-$C_6$ alkoxybenzyl, and benzyloxy($C_1$-$C_6$ alkyl)-groups;

the characterising group of a natural $\alpha$ amino acid, in which any functional group may be protected;

groups $-[Alk]_nR_6$ where Alk is a $(C_1$-$C_6)$alkyl or $(C_2$-$C_6)$alkenyl group optionally interrupted by one or more -O-, or -S- atoms or $-N(R_7)$- groups [where $R_7$ is a hydrogen atom or a $(C_1$-$C_6)$alkyl group], n is 0 or 1, and $R_6$ is an optionally substituted cycloalkyl or cycloalkenyl group;

a benzyl group substituted in the phenyl ring by a group of formula $-OCH_2COR_8$ where $R_8$ is hydroxyl, amino, $(C_1$-$C_6)$alkoxy, phenyl$(C_1$-$C_6)$alkoxy, $(C_1$-$C_6)$alkylamino, di($(C_1$-$C_6)$alkyl)amino, phenyl$(C_1$-$C_6)$alkylamino, the residue of an amino acid or acid halide, ester or amide derivative thereof, said residue being linked via an amide bond, said amino acid being selected from glycine, $\alpha$ or $\beta$ alanine, valine, leucine, isoleucine, phenylalanine, tyrosine, tryptophan, serine, threonine, cysteine, methionine, asparagine, glutamine, lysine, histidine, arginine, glutamic acid, and aspartic acid;

a heterocyclic$(C_1$-$C_6)$alkyl group, either being unsubstituted or mono- or di-substituted in the heterocyclic ring with halo, nitro, carboxy, $(C_1$-$C_6)$alkoxy, cyano, $(C_1$-$C_6)$alkanoyl, trifluoromethyl $(C_1$-$C_6)$alkyl, hydroxy, formyl, amino, $(C_1$-$C_6)$alkylamino, di-$(C_1$-$C_6)$alkylamino, mercapto, $(C_1$-$C_6)$alkylthio, hydroxy$(C_1$-$C_6)$alkyl, mercapto$(C_1$-$C_6)$alkyl or $(C_1$-$C_6)$alkylphenylmethyl; and

a group $-CR_aR_bR_c$ in which:

each of $R_a$, $R_b$ and $R_c$ is independently hydrogen, $(C_1$-$C_6)$alkyl, $(C_2$-$C_6)$alkenyl, $(C_2$-$C_6)$alkynyl, phenyl$(C_1$-$C_6)$alkyl, $(C_3$-$C_8)$cycloalkyl; or

$R_c$ is hydrogen and $R_a$ and $R_b$ are independently phenyl or heteroaryl such as pyridyl; or

$R_c$ is hydrogen, $(C_1-C_6)$alkyl, $(C_2-C_6)$alkenyl, $(C_2-C_6)$alkynyl, phenyl$(C_1-C_6)$alkyl, or $(C_3-C_8)$cycloalkyl, and $R_a$ and $R_b$ together with the carbon atom to which they are attached form a 3 to 8 membered cycloalkyl or a 5- to 6-membered heterocyclic ring; or

$R_a$, $R_b$ and $R_c$ together with the carbon atom to which they are attached form a tricyclic ring (for example adamantyl); or

$R_a$ and $R_b$ are each independently $(C_1-C_6)$alkyl, $(C_2-C_6)$alkenyl, $(C_2-C_6)$alkynyl, phenyl$(C_1-C_6)$alkyl, or a group as defined for $R_c$ below other than hydrogen, or $R_a$ and $R_b$ together with the carbon atom to which they are attached form a cycloalkyl or heterocyclic ring, and $R_c$ is hydrogen, -OH, -SH, halogen, -CN, $-Co_2H$, $(C_1-C_4)$perfluoroalkyl, $-CH_2OH$, $-CO_2(C_1-C_6)$alkyl, $-O(C_1-C_6)$alkyl, $-O(C_2-C_6)$alkenyl,$-S(C_1-C_6)$alkyl, $-SO(C_1-C_6)$alkyl, $-SO_2(C_1-C_6)$ alkyl, $-S(C_2-C_6)$alkenyl, $-SO(C_2-C_6)$alkenyl, $-SO_2(C_2-C_6)$alkenyl or a group -Q-W wherein Q represents a bond or -O-, -S-, -SO- or $-SO_2-$ and W represents a phenyl, phenylalkyl, $(C_3-C_8)$cycloalkyl, $(C_3-C_8)$cycloalkylalkyl, $(C_4-C_8)$cycloalkenyl, $(C_4-C_8)$cycloalkenylalkyl, heteroaryl or heteroarylalkyl group, which group W may optionally be substituted by one or more substituents independently selected from, hydroxyl, halogen, -CN, $-CO_2H$, $-CO_2(C_1-C_6)$alkyl, $-CONH_2$, $-CONH(C_1-C_6)$alkyl, $-CONH(C_1-C_6alkyl)_2$, -CHO, $-CH_2OH$, $(C_1-C_4)$perfluoroalkyl, $-O(C_1-C_6)$alkyl, $-S(C_1-C_6)$alkyl, $-SO(C_1-C_6)$alkyl, $-SO_2(C_1-C_6)$alkyl , - $NO_2$, $-NH_2$, $-NH(C_1-C_6)$alkyl, $-N((C_1-C_6)alkyl)_2$, $-NHCO(C_1-C_6)$alkyl, $(C_1-C_6)$ alkyl, $(C_2-C_6)$alkenyl, $(C_2-C_6)$alkynyl, $(C_3-C_8)$cycloalkyl, $(C_4-C_8)$cycloalkenyl, phenyl or benzyl.

Examples of particular $R_2$ groups include benzyl, phenyl, cyclohexylmethyl, pyridin-3-ylmethyl, tert-butoxymethyl, iso-butyl, sec-butyl, tert-butyl, 1-benzylthio-1-methylethyl, 1-methylthio-1-methylethyl, and 1-mercapto-1-methylethyl, phenylethyl. Presently preferred $R_2$ groups include phenyl, benzyl, tert-butoxymethyl, phenylethyl and iso-butyl.

*The group* $R_4$

$R_4$ may be optionally substituted $C_1-C_6$ alkyl, $C_3-C_7$cycloalkyl, aryl or heteroaryl, for example methyl, ethyl, n- or iso-propyl, cyclopropyl, cyclopentyl, cyclohexyl, phenyl, or pyridyl.

*The ring or ring system* B

Ring or ring system B may be one chosen from, for example, the following:

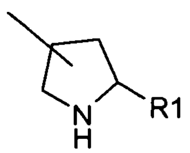
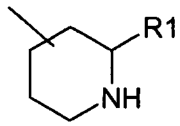
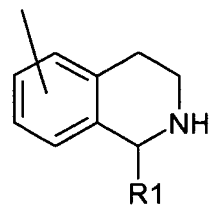
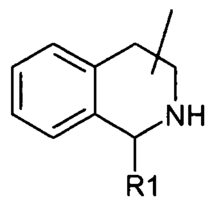

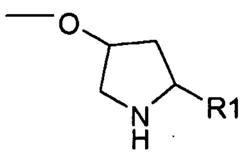
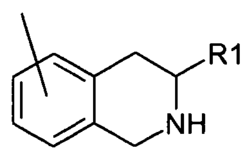
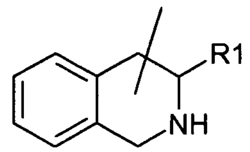

The radical $-Y-L-X-[CH_2]_z-$

When the alpha amino acid ester is conjugated to the inhibitor as a radical of formula (IA) this radical (or bond) arises from the particular chemistry strategy chosen to link the amino acid ester motif $R_1CH(R_2)NH-$ to the modulator. Clearly the chemistry strategy for that coupling may vary widely, and thus many combinations of the variables Y, L, X and z are possible.

It should also be noted that the benefits of the amino acid ester carboxyl esterase motif described above (facile entry into the cell, carboxylesterase hydrolysis within the cell, and accumulation within the cell of active carboxylic acid hydrolysis product) are best achieved when the linkage between the amino acid ester motif and the modulator is not a substrate for peptidase activity within the cell, which might result in cleavage of the amino acid from the molecule. Of course, stability to intracellular peptidases is easily tested by incubating the compound with disrupted cell contents, and analysing for any such cleavage.

With the foregoing general observations in mind, taking the variables making up the radical $-Y-L-X-[CH_2]_z-$ in turn:

z may be 0 or 1, so that a methylene radical linked to the modulator is optional.

specific preferred examples of Y include a bond.

In the radical L, examples of $Alk^1$ and $Alk^2$ radicals, when present, include

- $CH_2$-, $-CH_2CH_2$- $-CH_2CH_2CH_2$-, $-CH_2CH_2CH_2CH_2$-, -CH=CH-, $-CH=CHCH_2$-, $-CH_2CH=CH$-, $CH_2CH=CHCH_2$-,-C≡C-, $-C≡CCH_2$-, $CH_2C≡C$-, and $CH_2C≡CCH_2$. Additional examples of $Alk^1$ and $Alk^2$ include $-CH_2W$-,- $CH_2CH_2W$- $-CH_2CH_2WCH_2$-, $-CH_2CH_2WCH(CH_3)$-, $-CH_2WCH_2CH_2$-, $-CH_2WCH_2CH_2WCH_2$-, and $-WCH_2CH_2$- where W is -O-, -S-, -NH-, $-N(CH_3)$-, or $-CH_2CH_2N(CH_2CH_2OH)CH_2$-. Further examples of $Alk^1$ and $Alk^2$ include divalent cyclopropyl, cyclopentyl and cyclohexyl radicals.

In L, when n is 0, the radical is a hydrocarbon chain (optionally substituted and perhaps having an ether, thioether or amino linkage). Presently it is preferred that there be no optional substituents in L. When both m and p are 0, L is a divalent mono- or bicyclic carbocyclic or heterocyclic radical with 5 - 13 ring atoms (optionally substituted). When n is 1 and at least one of m and p is 1, L is a divalent radical including a hydrocarbon chain or chains and a mono- or bicyclic carbocyclic or heterocyclic radical with 5 - 13 ring atoms (optionally substituted). When present, Q may be, for example, a divalent phenyl, naphthyl, cyclopropyl, cyclopentyl, or cyclohexyl radical, or a mono-, or bi-cyclic heterocyclic radical having 5 to13 ring members, such as piperidinyl, piperazinyl, indolyl, pyridyl, thienyl, or pyrrolyl radical, but 1,4-phenylene is presently preferred.

Specifically, in some embodiments of the invention, m and p may be 0 with n being 1. In other embodiments, n and p may be 0 with m being 1. In further embodiments, m, n and p may be all 0. In still further embodiments m may be 0, n may be 1 with Q being a monocyclic heterocyclic radical, and p may be 0 or 1. $Alk^1$ and $Alk^2$, when present, may be selected from $-CH_2$-,
$-CH_2CH_2$-, and $-CH_2CH_2CH_2$- and Q may be 1,4-phenylene.

Specific examples of the radical $-Y-L-X-[CH_2]_z-$ include $-(CH_2)_v-$, $-(CH_2)_vO-$ $(CH_2)_wO-$

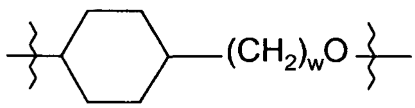

and

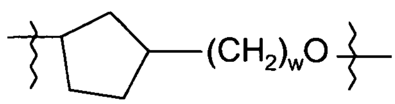

wherein v is 1, 2, 3 or 4 and w is 1, 2 or 3, such as $-CH_2$- and $-CH_2O$.

<u>The radical $-L-Y^1-$</u>

When the alpha amino acid ester is conjugated to the inhibitor as a radical of formula (IB) this radical (or bond) arises from the particular chemistry strategy chosen to link the alpha carbon of the amino acid ester motif in formula (IB) or (IC) to the modulator. (In the latter case, the $-L-Y^1-$ radical is indirectly linked to the alpha carbon through the intervening ring atoms of ring system B.) Clearly the chemistry strategy for that coupling may vary widely, and thus many combinations of the variables L and $Y^1$ are possible.

For example, L may be as discussed above in the context of the radical $-Y-L-X-[CH_2]_z-$. For example, in some embodiments m and n are 1 and p is 0; Q is -O-; and $Alk^1$ is an optionally substituted, straight or branched, $C_1$-$C_6$ alkylene, $C_2$-$C_6$ alkenylene or $C_2$-$C_6$ alkynylene radical which may optionally contain or terminate in an ether (-O-), thioether (-S-) or amino ($-NR^A$-) link wherein $R^A$ is hydrogen or optionally substituted $C_1$-$C_4$ alkyl. In other embodiments, m, n and p may each be 1, and in such cases Q may be, for example a 1,4 phenylene radical, or a cyclopentyl, cyclohexyl, piperidinyl or piperazinyl radical. In all embodiments, $Y^1$ may be, for example, a bond, or -(C=O)-, -(C=O)NH-, and -(C=O)O-.

For compounds of the invention which are to be administered systemically, esters with a slow rate of carboxylesterase cleavage are preferred, since they are less susceptible to pre-systemic metabolism. Their ability to reach their target tissue intact is therefore increased, and the ester can be converted inside the cells of the target tissue into the acid product. However, for local administration, where the ester is either directly applied to the target tissue or directed there by, for example, inhalation, it will often be desirable that the ester has a rapid rate of esterase cleavage, to minimise systemic exposure and consequent unwanted side effects. Where the esterase motif is linked to the modulator via its

amino group, as in formula (IA) above, if the carbon adjacent to the alpha carbon of the alpha amino acid ester is monosubstituted, ie $R_2$ is $CH_2R^z$ ($R^z$ being the mono-substituent) then the esters tend to be cleaved more rapidly than if that carbon is di- or tri-substituted, as in the case where $R_2$ is, for example, phenyl or cyclohexyl. Similarly, where the esterase motif is linked to the modulator via a carbon atom as in formulae (IB) and (IC) above, if a carbon atom to which the $R_4NHCH(R_1)$- or $R_1$-(ring B)- esterase motifs are attached is unsubstituted, ie $R_4NHCH(R_1)$- or $R_1$-(ring B)- is attached to a methylene ($-CH_2$)- radical, then the esters tend to be cleaved more rapidly than if that carbon is substituted, or is part of a ring system such as a phenyl or cyclohexyl ring.

*Modulators of Intracellular Enzymes and Receptors*

The principles of this invention can be applied to modulators of a wide range of intracellular targets which are implicated in a wide range of diseases. As discussed, the binding modes of known modulators to their targets are generally known soon after the modulators themselves become known. In addition, modern techniques such as X-ray crystallography and NMR are capable of revealing such binding topologies and geometries, as are traditional medicinal chemistry methods of characterising structure-activity relationships. With such knowledge, it is straightforward to identify where in the structure of a given modulator an carboxylesterase ester motif could be attached without disrupting the binding of the modulator to the enzyme or receptor by use of structural data. For example, Table 1 lists some intracellular enzyme or receptor targets where there is published crystal structural data. The present invention relates to inhibitors of histone deacetylase. The remaining modulators are provided for reference.

Table 1

| Target | Crystal Structure reference | Target Disease |
|---|---|---|
| CD45 | Nam et al., J Exp Med 201, 441 (2005) | Autoimmune disease |
| Lck | Zhu et al., Structure 7, 651 (1999) | Inflammation |
| ZAP-70 | Jin et al., J Biol Chem 279, 42818 (2004) | Autoimmune disease |
| PDE4 | Huai et al., Biochemistry 42, 13220 (2003) | Inflammation |
| PDE3 | Scapin et al., Biochemistry 43, 6091 (2004) | Asthma |
| IMPDH | Intchak et al., Cell 85, 921 (1996) | Psoriasis |
| p38 MAPK | Wang et al., Structure 6, 1117 (1998) | Inflammation |
| COX2 | Kiefer et al., J Biol Chem 278, 45763, (2003) | Inflammation |
| Adenosine Kinase | Schumacher et al., J Mol Biol 298, 875 (2000) | Inflammation |
| PLA2 | Chandra et al., Biochemistry B 10914 (2002) | Psoriasis |
| PLC | Essen et al., Biochemistry 36, 1704, (1997) | Rheumatoid arthritis |
| PLD | Leiros et al., J Mol Biol 339, 805 (2004) | Inflammation |
| iNOS | Rosenfeld et al., Biochemistry 41, 13915 (2002) | Inflammation |
| LTA4 hydrolase | Rudberg et al., J Biol Chem 279, 27376 (2004) | Inflammation |
| ICE | Okamato et al., Chem Pharm Bull 47, 11 (1999) | Rheumatoid arthritis |
| GSK3β | Bertrand et al., J Mol Biol 333, 393 (2003) | Rheumatoid arthritis |
| PKC | Xu et al., JBC 279, 50401 (2004) | Inflammation |
| PARP | Ruf et al., PNAS (USA) 93, 7481 (1996) | Proliferative disorders |
| MetAP2 | Sheppard et al Bioorg Med Chem Lett 14, 865 (2004) | Rheumatoid arthritis |

(continued)

| Target | Crystal Structure reference | Target Disease |
|---|---|---|
| Corticosteroid receptor | Bledsoe et al., Cell 110, 93 (2002) | Inflammation |
| PI3K | Walker et al., Mol Cell Biol 6, 909 (2000) | Proliferative disorders |
| Raf | Wan et al., Cell 116, 855 (2004) | Proliferative |
| | | disorders |
| AKT/PKB | Yang et al., Nat Struct Biol 9, 940 (2002) | Proliferative disorders |
| HDAC | Finnin et al., Nature 401, 188 (1999) | Proliferative disorders |
| c-Abl | Nagar et al., Cancer Res 62, 4236 (2002) | Proliferative disorders |
| IGF-1R | Munshi et al., Acta Crystallogr Sect D 59, 1725 (2003) | Proliferative disorders |
| Thymidylate Synthetase | Stout et al., Structure 6, 839 (1998) | Proliferative disorders |
| Glycinamide Ribonucleotide Formyltransferase | Klein et al., J Mol Biol 249, 153 (1995) | Proliferative disorders |
| Purine Nucleoside Phosphorylase | Koelner et al., J Mol Biol 280, 153 (1998) | Proliferative disorders |
| Estrone Sulphatase | Hernandez-Guzman et al., J Biol Chem 278, 22989 (2003) | Proliferative disorders |
| EGF-RTK | Stamos et al., J Biol Chem 277, 46265 (2002) | Proliferative disorders |
| Src kinase | Lamers et al., J Mol Biol 285, 713 (1999) | Proliferative disorders |
| VEGFR2 | McTigue et al., Structure 7, 319 (19999) | Proliferative disorders |
| Superoxide Dismutase | Hough et al., J Mol Biol 287, 579 (1999) | Proliferative disorders |
| Ornithine Decarboxylase | Almrud et al., J Mol Biol 295, 7 (2000) | Proliferative disorders |
| Topoisomerase II | Classen et al., PNAS (USA) 100, 10629 (2003) | Proliferative disorders |
| Topoisomerase I | Staker et al., PNAS (USA), 99, 15387 (2002) | Proliferative disorders |
| Androgen Receptor | Matias et al., J Biol Chem 275, 26164 (2000) | Proliferative disorders |
| JNK | Heo et al., EMBO J 23, 2185 (2004) | Proliferative disorders |
| Farnesyl Transferase | Curtin et al., Bioorg Med Chem Lett 13, 1367 (2003) | Proliferative disorders |
| CDK | Davis et al., Science 291, 134 (2001) | Proliferative disorders |
| Dihydrofolate Reductase | Gargaro et al., J Mol Biol 277, 119 (1998) | Proliferative disorders |
| Flt3 | Griffith et al., Mol Cell 13, 169 (2004) | Proliferative disorders |
| Carbonic Anhydrase | Stams et al., Protein Sci 7, 556 (1998) | Proliferative disorders |
| Thymidine Phosphorylase | Norman et al., Structure 12, 75 (2004) | Proliferative disorders |
| Dihydropyrimidine Dehydrogenase | Dobritzsch et al., JBC 277, 13155, (2002) | Proliferative disorders |
| Mannosidase $\alpha$ | Van den Elsen et al., EMBO J 20, 3008 (2001) | Proliferative disorders |
| Peptidyl-prolyl isomerase (Pin1) | Ranganathan et al., Cell 89, 875 (1997) | Proliferative disorders |
| Retinoid X Receptor | Egea et al., EMBO J 19, 2592 (2000) | Proliferative disorders |
| $\beta$-Glucuronidase | Jain et al., Nat Struct Biol 3, 375 (1996) | Proliferative disorders |

(continued)

| Target | Crystal Structure reference | Target Disease |
|---|---|---|
| Glutathione Transferase | Oakley et al., J Mol Biol 291, 913 (1999) | Proliferative disorders |
| hsp90 | Jez et al., Chem Biol 10, 361 (2003) | Proliferative disorders |
| IMPDH | intchak et al., Cell 85, 921 (1996) | Proliferative disorders |
| Phospholipase A2 | Chandra et al., Biochemistry 41, 10914 (2002) | Proliferative disorders |
| Phospholipase C | Essen et al., Biochemistry 36, 1704, (1997) | Proliferative disorders |
| Phospholipase D | Leiros et al., J Mol Biol 339, 805 (2004) | Proliferative disorders |
| MetAP2 | Sheppard et al Bioorg Med Chem Lett 14, 865 (2004) | Proliferative disorders |
| PTP-1 B | Andersen et al., J Biol Chem 275, 7101 (2000) | Proliferative disorders |
| Aurora Kinase | Fancelli et al., in press | Proliferative disorders |
| PDK-1 | Komander et al., Biochem J 375, 255 (2003) | Proliferative disorders |
| HMGCoA reductase | Istvan and Deisenhofer Science 292, 1160 (2001) | Atheriosclerosis |
| Oxidosqualene cyclase | Lenhart et al., Chem Biol 9, 639 (2002) | Hypercholesterolaem ia |
| Pyruvate dehydrogenase stimulator | Mattevi et al., Science 255, 1544 (1992) | Cardiovascular disease |
| Adenylate cyclase | Zhang et al., Nature 386, 247 (1997) | Cardiovascular disease |
| PPARγ agonist | Ebdurp et al., J Med Chem 46, 1306 (2003) | Diabetes |
| Alcohol dehydrogenase | Bahnson et al., PNAS USA 94, 12797 (1997) | Alcohol poisoning |
| Hormone sensitive lipase | Wei et al., Nat Struct Biol 6, 340 (1999) | Insulin resistant diabetes |
| Adenosine kinase | Mathews et al., Biochemistry 37, 15607 (1998) | Epilepsy |
| Aldose reductase | Urzhmsee al.,Structure 5, 601 (1997) | Diabetes |
| Vitamin D3 receptor | Tocchini-Vatentini et al., PNAS USA 98, 5491 (2001) | Osteoporosis |
| Protein tyrosine phosphatase | Andersen et al., J Biol Chem 275, 7101 (2000) | Diabetes |
| HIV Protease | Louis et al., Biochemistry 37, 2105 (1998) | HIV |
| HCV Polymerase | Bressanelli et al., PNAS USA 96, 13034 (1999) | Hepatitis C |
| Neuraminidase | Taylor et al., J Med Chem 41, 798 (1998) | Influenza |
| Reverse Transcriptase | Das et al., J Mol Biol 264, 1085 (1996) | HIV |
| CMV Protease | Khayat et al., Biochemistry 42, 885 (2003) | CMV infection |
| Thymidine Kinase | Champness et al., Proteins 32, 350 (1998) | Herpes infections |
| HIV Integrase | Molteni et al., Acta Crystallogr Sect D 57, 536 (2001) | HIV |

For the purpose of illustration, reference is made to known inhibitors of 5 of the above intracellular targets, whose binding mode to the target is known. These examples illustrate how such structural data can be used to determine

the appropriate positions for the attachment of the carboxylesterase ester motif. Schematics of the active sites are shown together with representative inhibitors. In general, positions remote from the binding interface between modulator and target, and therefore pointing away from the enzyme binding interface into solvent are suitable places for attachment of the carboxylesterase ester motif and these are indicated in the diagrams.

## HDAC

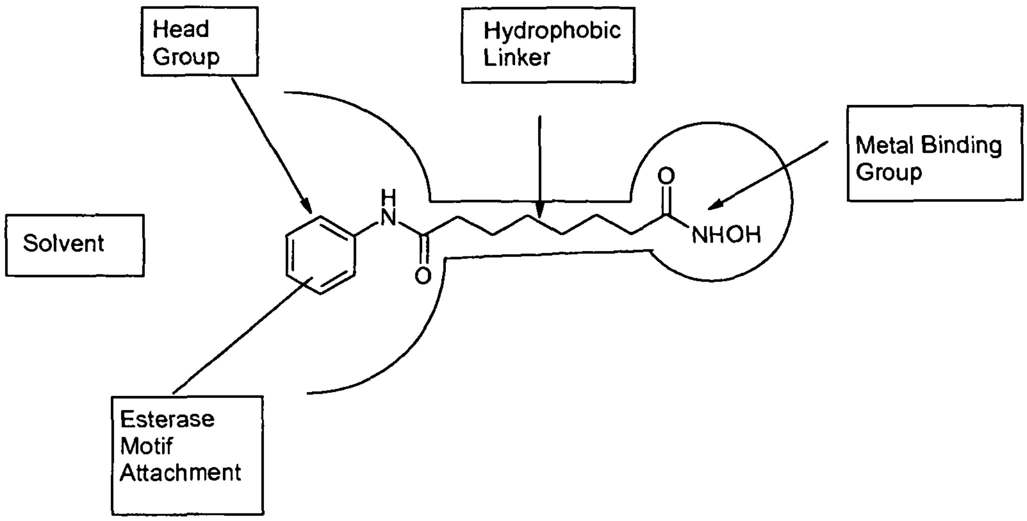


## Aurora kinase (reference example)

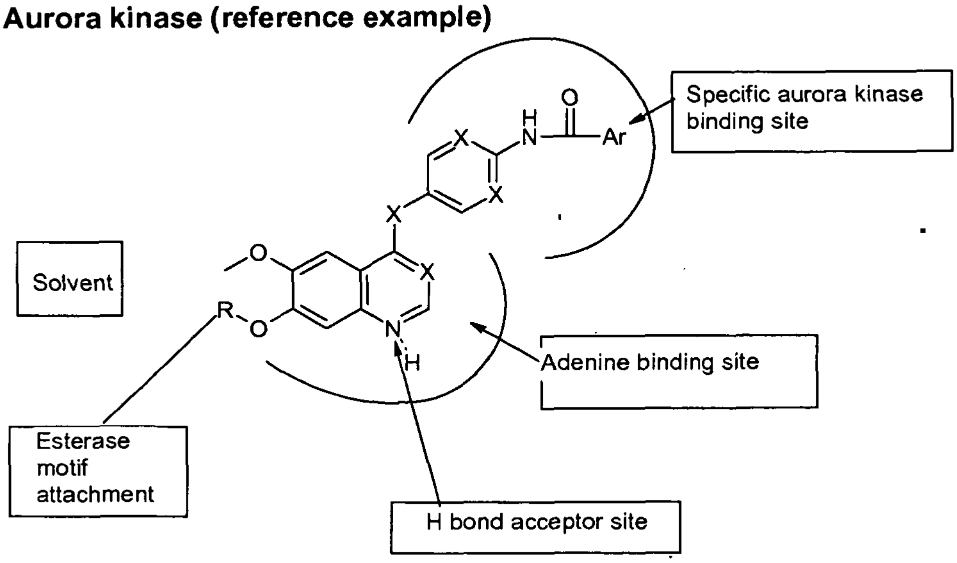

**PI3 Kinase (reference example)**

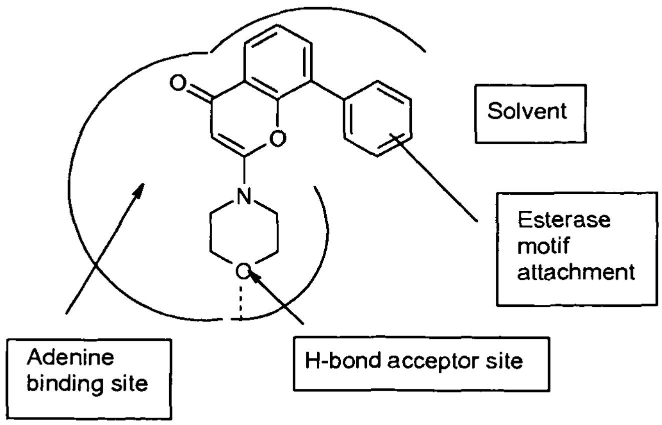


**P38 MAP Kinase (reference example)**

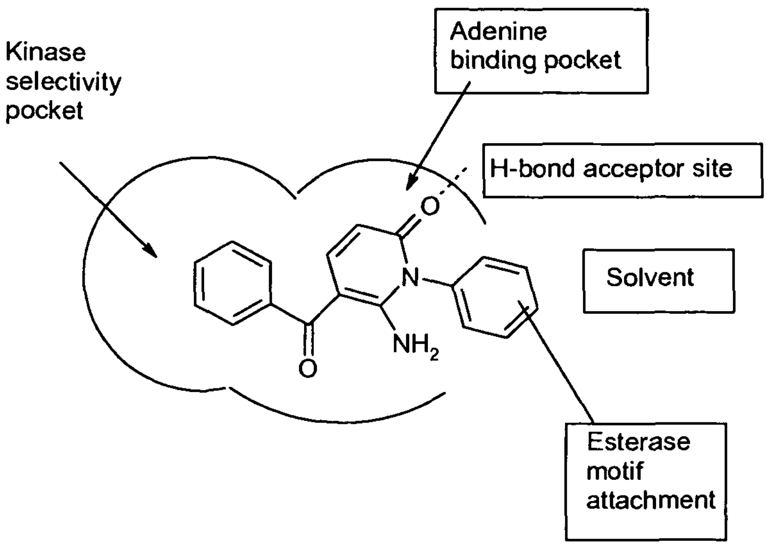

**IKK kinase (reference example)**

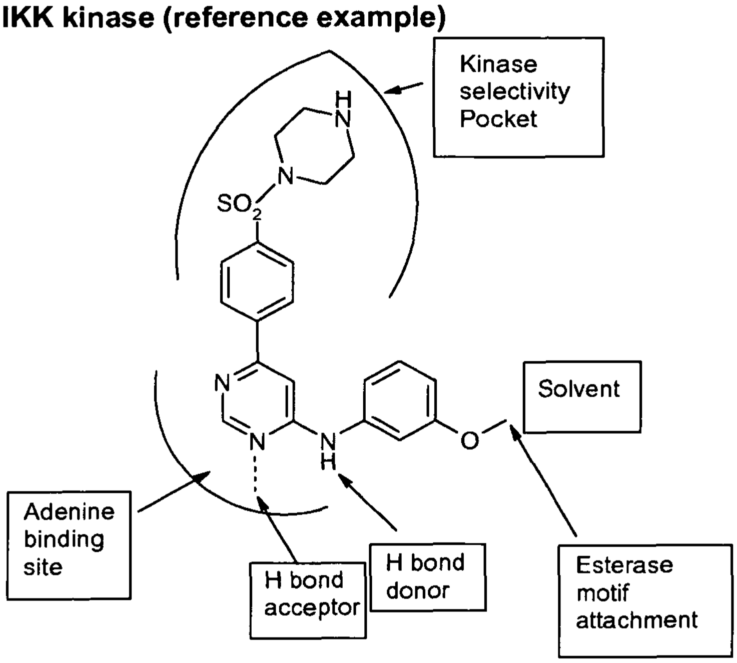


A similar approach can also be used for the other examples identified in Table 1. The method of the invention, for increasing cellular potency and/or intracellular residence time of an inhibitor of the activity of the target histone deacetylase, may involve several steps:

__Step 1:__ Identify a position or positions on a given inhibitor of the activity of the target intracellular enzyme histone deacetylase, or on a plurality of given inhibitors of the activity of the target intracellular enzyme histone deacetylase sharing the same binding mode for the target histone deacetylase enzyme, said position or positions being remote from the binding interface between the inhibitor(s) and the target histone deacetylase enzyme.

Usually such positions are identified from the X-ray co-crystal structure (or structure derived by nmr) of the target histone deacetylase enzyme with a known modulator (or a close structural analogue thereof) bound to the enzyme, by inspection of the structure. Alternatively the X-ray crystal structure of the target histone deacetylase enzyme with the modulator docked into the active site of the enzymeis modelled by computer graphics methods, and the model is inspected The presumption is that structural modification of the modulator at positions remote from the binding interface is unlikely to interfere significantly with the binding of the modulator to the active site of the enzyme. Suitable positions will normally appear from the co-crystal structure or docked model to be orientated towards solvent.

__Step 2:__ Covalently modify the inhibitor(s) by attachment of an alpha amino acid ester radical, or a range of different alpha amino acid ester radicals at one or more of the positions identified in Step 1.

Attachment of alpha amino acid ester radicals (ie the potential carboxylesterase motifs) may be via an existing covalent coupling functionality on the modulator(s), or via a suitable functionality specifically introduced for that purpose. The carboxylesterase motifs may be spaced from the main molecular bulk by a spacer or linker element, to position the motif deeper into solvent and thereby reduce still further any small effect of the motif on the binding mode of the modulator and/or to ensure that the motif is accessible to the carboxylesterase by reducing steric interference that may result from the main molecular bulk of the modulator.

Performance of Step 2 results in the preparation of one or, more usually, a small library of candidate modulators, each covalently modified relative to its parent inhibitor by the introduction of a variety of amino acid ester radicals, at one or more points of attachment identified in Step 1.

__Step 3:__ Test the alpha amino acid-conjugated modulator(s) prepared in step 2 to determine their activity against the target histone deacetylase enzyme.

As is normal in medicinal chemistry, the carboxylesterase motif version(s) of the parent modulator(s), prepared as a result of performing Steps 1 and 2, are preferably tested in assays appropriate to determine whether the expected retention of modulator activity has in fact been retained, and to what degree and with what potency profile. In accordance with the underlying purpose of the invention, which is to cause the accumulation of modulator activity in cells, suitable assays will normally include assays in cell lines to assess degree of cellular activity, and potency profile, of the modified modulators. Other assays which may be employed in Step 3 include in vitro enzyme inhibition assays to determine the intrinsic activity of the modified modulator and its putative carboxylesterase hydrolysis product; assays to determine the rate of conversion of the modified modulators to the corresponding carboxylic acid by carboxylesterases; and assays

to determine the rate and or level of accumulation of the carboxylesterase hydrolysis product (the carboxylic acid) in cells. In such assays, both monocytic and non-monocytic cells, and/or a panel of isolated carboxylesterases, can be used in order to identify compounds that show cell selectivity.

If necessary or desirable, step 3 may be repeated with a different set of candidate alpha amino acid ester-conjugated versions of the parent modulator.

**Step 4:** From data acquired in Step 3, select one or more of the tested alpha amino acid ester-conjugated versions of the parent modulator(s) which cause modulation of histone deacetylase activity inside cells, are converted to and accumulate as the corresponding carboxylic acid inside cells, and which show increased or prolonged cellular potency.

The above described Steps 1-4 represent a general algorithm for the implementation of the principles of the present invention. The application of the algorithm is illustrated in Reference Example A below, applied to a known inhibitor of the intracellular enzyme dihydrofolate reductase (DHFR).

**Reference Example A**

Folic (pteroylglutamic) acid is a vitamin which is a key component in the biosynthesis of purine and pyrimidine nucleotides. Following absorption dietary folate is reduced to dihydrofolate and then further reduced to tetrahydrofolate by the enzyme dihydrofolate reductase (DHFR). Inhibition of DHFR leads to a reduction in nucleotide biosynthesis resulting in inhibition of DNA biosynthesis and reduced cell division. DHFR inhibitors are widely used in the treatment of cancer (Bertino J, J.Cin. Oncol. 11, 5-14, 1993), cell proliferative diseases such as rheumatoid arthritis (Cronstein N., Pharmacol. Rev. 57, 163-1723), psoriasis and transplant rejection. DHFR inhibitors have also found use as antiinfective (Salter A., Rev. Infect. Dis. 4,196-236, 1982) and antiparasitic agents (Plowe C. BMJ 328, 545-548, 2004).

Many types of DHFR inhibitor compounds have been suggested, and several such compounds are used as anti-cancer, anti-inflammatory, anti-infective and anti- parasitic agents. A general templates for known DHFR inhibitors is shown below:

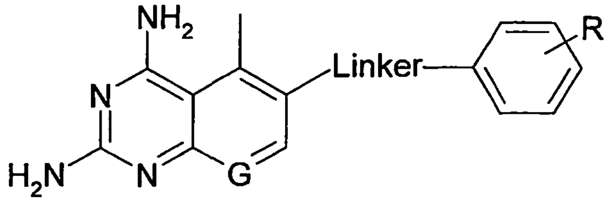

G = N or CH

**1**

Methotrexate (S)-2-(4-(((2,4-diaminopteridin-6-yl)methyl)methylamino)-benzamido)pentanedioic acid is the most widely used DHFR inhibitor and contains a glutamate functionality which enables it to be actively transported into, and retained inside, cells. However cancer cells can become resistant to methotrexate by modifying this active transport mechanism. Furthermore non-mammalian cells lack the active transport system and methotrexate has limited utility as an anti-infective agent. Lipophilic DHFR inhibitors such as trimetrexate (2,4-diamino-5-methyl-6-[(3,4,5-trimethoxyanilino)methyl]quinazoline) (2 G = CH) (GB patent 1345502) and analogues such as (2 G = N) (Gangjee et al J.Med.Chem. 1993, 36, 3437-3443) which can be taken up by passive diffusion have therefore been developed both to circumvent cancer cell resistance and for use as anti-infective agents.

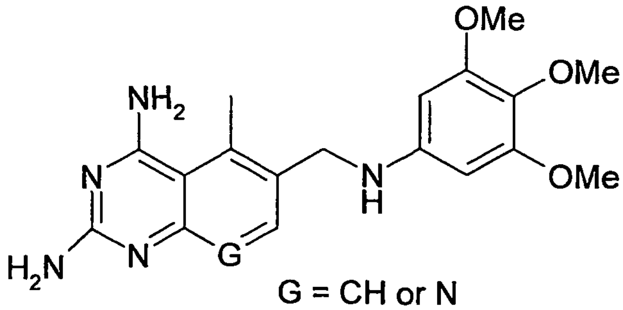

G = CH or N

2

However agents that passively diffuse into cells will also exit the cell readily and are not readily retained inside the cell. Thus a DHFR inhibitor modified in accordance with the present invention, that is lipophilic but whose activity accumulates inside the cell could have significant advantages. Furthermore both classes of DHFR inhibitors have side effects which limit the doses that can be used in the clinic. A DHFR inhibitor whose activity accumulates selectively in macrophages could have value as macrophages, via the production of cytokines, are known to play a key role in inflammatory disorders and evidence is increasing that they have a negative role in cancer.

__Step 1 of the General Algorithm Described Above__

The nmr structure of DHFR with trimetrexate (2 G = CH) docked in the active site is published (Polshakov, V.I. et al, Protein Sci. 1999, 8 ,467-481) and it is apparent that the most appropriate position to append a carboxylesterase motif in accordance with the invention was on the phenyl ring as shown below. It was inferred that attachment at that point in known close structural analogues of trimetrexate, such as (2 G = N) would also be suitable. Fgure 2 shows (2 G = N) docked into DHFR showing that a suitable point for attachment is the 4 position of the aromatic ring since this points away from the active site of the enzyme.

__Step 2 of the General Algorithm Described Above__

Compounds in which the carboxylesterase motif is linked via its alpha amino acid nitrogen were prepared as shown in schemes I and II. Within this series compounds with and without a carbonyl were made to identify potential macrophage selective compounds.

Scheme I

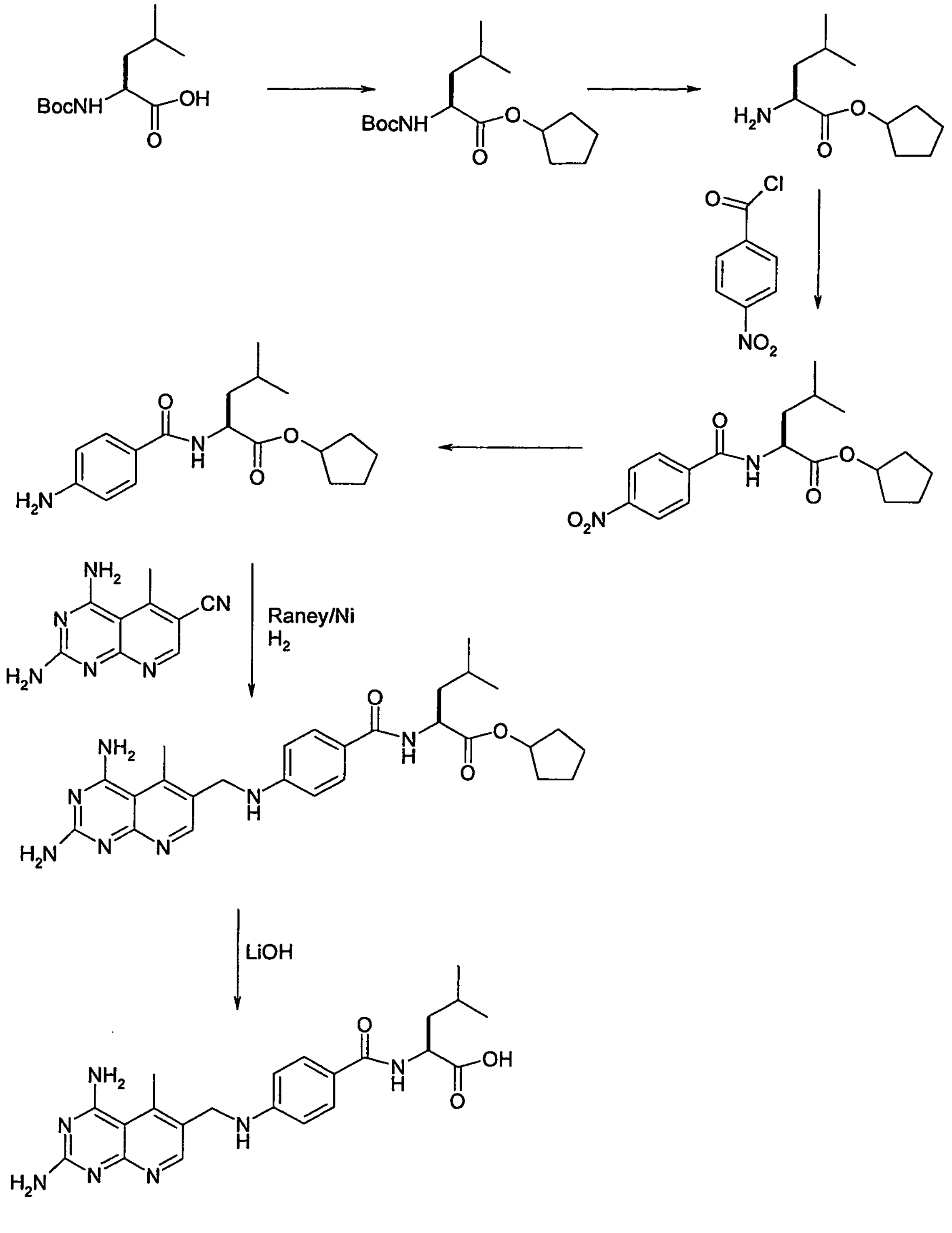

## Scheme II

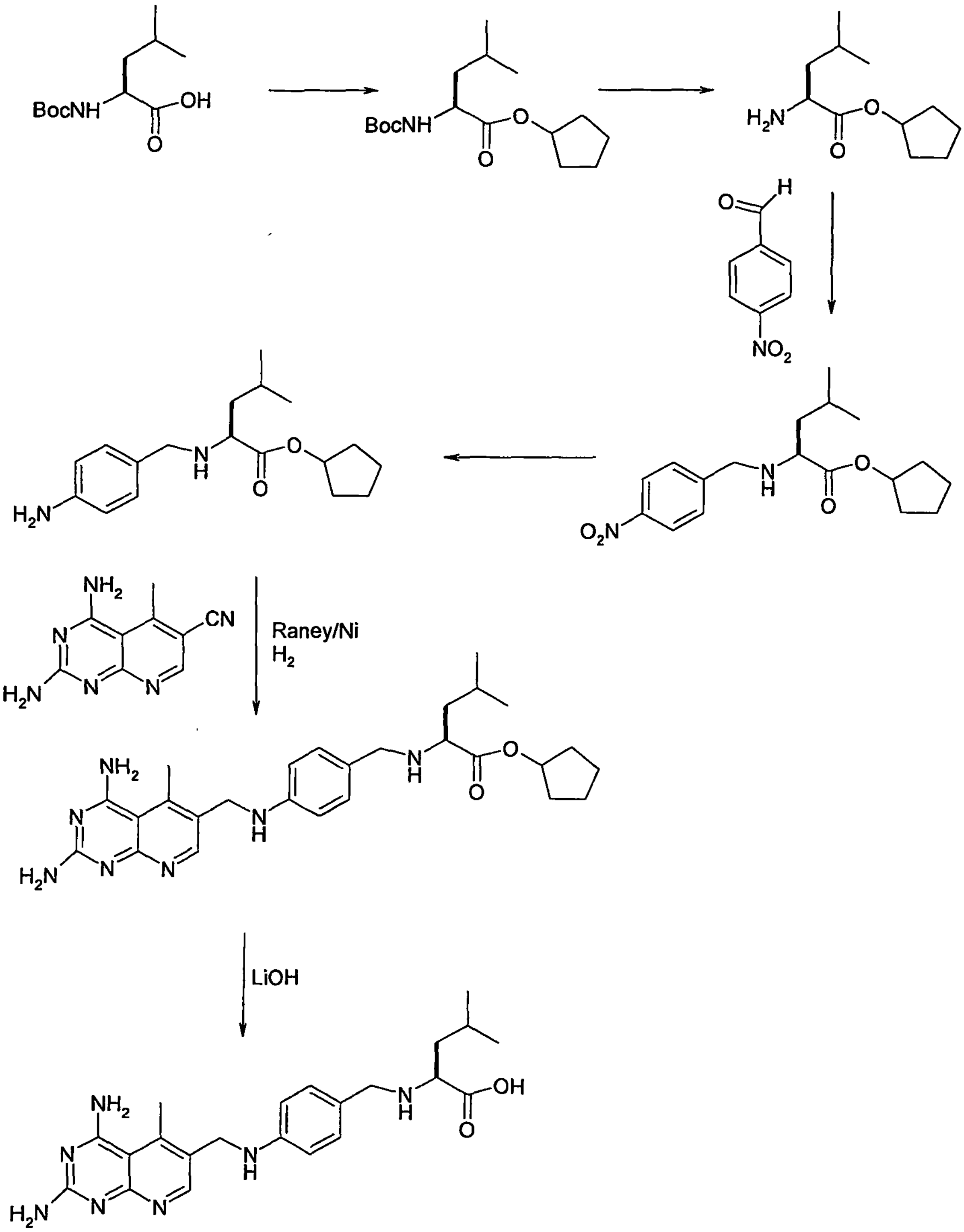


Compounds were also made in which the esterase motif was linked to the modulator via the alpha amino acid side chain schemes III and IV. Within this series compounds with alkyl substitution on the nitrogen were also prepared to identify macrophage selective compounds (scheme IV).

Scheme III

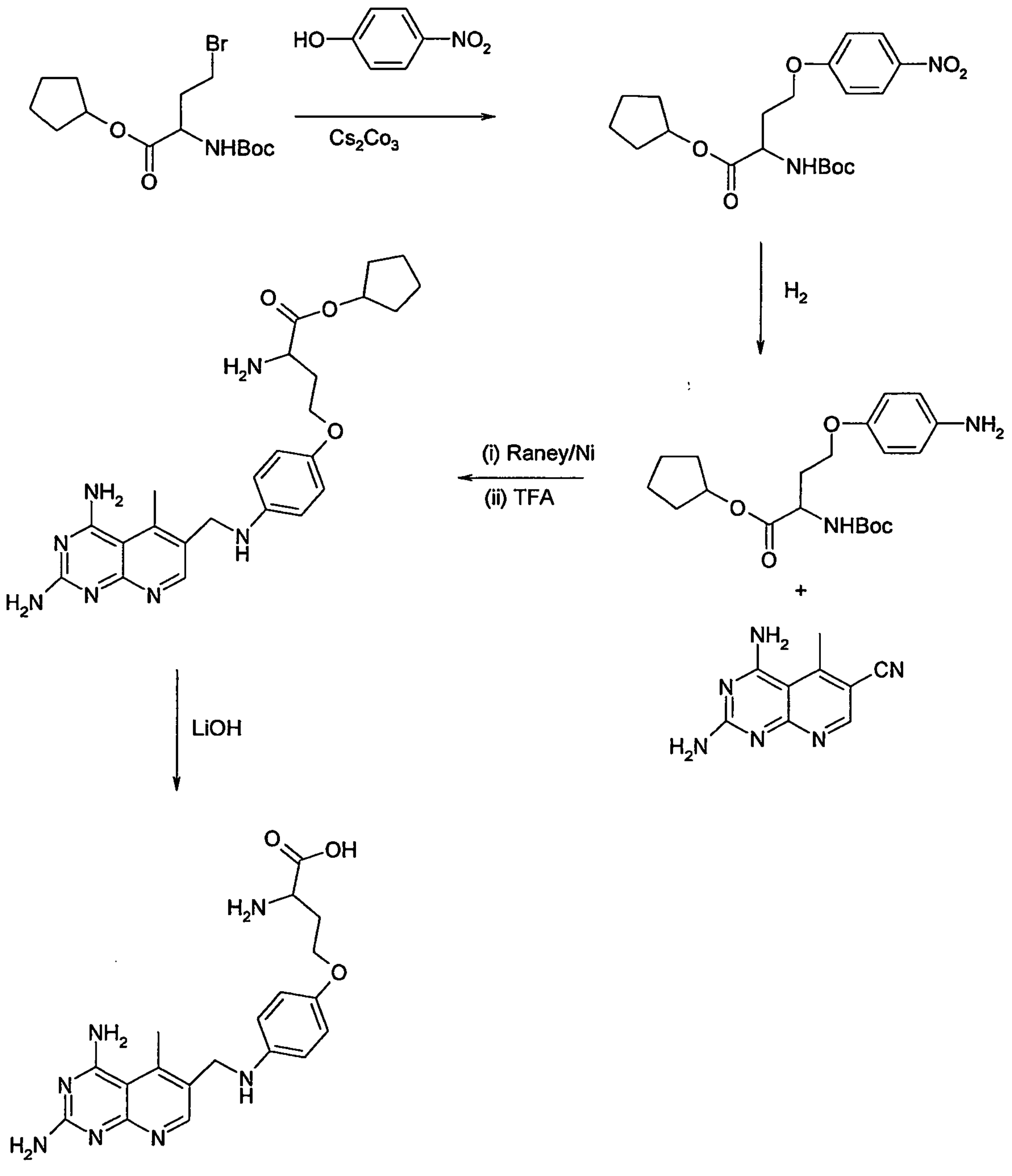

Scheme IV

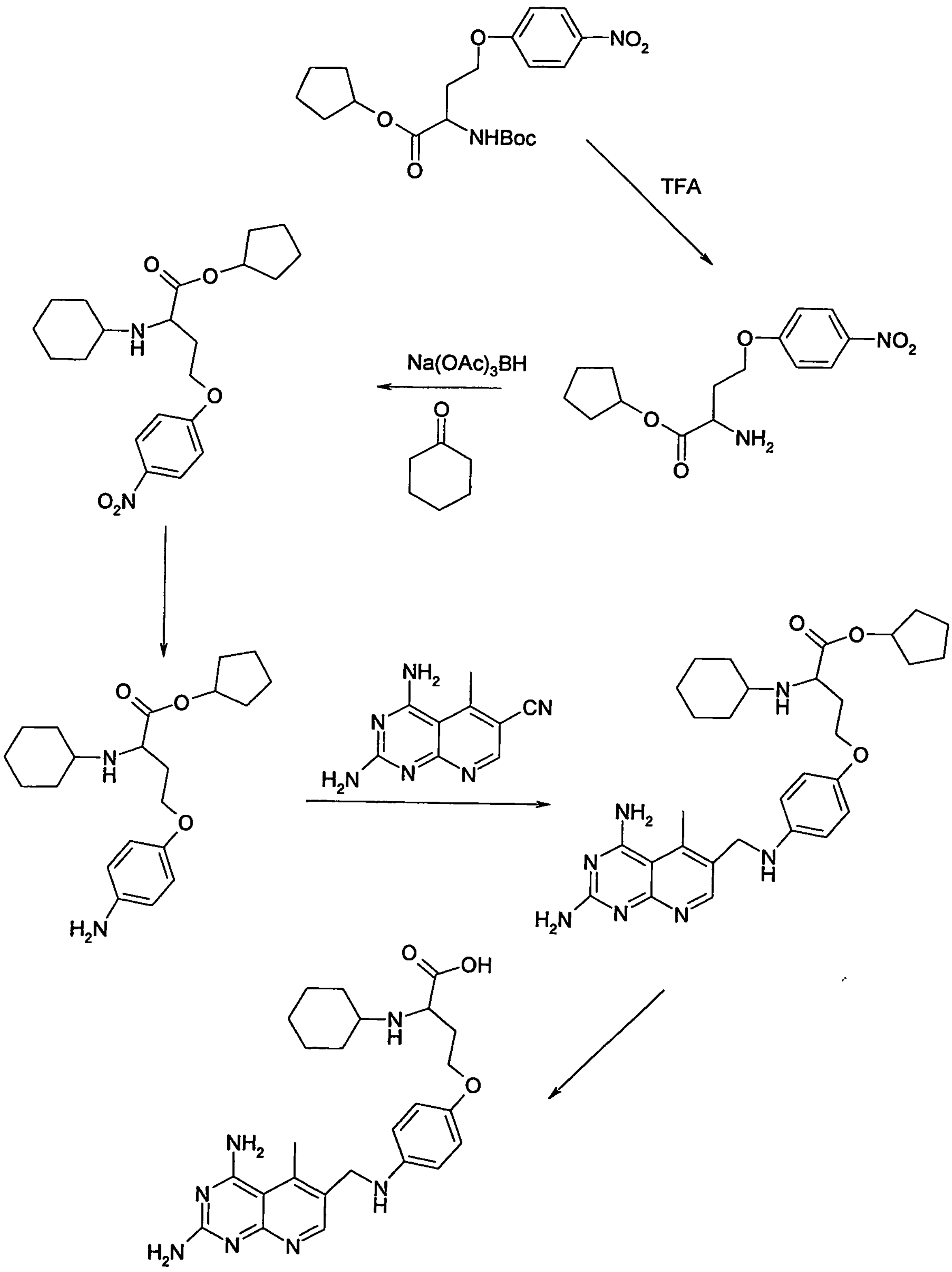


<u>**Step 3 of the General Algorithm Described Above**</u>

The compounds including the trimetrexate analogue (2 G = N) were tested in the DHFR enzyme assay, the cell proliferation assay, using both monocytic and non-monocytic cell lines, and the broken cell assay in order to assess the cleavability of the esters by monocytic and non-monocytic cell lines . Details of all these assays are given below.

**Step 4 of the General Algorithm Described Above**

As shown in Table 2 compounds were identified whose acids have activity against the enzyme comparable to the trimetrexate analogue (2 G = N). It can also be seen that by altering the way in which the esterase motif is linked can lead to a compound (6) that is 100 fold more potent in U937 cells and 15 fold more potent in the HCT116 than the unmodified analogue (2 G=N).

In addition modifications of either the linker or the substituent on the esterase motif nitrogen allowed identification of compounds 4 and 5 that showed selective cleavage in monocytic but not non-monocytic cell lines and which were significantly more anti-proliferative in monocytic cell lines then non-monocytic cell lines (see table 2).

Table 2

| | | U937 (Monocytic cell line) | | | HCT116 (non-monocytic cell line) | | |
|---|---|---|---|---|---|---|---|
| Compound | IC50 nM enzyme [1] (acid) | IC50 nM cell proliferation | Ratio IC50 cell/ enzyme | Acid Produced [2]ng/ml | IC50 nM cell proliferation | Ratio IC50 Cell/ enzyme | Acid produced [2]ng/ml |
| (2 G=N) 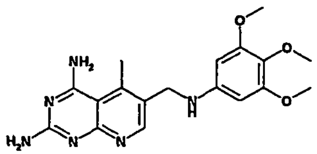 | 10 | 2200 | 220 | NA | 1700 | 170 | NA |
| 3 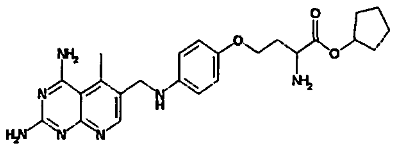 | 2700 (11) | 5100 | 1.9 | 80 | 7300 | 1.4 | 180 |
| 4 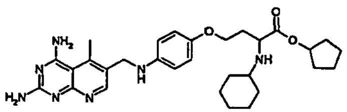 | 1033 (25) | 310 | 0.3 | 970 | 6900 | 6.6 | 40 |
| 5 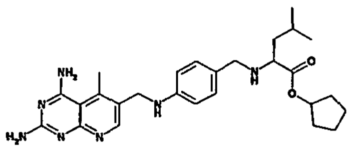 | 4000 (10) | 310 | 0.8 | 210 | 6700 | 1.7 | 2 |

(continued)

| | | U937 (Monocytic cell line) | | | HCT116 (non-monocytic cell line) | | |
|---|---|---|---|---|---|---|---|
| Compound | IC50 nM enzyme [1] (acid) | IC50 nM cell proliferation | Ratio IC50 cell/ enzyme | Acid Produced [2]ng/ml | IC50 nM cell proliferation | Ratio IC50 Cell/ enzyme | Acid produced [2]ng/ml |
| 6 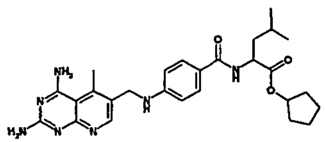 | 1700 (8) | 23 | 0.013 | 110 | 110 | 0.04 | 150 |

**Notes** [1]The figures in brackets refer to the enzyme IC50s for the acid resulting from cleavage of the esters
[2]The amount of acid produced after incubation of the ester for 80 minutes in the broken cell carboxylesterase assay described below

Using similar strategies the concept has successfully been applied to a range of intracellular targets as outlined in the examples below.

By way of further illustration of principles of this invention the following Examples are presented. In the compound syntheses described below:

Commercially available reagents and solvents (HPLC grade) were used without further purification.
Microwave irradiation was carried out using a CEM Discover focused microwave reactor. Solvents were removed using a GeneVac Series I without heating or a Genevac Series II with VacRamp at 30° C.
Purification of compounds by flash chromatography column was performed using silica gel, particle size 40-63 μm (230-400 mesh) obtained from Silicycle. Purification of compounds by preparative HPLC was performed on Gilson systems using reverse phase ThermoHypersil-Keystone Hyperprep HS C18 columns (12 μm, 100 X 21.2 mm), gradient 20-100% B (A= water/ 0.1 % TFA, B= acetonitrile/ 0.1% TFA) over 9.5 min, flow = 30 ml/min, injection solvent 2:1 DMSO:acetonitrile (1.6 ml), UV detection at 215 nm.

$^1$H NMR spectra were recorded on a Bruker 400 MHz AV spectrometer in deuterated solvents. Chemical shifts (δ) are in parts per million. Thin-layer chromatography (TLC) analysis was performed with Kieselgel 60 $F_{254}$ (Merck) plates and visualized using UV light. Analytical HPLCMS was performed on Agilent HP1100, Waters 600 or Waters 1525 LC systems using reverse phase Hypersil BDS C18 columns (5 μm, 2.1 X 50 mm), gradient 0-95% B (A= water/ 0.1 % TFA, B= acetonitrile/ 0.1 % TFA) over 2.10 min, flow = 1.0 ml/min. UV spectra were recorded at 215 nm using a Gilson G1315A Diode Array Detector, G1214A single wavelength UV detector, Waters 2487 dual wavelength UV detector, Waters 2488 dual wavelength UV detector, or Waters 2996 diode array UV detector. Mass spectra were obtained over the range m/z 150 to 850 at a sampling rate of 2 scans per second or 1 scan per 1.2 seconds using Micromass LCT with Z-spray interface or Micromass LCT with Z-spray or MUX interface. Data were integrated and reported using OpenLynx and OpenLynx Browser software
The following abbreviations have been used:

MeOH = MeOH
EtOH = EtOH
EtOAc = EtOAc
Boc = tert-butoxycarbonyl
DCM = DCM
DMF = dimethylformamide
DMSO = dimethyl sulfoxide
TFA = trifluoroacetic acid
THF = tetrahydrofuran
$Na_2CO_3$ = sodium carbonate
HCl = hydrochloric acid
DIPEA = diisopropylethylamine
NaH = sodium hydride
NaOH = sodium hydroxide
$NaHCO_3$ = sodium hydrogen carbonate
Pd/C = palladium on carbon
TBME = tert-butyl methyl ether
$N_2$ = nitrogen
PyBop = benzotriazole-1-yl-oxy-tris-pyrrolidino-phosphonium hexafluorophosphate
$Na_2SO_4$ = sodium sulphate
$Et_3N$ = triethylamine
$NH_3$ = ammonia
TMSCl = trimethylchlorosilane
$NH_4Cl$ = ammonium chloride
$LiAlH_4$ = lithium aluminium hydride
PyBrOP = Bromo-tris-pyrrolidino phosphoniumhexafluorophosphate
$MgSO_4$ = MgS04
$^n$BuLi = n-butyllithium
$CO_2$ = carbon dioxide
EDCl = *N*-(3-Dimethylaminopropyl)-*N'*-ethylcarbodiimide hydrochloride
$Et_2O$ = diethyl ether
LiOH = lithium hydroxide

HOBt = 1-hydroxybenzotriazole
ELS = Evaporative Light Scattering
TLC = thin layer chromatography
ml = millilitre
g = gram(s)
mg = milligram(s)
mol = moles
mmol = millimole(s)
LCMS = high performance liquid chromatography/mass spectrometry
NMR = nuclear magnetic resonance
r. t. = room temperature
min = minute(s)
h = hour(s)

## INTERMEDIATES

The following building blocks were used for the synthesis of the modified modulators:

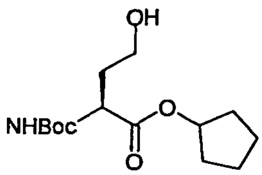

(S)-2-tert-Butoxycarbonylamino-4-hydroxy-butyric acid cyclopentyl ester

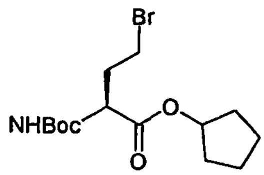

(S)-4-Bromo-2-tert-butoxycarbonylaminobutyric acid cyclopentyl ester

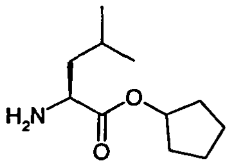

(S)-2-Amino-4-methyl-pentanoic acid cyclopentyl ester

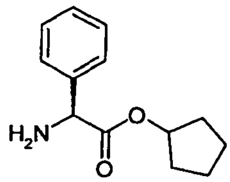

(S)-Amino-phenyl-acetic acid cyclopentyl ester

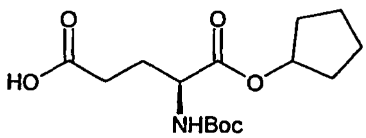

(S)-2-tert-Butoxycarbonylamino-pentanedioic acid 1-cyclopentyl ester

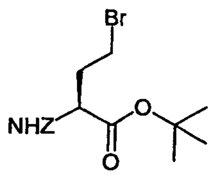

(S)-2-Benzyloxycarbonylamino-4-bromo-butyric acid tert-butyl ester

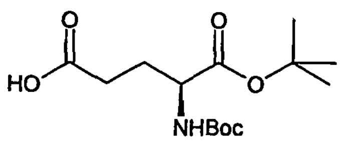

(S)-2-tert-Butoxycarbonylamino-pentanedioic acid 1-tert-butyl ester

*Synthesis of (S)-2-tert-Buloxycarbonylamino-4-hydroxy-butyric acid cyclopentyl ester and (S)-2-tert-Butoxycarbonylamino-4-hydroxy-butyric acid 1-tert-butyl ester*

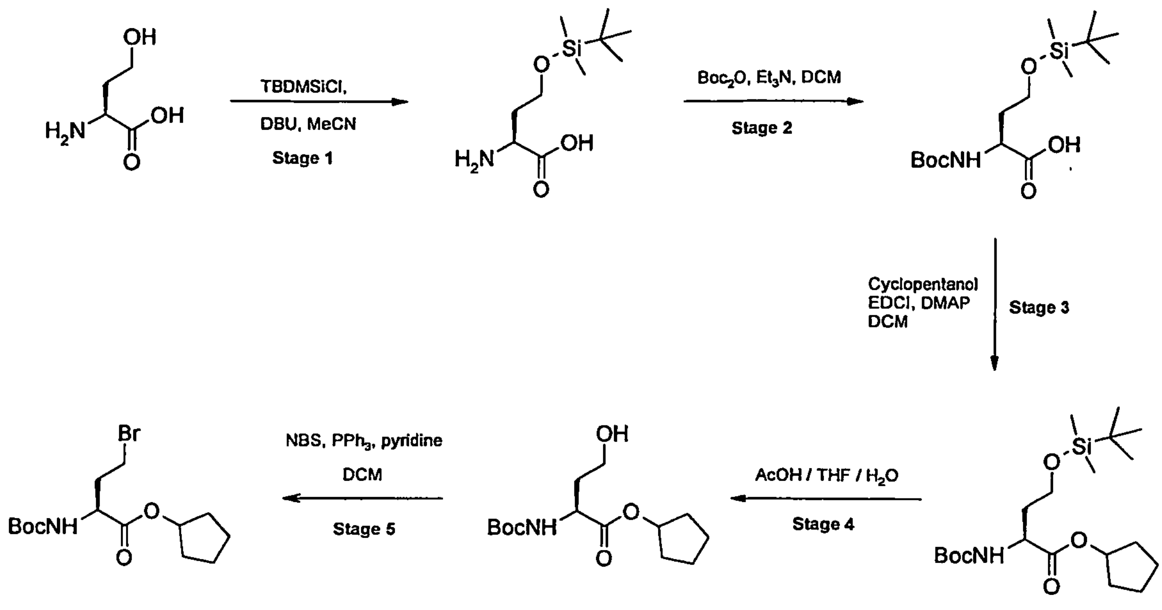


Stage 1 - Synthesis of (S)-2-Amino-4-(tert-butyl-dimethyl-silanyloxy)-butyric acid

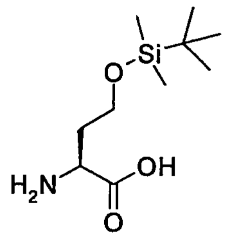

To a suspension of L-homoserine (1g, 8.4mmol) in acetonitrile (10ml) at 0°C was added 1,8-diazabicyclo[5.4.0] undec-7-ene (1.32ml, 8.8mmol, 1.05eq). Tert-butyl-dimethyl-silyl chloride (1.33g, 8.8mmol, 1.05eq) was then added portionwise over 5 min and the reaction mixture allowed to warm to r. t. and stirred for 16 h. A white precipitate had formed which was filtered off and washed with acetonitrile before drying under vacuum. The title compound was isolated as a white solid (1.8g, 92%). $^1H$ NMR (500 MHz, DMSO), δ: 7.5 (1H, bs), 3.7 (1H, m), 3.35 (4H, bm), 1.95 (1H, m), 1.70 (1H, m), 0.9 (9H, s), 0.1 (6H, s).

Stage 2 - Synthesis of (S)-2-tert-Butoxycarbonylamino-4-(tert-butyl-dimethyl-silanyloxy)-butyric acid

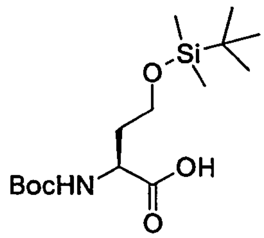

A suspension of Stage 1 product (1.8g, 7.7mmol) in DCM (100ml) at 0°C was treated with triethylamine (2.15ml, 15.4mmol, 2eq) and di-tert-butyl dicarbonate (1.77g, 8.1mmol, 1.05eq). The reaction mixture was stirred at r. t. for 16 h for complete reaction. The DCM was removed under reduced pressure and the mixture was treated with EtOAc / brine. The EtOAc layer was dried over $MgSO_4$ and evaporated under reduced pressure. The crude product was taken forward

without further purification (2.53g, 99%). $^1H$ NMR (500 MHz, CDCl3), δ: 7.5 (1H, bs), 5.85 (1H, d, J = 6.5Hz), 4.3 (1H, m), 3.75 (2H, m), 1.95 (2H, m), 1.40 (9H, s), 0.85 (9H, s), 0.1 (6H, s).

Stage 3 - Synthesis of (S)-2-tert-Butoxycarbonylamino-4-(tert-butyl-dimethyl-silanyloxy)-butyric acid cyclopentyl ester

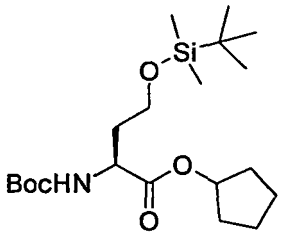

To a solution of Stage 2 product (2.53g, 7.6mmol) in DCM (50ml) at 0°C was added cyclopentanol (1.39ml, 15.3ml, 2eq), EDCI (1.61g, 8.4mmol, 1.1eq) and DMAP (0.093g, 0.76mmol, 0.1eq). The reaction mixture was stirred for 16 h at r. t. before evaporation under reduced pressure. The crude residue was dissolved in EtOAc (100ml) and washed with 1 M HCl, 1 M $Na_2CO_3$ and brine. The organic layer was then dried over $MgSO_4$ and evaporated under reduced pressure. The product was purified by column chromatography using EtOAc / heptane (1:4) to yield the title compound (2.24g, 73%). LCMS purity 100%, m/z 402.5 [$M^+$+H], $^1H$ NMR (250 MHz, CDCl3), δ: 5.2 (1H, d, J = 6.3Hz), 5.15 (1H, m), 4.2 (1H, m), 3.6 (2H, m), 2.0 (1H, m), 1.95-1.55 (9H, bm), 1.4 (9H,s), 0.85 (9H,s), 0.1 (6H,s).

Stage 4 - Synthesis of (S)-2-tert-Butoxycarbonylamino-4-hydroxy-butyric acid cyclopentyl ester

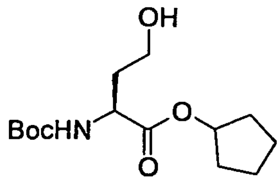

Stage 3 product (1.57g, 3.9mmol) was dissolved in acetic acid:THF:water (3:1:1, 100ml). The reaction mixture was stirred at 30°C for 16 h for complete reaction. EtOAc (200ml) was added and washed with 1 M $Na_2CO_3$, 1 M HCl and brine. The EtOAc extracts were dried over $MgSO_4$ and evaporated under reduced pressure to give the product as a clear oil which crystallised on standing (1.0g, 95%). LCMS purity 100%, m/z 310.3 [$M^+$+Na], $^1H$ NMR (250 MHz, CDCl3), δ: 5.4 (1H, d, J = 6.5Hz), 5.2 (1H, m), 4.4 (1H, m), 3.65 (2H, m), 2.15 (1H, m), 1.9-1.55 (9H, bm), 1.45 (9H, s).

Stage 5 - Synthesis of (S)-4-Bromo-2-tert-butoxycarbonylamino -butyric acid cyclopentyl ester

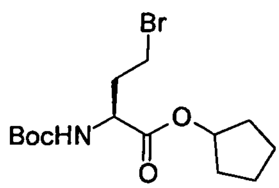

To a slurry of N-bromo succinimide (1.86g, 10.4mmol) in DCM (16.2ml) was added a solution of triphenyl phosphine (2.56g, 9.74mmol) in DCM (7.2ml). The solution was stirred for a further 5 min after addition. Pyridine (338μl, 4.18mmol) was added, followed by a solution of Stage 4 product (1.00g, 3.48mmol) in DCM (8.8ml). The solution was stirred for 18 h, concentrated under reduced pressure and the residual solvent azeotroped with toluene (3 x 16ml). The residue was triturated with diethyl ether (10ml) and ethyl acetate : heptane (1:9, 2 x 10ml). The combined ether and heptane solutions was concentrated onto silica and purified by column chromatography eluting with EtOAc / heptane

(1:9 to 2:8) to provide the title compound (1.02g, 84%). $^{1}H$ NMR (300 MHz, $CDCl_3$), δ: 5.30-5.05 (2H, m), 4.45-4.30(1H, m), 3.45 (2H, t, J = 7.3 Hz), 2.50-2.30 (1H, m), 2.25- 2.10 (1H, m), 1.95- 1.60 (8H, br m), 1.47 (9H, s).

*Synthesis of (S)-2-Amino-4-methyl-pentanoic acid cyclopentyl ester*

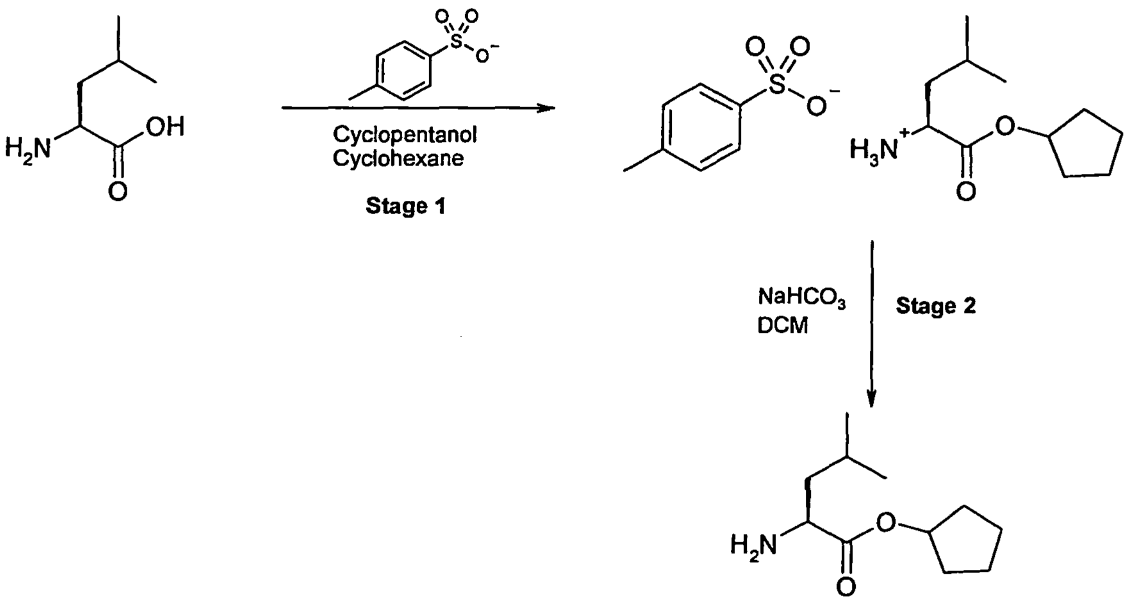


Stage 1 - Synthesis of (S)-2-Amino-4-methyl-pentanoic acid cyclopentyl ester toluene-4-sulfonic acid

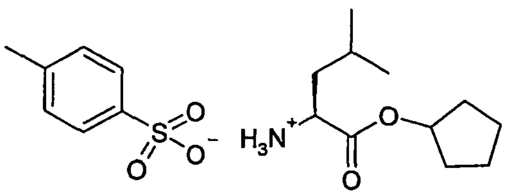

To a suspension of (S)-leucine (15g, 0.11mol) in cyclohexane (400ml) was added cyclopentanol (103.78ml, 1.14mmol) and p-toluene sulfonic acid (23.93g, 0.13mol). The suspension was heated at reflux to effect □ulphate□. After refluxing the solution for 16 h, it was cooled to give a white suspension. Heptane (500ml) was added to the mixture and the suspension was filtered to give the product as a white solid (35g, 85%). $^{1}H$ NMR (300 MHz, MeOD), δ: 1.01 (6H, t, J = 5.8Hz), 1.54-2.03 (11 H, m), 2.39 (3H, s), 3.96 (1H, t, J = 6.5Hz), 5.26-5.36 (1H, m), 7.25 (2H, d, J = 7.9Hz), 7.72 (2H, d, J = 8.3Hz).

Stage 2 - Synthesis of (S)-2-Amino-4-methyl-pentanoic acid cyclopentyl ester

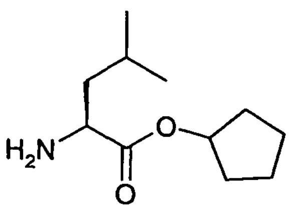

A solution of Stage 1 product (2.57g, 0.013mol) in DCM (5ml) was washed with sat. aq. $NaHCO_3$ solution (2 x 3ml). The combined aq. Layers were back extracted with DCM (3 x 4ml). The combined organic layers were dried ($MgSO_4$), and the solvent removed *in vacuo* to give the title compound as a colourless oil (1.10g, 80%). $^1H$ NMR (300 MHz, $CDCl_3$), δ: 0.90 (6H, t, J = 6.4Hz), 1.23-1.94 (11 H, m), 3.38 (1H, dd, J = 8.4, 5.9Hz), 5.11-5.22 (1H, m).

*Synthesis of (S)-Amino-phenyl-acetic acid cyclopentyl ester*

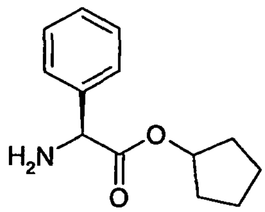

(S)-Amino-phenyl-acetic acid cyclopentyl ester was prepared from (S)-Amino-phenyl-acetic acid following the same procedure used for the synthesis of (S)-2-Amino-4-methylpentanoic acid cyclopentyl ester

*Synthesis of (S)-2-tert-Butoxycarbonylamino-pentanedioic acid 1-cyclopentyl ester*

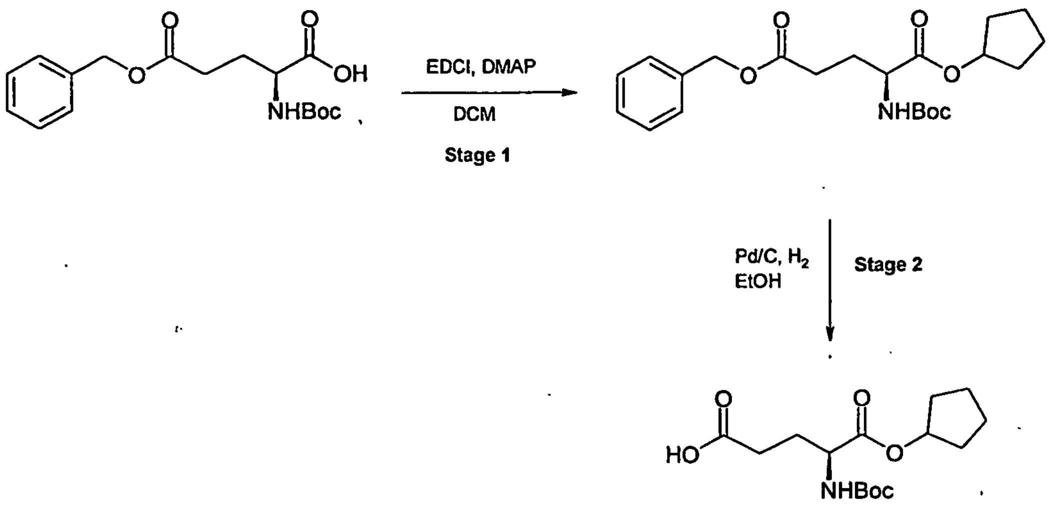


Stage 1 - Synthesis of (S)-2-tert-Butoxycarbonylamino-pentanedioic acid 5-benzyl ester 1-cyclopentyl ester

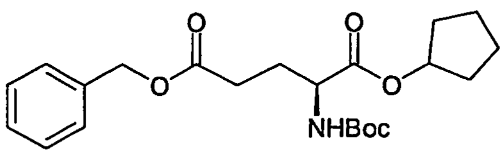

To a stirred solution of (S)-2-tert-butoxycarbonylamino-pentanedioic acid 5-benzyl ester (5g, 14.8mmol) in a mixture of DCM (50ml) and DMF (30ml) at 0°C was added cyclopentanol (2.7ml, 29.6mmol), EDCI (4.25g, 22.2mmol) and DMAP (0.18g, 1.48mmol). Stirring was continued at r. t. overnight, after which time LCMS showed completion of reaction. DCM was removed under reduced pressure. The reaction mixture was diluted with EtOAc (200ml), washed with water (100ml), 1 M aq HCl (50ml) followed by sat aq $NaHCO_3$ (50ml). The EtOAc layer was dried ($Na_2SO_4$), filtered and concentrated *in vacuo* to give a viscous oil which solidified on standing overnight. Trituration with $Et_2O$ (2 x 10ml)

afforded the title compound as a white solid (43.78g, 80%). LCMS purity 94%.

Stage 2 - Synthesis of (S)-2-tert-Butoxycarbonylamino-pentanedioic acid 1-cyclopentyl ester

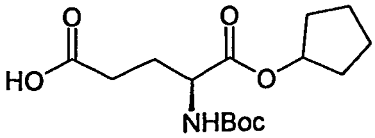

A mixture of Stage 1 product (1.3g, 3.20mmol), and 10% Pd/ C (0.5g) in EtOH (150ml) was stirred under $H_2$ (balloon) at r. t. for 4 h, after which time LCMS showed completion of reaction. The reaction mixture was filtered through a pad of celite, washed with EtOH (20ml) and concentrated *in vacuo* to give a white solid. To remove residual EtOH the solid was dissolved in toluene / THF mixture (5/1) (20ml) and concentrated *in vacuo* to yield the title compound (0.8g, 79%). $^1$H NMR (400 MHz, MeOD), δ: 1.35 (9H, s), 1.60-2.10 (10H, m), 4.05 (1H, m), 5.20 (1H, m).

*Synthesis of (S)-2-tert-Butoxycarbonylamino-pentanedioic acid 1-tert-butyl ester*

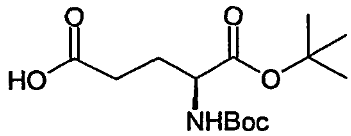

(S)-2-tert-Butoxycarbonylamino-pentanedioic acid 1-tert-butyl ester was prepared from (S)-2-tert-butoxycarbonylamino-pentanedioic acid 5-benzyl ester following the same procedure used for the synthesis of (S)-2-tert-Butoxycarbonylamino-pentanedioic acid 1-cyclopentyl ester

*Synthesis of (S)-2-Benzyloxycarbonylamino-4-bromo-butyric acid tert-butyl ester*

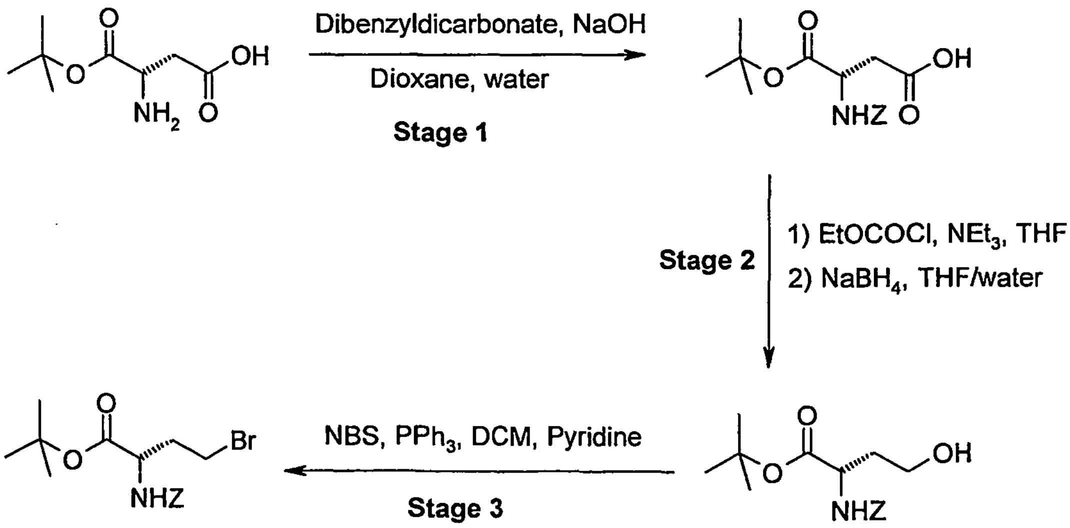


Stage 1 - Synthesis of (S)-2-Benzyloxycarbonylamino-succinic acid 1-tert-butyl ester

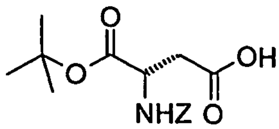

(S)-2-Amino-succinic acid 1-tert-butyl ester (0.9g, 4.75mmol) and sodium hydroxide (0.28g, 7.13mmol, 1.5eq) were dissolved in 25% water in dioxane (50ml). The solution was stirred at 5°C and dibenzyldicarbonate (2g, 4.13mmol, 1.5eq) in dioxane (10ml) was added slowly. The mixture was stirred at 0°C for 1 h and then at r. t. overnight. Water (10ml) was added and the mixture was extracted with EtOAc (2 x 20ml). The organic phase was back extracted with a sat. aq. Solution of sodium bicarbonate (2 x 10ml). The combined aq. Layers were acidified to pH 1 with 1 M HCl, and extracted with EtOAc (3 x 10ml). The combined organic fractions were dried over $MgSO_4$ and concentrated under reduced pressure. The product was purified by column chromatography (35% EtOAc in heptane) to afford the title compound as a colorless oil (0.76g, 50%). m/z 346 $[M+23]^+$, $^1H$ NMR (300 MHz, CDCl3), δ: 7.39-7.32 (5H, m), 5.72 (1H, d, J = 8.1Hz), 5.13 (2H, s), 4.58-4.50 (1H, m), 3.10-2.99 (1H, m), 2.94-2.83 91 H, m), 1.45 (9H, s).

Stage 2 - Synthesis of (S)-2-Benzyloxycarbonylamino-4-hydroxy-butyric acid tert-butyl ester

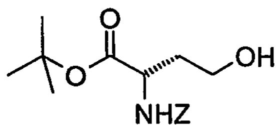

To a solution of Stage 1 product (0.6g, 1.87mmol) in anhydrous THF (20ml) at -20°C was slowly added triethylamine (0.032ml, 2.24mmol, 1.2eq) and ethyl chloroformate (0.021 ml, 2.24mmol, 1.2eq). The mixture was stirred at -20°C for 2 h. The solid formed was filtered off and washed with THF (2 x 10ml). The filtrate was added dropwise to a solution of sodium borohydride (0.2g, 5.61 mmol, 3eq) at 0°C and stirred at r. t. for 4 h. The solvent was removed under reduced pressure, the residue was diluted with water (10ml) acidified to pH 5 with 1M HCl and extracted with EtOAc. The organic fractions were combined washed with 10% aq. Sodium hydroxide, water and brine, dried on $MgSO_4$ and concentrated under reduced pressure to give the title compound as clear oil (0.3g, 51%). m/z 332 $[M+23]^+$.

Stage 3 - Synthesis of (S)-2-Benzyloxycarbonylamino-4-bromo-butyric acid tert-butyl ester

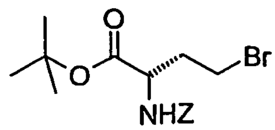

To a solution of N-bromosuccinimide (0.52g, 2.91mmol, 3eq) in DCM (10ml) was slowly added a solution of triphenylphosphine (0.71g, 2.72mmol, 2.8eq) in DCM (10ml). The mixture was stirred at r. t. for 5 min. Pyridine (0.094ml, 1.16mmol, 1.2eq) and a solution of Stage 2 product (0.3g, 0.97mmol, 1eq) in DCM (20ml) were added dropwise and the mixture stirred at r. t. overnight.

The solvent was removed under reduced pressure, the residue was azeotroped with toluene (2 x 15ml) and triturated with diethyl ether (2 x 25ml) and 10% EtOAc in heptanes. The solutions from the trituration were combined and evaporated to dryness. The crude product was purified by column chromatography (15% EtOAc in heptanes) to give the title compound as a clear oil (0.16g, 44%). m/z 395 $[M+23]^+$, $^1H$ NMR (300 MHz, CDCl3), δ: 7.39-7.30 (5H, m), 5.40 (1H, d, J = 6.8Hz), 5.12 (2H, s), 4.38 (1H, q, J = 7.7Hz), 3.47-3.38 (2H, m), 5.49-2.33 (1H, m), 2.28-2.13 (1H, m), 1.48 (9H, s).

## EXAMPLE 1

This example describes the modification of the known HDAC (Histone Deacetylase) inhibitor Suberoylanilide

hydroxamic acid (compound 7) herein referred to as "SAHA", by the attachment of amino acid ester motifs at points remote from the binding interface with the target, where no disruption of its binding mode occurs.

**Compound 7:** Suberoylanilide hydroxamic acid (SAHA)

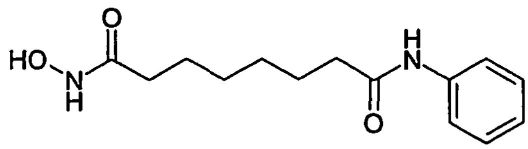

SAHA was purchased from BioCat GmbH, Heidelberg, Germany.

Standard wash procedure for resin chemistry

Resin was washed in the following sequence: DMF, MeOH, DMF, MeOH, DCM, MeOH, DCM, MeOH x 2, TBME x 2.

Resin test cleavage

A small amount of functionalised hydroxylamine 2-chlorotrityl resin (ca 0.3ml of reaction mixture, ca 10mg resin) was treated with 2% TFA/DCM (0.5ml) for 10min at r. t.. The resin was filtered and the filtrate was concentrated by blowing with a stream of $N_2$ gas. LCMS of the residue was obtained.

Preparation of Suberic acid Derivatised Hydroxylamine 2-Chlorotrityl Resin

Stage 1 - Immobilisation to 2-chlorotrityl-O-$NH_2$ resin

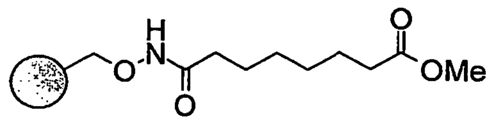

To a round bottomed flask charged with 2-chlorotrityl-O-$NH_2$ resin (6g, loading 1.14mmol/g, 6.84mmol) and DCM (60ml) was added DIPEA (5.30g, 41.0mmol, 6eq). Methyl 8-chloro-8-oxooctanoate (4.2g, 20.5mmol, 3eq) was slowly added to the reaction mixture with orbital shaking and the reaction mixture shaken for 48 h. The resin was filtered and washed using the standard washing procedure. The resin was dried under vacuum. LCMS purity was determined by ELS detection, 100%, m/z 204 $[M^{+}+H]^{+}$.

Stage 2 - Saponification

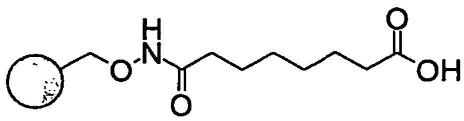

To a round bottomed flask charged with Stage 1 resin (4g, loading 1.14mmol/g, 4.56mmol) was added THF (16ml) and MeOH (16ml). To the reaction was added a solution of NaOH (0.91g, 22.8mmol, 5eq) in water (16ml). The reaction mixture was shaken for 48 h. The resin was filtered and washed with water x 2, MeOH x 2, followed by the standard wash procedure. The resin was dried under vacuum. LCMS purity was determined by ELS detection, 100% m/z 190 $[M^{+}+H]^{+}$.

Preparation of SAHA derivatives

Compounds based on SAHA were prepared by the methods outlined below.

*Compounds* **(8)**, **(9)** *and* ***(10)*** *were prepared by the methodology described in the following scheme:*

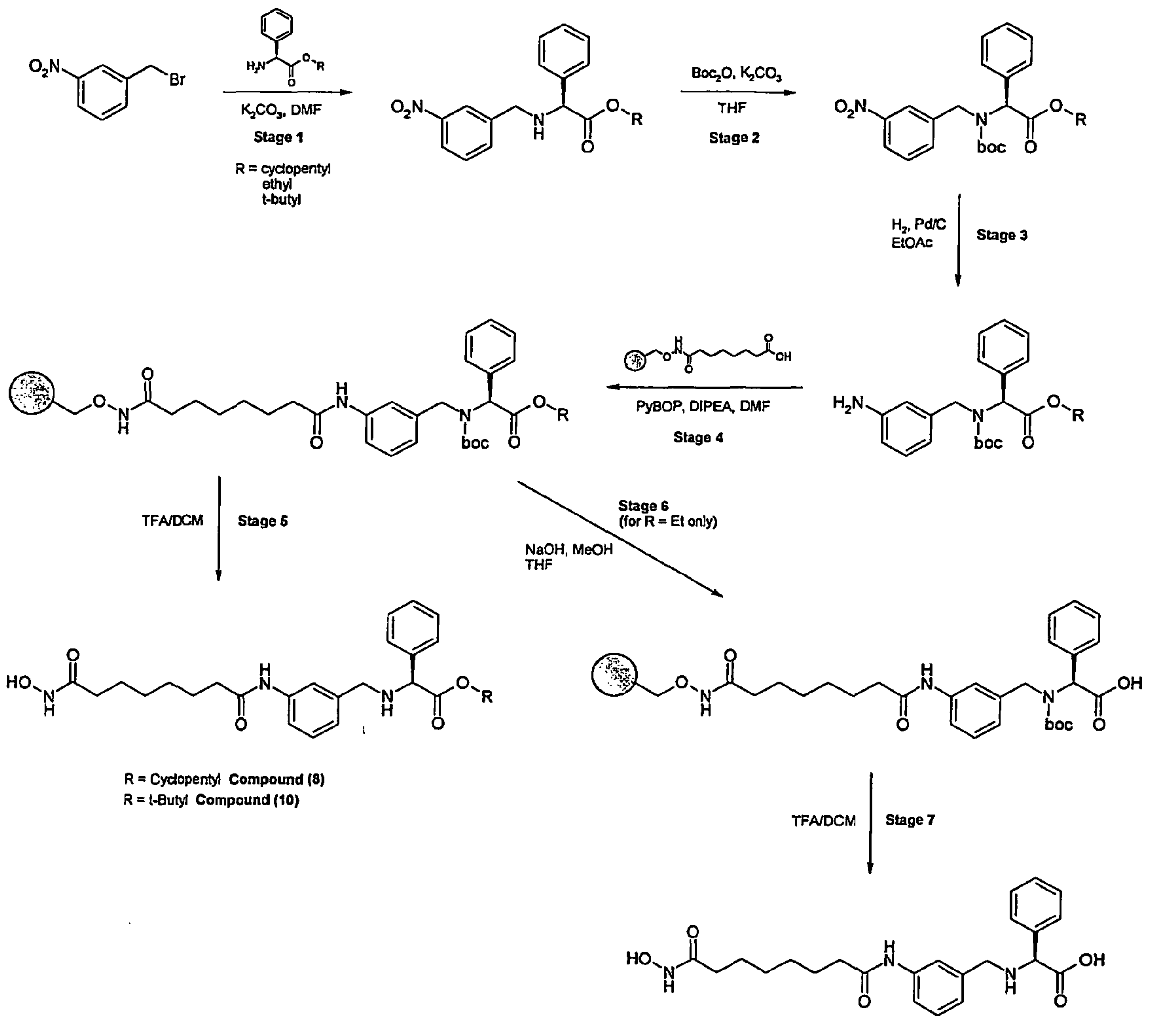


Compound (9)

*Stages 1 to* 5 *are exemplified for R = cyclopentyl*

Stage 1 - Synthesis of (S)-(3-Nitro-benzylamino)-phenyl-acetic acid cyclopentyl ester

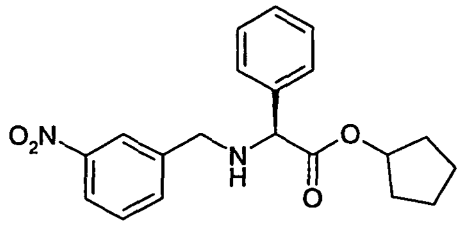

3-Nitrobenzyl bromide (46mmol) was dissolved in DMF (180ml) and potassium carbonate (92mmol) added, followed by the (S)-phenylglycine ester (10.6g, 46mmol). The reaction was stirred for 17h at r. t. before evaporating to dryness. The residue was re-dissolved in EtOAc (150ml) and washed with water (3 x 80ml), dried ($Na_2SO_4$) filtered and concentrated to dryness. After purification by flash column chromatography (30% EtOAc / hexane) the ester of was obtained and used directly in Stage 2.

Stage 2 - Synthesis of (S)-[tert-Butoxycarbonyl-(3-nitro-benzyl)-amino]-phenyl-acetic acid cyclopentyl ester

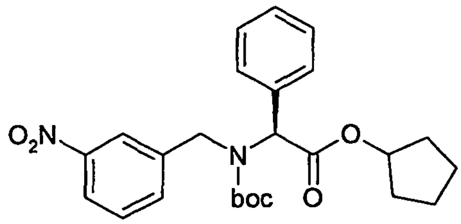

The Stage 1 product (40.9mmol) was dissolved in THF (250ml) before addition of potassium carbonate (61.4mmol) and water (150ml). Di-tert-butyl-dicarbonate (163mmol) was added and the reaction mixture heated to 50°C for 18 h. DCM was added the resultant mixture washed consecutively with 0.1 M HCl (150ml), sat. aq. $NaHCO_3$ and water (150 ml). The DCM layer was dried ($Na_2SO_4$), filtered and concentrated to dryness. After purification by flash column chromatography (5% EtOAc / hexane) the title □ulphate was isolated and used directly in Stage 3.

Stage 3 - Synthesis of (S)-[(3-Amino-benzyl)-tert-butoxycarbonyl-amino]-phenyl-acetic acid cyclopentyl ester

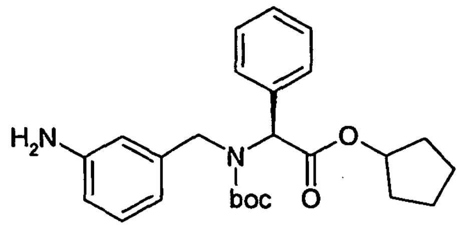

The Stage 2 product (11.5mmol) was dissolved in EtOAc (150ml) before addition of Pd/C (10% wet) catalyst (0.8g) and hydrogenated under balloon pressure at r. t. for 18 h. The reaction mixture was filtered through a pad of celite and evaporated to dryness to give a solid.

Stage 4 - Resin coupling

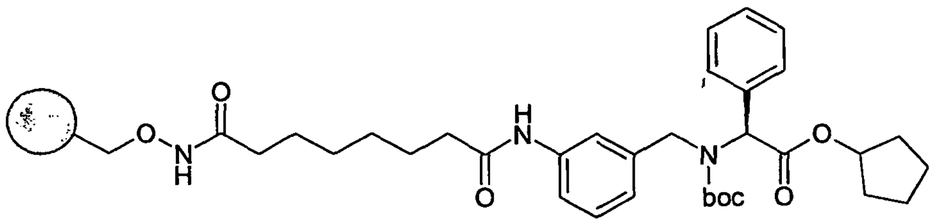

Hydroxylamine 2-chlorotrityl resin derivatized with suberic acid (1.0 g, loading 0.83mmol/g) was swollen in DMF (15ml) and PyBOP (1.36g, 2.61 mmol) added, followed by DIPEA (1.5ml, 8.7mmol). Stage 3 product (2.61mmol) was dissolved in DCM (15ml) and added to the reaction mixture. The reaction was shaken for 24 h at r. t.. The resin was filtered and washed using the standard wash procedure. The resin was dried under vacuum.

Stage 5 - Synthesis of (S)-[3-(7-Hydroxycarbamoyl-heptanoylamino)-benzylamino]-phenylacetic acid cyclopentyl ester compound (8)

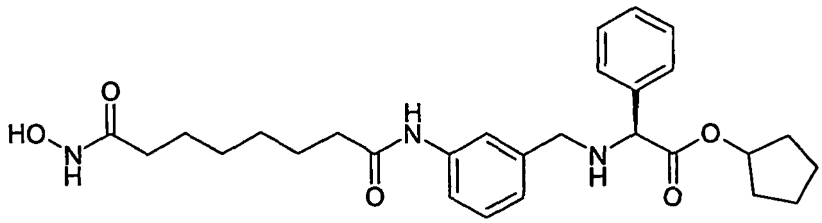

The Stage 4 product (loading 0.83mmol) was gently shaken in 2% TFA/DCM (10ml) for 20min. The resin was filtered. The filtrate was evaporated under reduced pressure at r. t.. The resin was re-treated with 2% TFA/DCM (10ml) and was filtered after 20min. The combined filtrates were evaporated to dryness under reduced pressure at r. t. to give an oily residue. The residue was allowed to stand in 20% TFA/DCM for 40 min. After evaporation to dryness, also under reduced pressure at r. t., the crude product was purified by preparative HPLC.

**Analytical data for Compound 8**

LCMS purity 100%, m/z 496 $[M^{+}+H]^{+}$, $^{1}$H NMR (400MHz, MeOD), $\delta$: 1.30-1.70 (16H, m), 2.00 (2H, t), 2.30 (2H, t), 4.05 (2H, dd), 5.00 (1H, m), 5.15 (1H, m), 7.05 (1H, m), 7.30 (2H, m), 7.40 (5H, m), 7.75 (1H, m).

**Analytical data for Compound (10)**

LCMS purity 97%, m/z 484 $[M^{+}+H]^{+}$, $^{1}$H NMR (400 MHz, MeOD), $\delta$: 1.30 (13H, m), 1.45-1.65 (4H, m), 1.93-2.05 (2H, m), 2.20-2.40 (2H, m), 3.99 (2H, q), 4.65-4.95 (1H, m) 7.05 (1H, d), 7.25-7.33 (2 H, m), 7.35-7.50 (5H, m), 7.75 (1H, s).

Stage 6 - Saponification

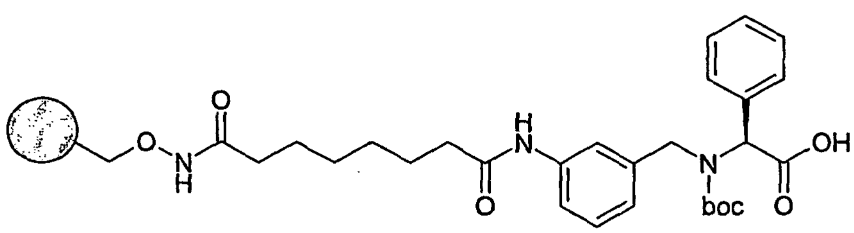

The Stage 5 product where R = Et (1.4g, loading 0.83mmol) was suspended in THF (8.6ml) and MeOH (8.6ml)

and 1.4M sodium hydroxide solution (5.98mmol) was added. The mixture was shaken for 24 h and the resin was filtered and washed with water x 2, MeOH x 2, followed by the standard wash procedure. The resin was dried under vacuum.

Stage 7 - Synthesis of (S)-[3-(7-Hydroxycarbamoyl-heptanoylamino)-benzylamino]-phenylacetic acid (9)

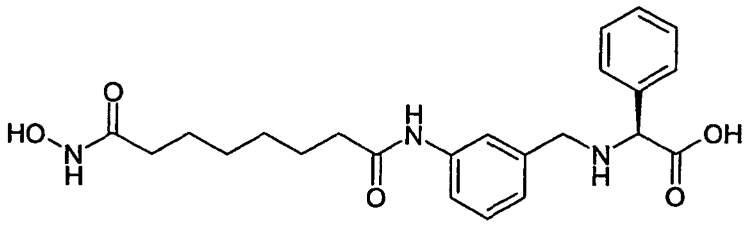

Stage 6 product (1.44g, loading 0.83mmol) was then gently shaken in 2% TFA/DCM (10ml) for 20 min. The resin was filtered and the filtrate evaporated under reduced pressure at r. t.. The resin was re-treated with 2% TFA/DCM (10ml) and was filtered after 20 min. The combined filtrates were evaporated to dryness under reduced pressure at r. t. to give an oily residue. The residue was allowed to stand in 20% TFA/DCM for 40 min. After evaporation to dryness, under reduced pressure at r. t., the crude product was purified by preparative HPLC to yield compound (9). LCMS purity 100%, m/z 428 $[M^{+}+H]^{+}$, $^{1}H$ NMR (400MHz, MeOD), $\delta$: 1.20-1.35 (4H, m), 1.50-1.65 (4H, m), 2.00 (2H, m), 2.30 (2H, m), 4.00 (2H, dd), 4.90 (1H, m), 7.05 (1H, m), 7.25-7.50 (7H, m), 7.70 (1H, m).

*Compound **(24)** was prepared following the same methodology described for the synthesis of compound (8).*

({(R)-[4-7-Hydroxycarbamoyl-heptanoylamino)-phenyl)-phenyl-methyl}-amino)acetic acid cyclopentyl ester (24)

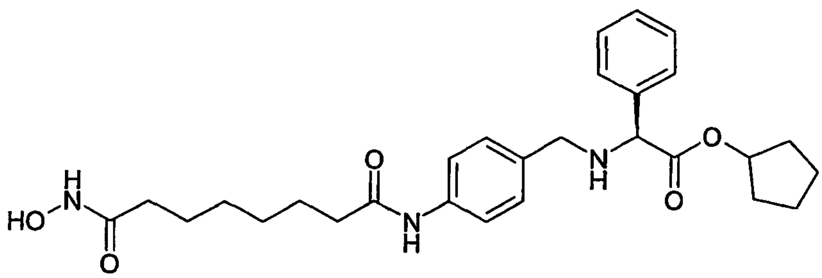

LCMS purity 95%, m/z 496 $[M^{+}+H]^{+}$, $^{1}H$ NMR (400 MHz, DMSO), $\delta$:1.30-1.50 (6H, m), 1.50-1.70 (8H, m), 1.80 (2H, m), 2.10 (2H, t), 2.45 (2H, t), 4.1 (2H, dd), 5.25 (1H, m), 5.35 (1H, m), 7.45 (2H, d), 7.60 (5H, m), 7.80 (2H, d), 10.00-10.10 (2H, br s), 10.50 (1H, s).

## REFERENCE EXAMPLE 2

This example describes the modification of the known Aurora Kinase A ("Aurora A") inhibitor N-{4-(7-methoxy-6-methoxy-quinoline-4-yloxy)-phenyl}-benzamide (compound (**11**)) by the attachment of an amino acid ester motif at a point where no disruption of its binding mode occurs.

Compound (11): N-{4-(7-methoxy-6-methoxy-quinoline-4-yloxy)-phenyl}-benzamide

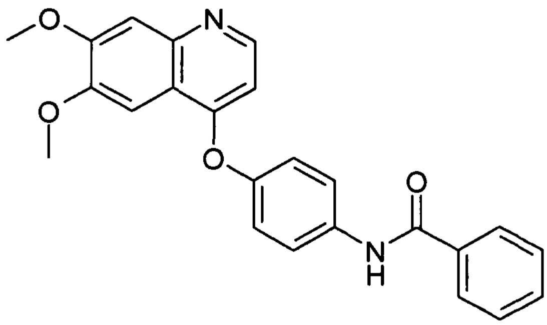

Compound (11) was prepared as described in US Patent No. 6,143,764

Compounds based on compound (11) were prepared by the methods outlined below.

*Compounds **(12)** and **(13)** were prepared by the method described in the following scheme:*

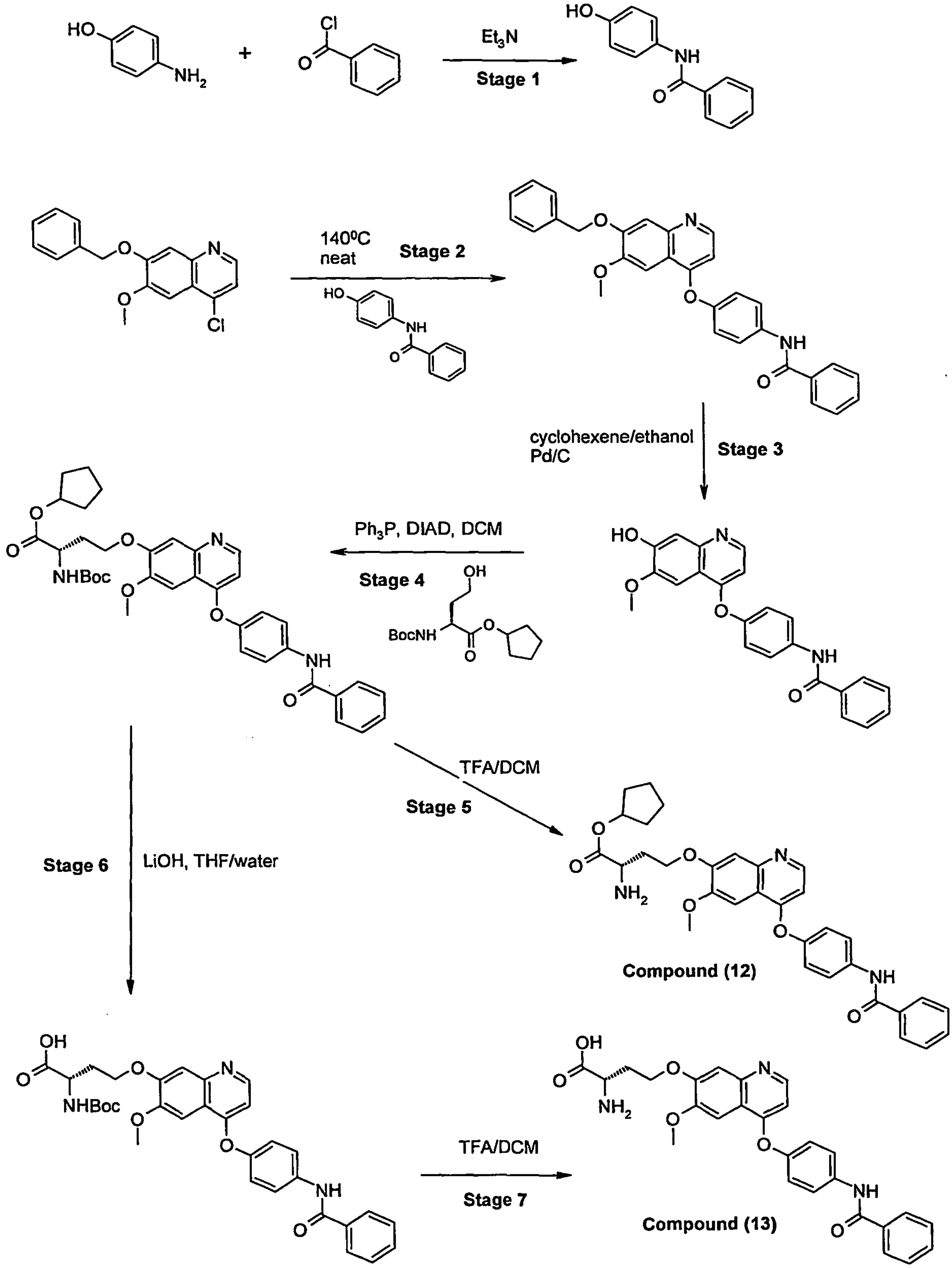


Stage 1 - Synthesis of N-(4-Hydroxy-phenyl)-benzamide

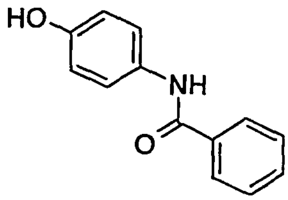

To a solution of 4-aminophenol (4.27g, 39.1 mmol) in DMF (50ml) at 0°C under an atmosphere of argon was added triethylamine (7.44ml, 53.4mmol, 1.5eq). The reaction was stirred for 10 min before slow addition of benzoyl chloride (5g, 35.6mmol, 1eq) over a period of 5 min. The reaction mixture was allowed to warm to r. t. and stirred over 18 h. The DMF was removed under reduced pressure and the mixture was treated with EtOAc/water. Precipitation of a white solid resulted, this was filtered off and dried under reduced pressure to give the title compound (8.0g, 96%). $^1H$ NMR (270 MHz, DMSO), δ: 10.0 (1H, s), 9.35 (1H, s), 7.9 (2H, d, J = 7.2Hz), 7.5 (5H, m), 6.75 (2H, d, J = 7.4Hz).

Stage 2 - Synthesis of N-[4-(7-Benzyloxy-6-methoxy-quinolin-4-yloxy)-phenyl]-benzamide

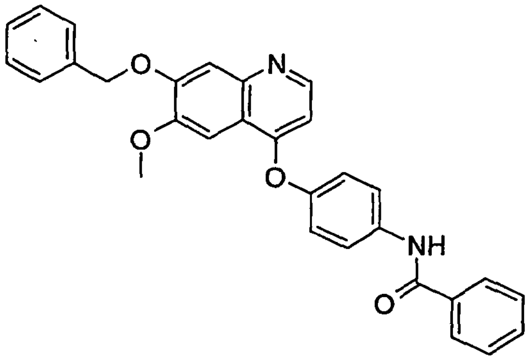

To a round bottomed flask charged with 4-chloro-6-methoxy-7-benzyloxyquinoline [see Org. Synth. Col. Vol. 3, 272 (1955) and US006143764A (Kirin Beer Kabushiki Kaisha) for methods of synthesis] (1.09g, 3.6mmol) was added Stage 1 product (2.33g, 10.9 mmol, 3 eq). Reaction was heated to 140°C for 3 h. After cooling to r. t., water was added to the reaction mixture and the mixture extracted 3 times with EtOAc. The combined EtOAc layer was washed with 5% aq. NaOH, brine and dried over $MgSO_4$. The solvent was removed under reduced pressure and purified by column chromatography eluting with EtOAc / heptane (2:1) to obtain the title compound (0.56g, 32%). m/z 477 $[M^+ + H]$.

Stage 3 - Synthesis of N-[4-(7-Hydroxy-6-methoxy-quinolin-4-yloxy)-phenyl]-benzamide

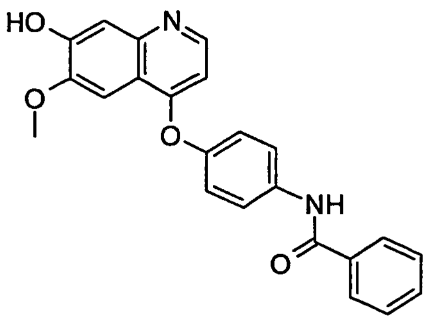

A mixture of Stage 2 product (0.56g, 1.17mmol) and 10% Pd/C (0.08g) in 10% cyclohexene / EtOH (80ml) was heated under reflux for 3 h. The Pd/C catalyst was filtered through a pad of celite, washing twice with MeOH. The filtrate was concentrated under reduced pressure to yield the title compound as a yellow solid (0.34g, 75%). m/z 387 $[M^+ + H]$.

Stage 4 - Synthesis of (S)-4-[4-(4-Benzoylamino-phenoxy)-6-methoxy-quinolin-7-yloxy]-2-tert-butoxycarbonylamino-butyric acid cyclopentyl ester

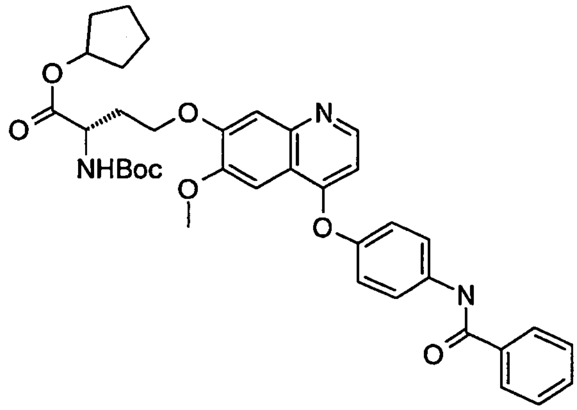

To a solution of Stage 3 product (0.2g, 0.52mmol) in anhydrous DCM (30ml) at 0°C was added (S)-2-tert-Butoxycarbonylamino-4-hydroxy-butyric acid cyclopentyl ester (0.223g, 0.78mmol, 1.5eq) in 5ml of DCM. $Ph_3P$ (0.557g, 2.1mmol, 4.1eq) and DIAD (0.412ml, 2.1mmol, 4.1 eq) were then added and the reaction mixture allowed to warm to r. t. and stirred for 16 h. The crude reaction mixture was evaporated under reduced pressure and purified by column chromatography to give the title compound (0.135g, 46%). m/z 656.3 $[M^{+}+H]$.

Stage 5 - Synthesis of ((S)-2-amino-4-[4-(4-benzoylamino-phenoxy)-6-methoxy-quinolin-7-yloxy]-butyric acid cyclopentyl ester) (12)

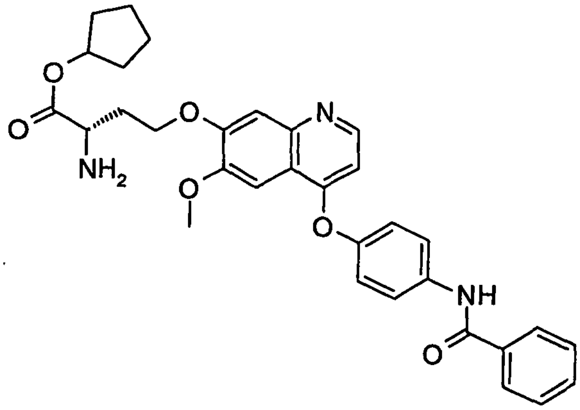

To a solution of Stage 4 product (5.8mg, 0.009mmol) in DCM (1ml) was added TFA (1ml). The reaction mixture was allowed to stir for 16 h before evaporation under reduced pressure, azeotroping with toluene to remove the traces of TFA. Compound (12) was isolated as an off-white solid (4.7mg). LCMS purity 95%, m/z 556.2 $[M^{+}+H]$, $^{1}H$ NMR (270 MHz, DMSO), $\delta$: 10.4 (1H, s), 8.8 (1H, d, J = 6.5Hz), 8.55 (2H,bs), 8.01 (4H, m), 7.65 (4H, m), 7.35 (1H, d, J = 7.6Hz), 6.75 (1H, d, J = 6.5Hz), 5.25 (1H, m), 4.35 (3H, m), 4.0 (3H, s), 2.4 (2H, m), 1.85-1.4 (8H, bm).

Stage 6 - Synthesis of (S)-4-[4-(4-Benzoylamino-phenoxy)-6-methoxy-quinolin-7-yloxy]-2-tert-butoxycarbonylamino-butyric acid

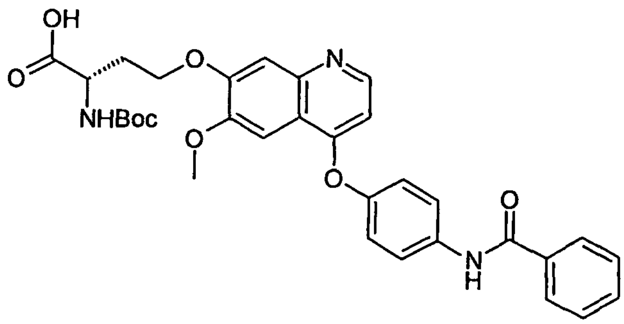

To a solution of Stage 4 product (17mg, 0.02mmol) in THF (1ml) was added 2M NaOH (0.026ml, 0.046mmol, 2eq). After 16 h, the reaction was incomplete so an additional 2 equivalents of NaOH was added. Stirring was completed after 6 h and the THF was removed under reduced pressure. The aq. Layer was diluted with 3ml of water and acidified to pH 6 with 1 M HCl. The title compound was extracted into EtOAc, dried over $MgSO_4$ and isolated as a white solid. This was used directly in Stage 7 without further purification.

Stage 7 - Synthesis of (S)-4-[4-(4-benzoylamino-phenoxy)-6-methoxy-quinolin-7-yloxy]-2-tert-butylcarbonylamino-butyric acid **(13)**

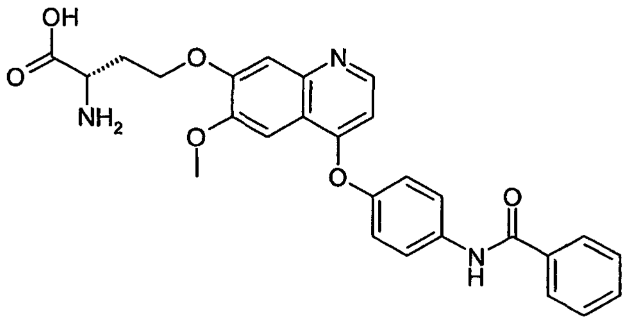

To a solution of Stage 6 product (6.5mg, 0.011mmol) in DCM (1ml) was added TFA (1ml). The reaction was allowed to stir for 6 h and then evaporated under reduced pressure to give the title compound (13) as an off-white solid (90%). LCMS purity 100%, m/z 488.2 $[M^{+}+H]$, $^{1}H$ NMR (300 MHz, MeOD), δ: 8.75 (1H, d, J = 7.8Hz), 8.00 (4H, m), 7.65 (4H, m), 7.4 (1H, d, J = 7.6Hz), 6.95 (1H, d, J = 8.0Hz), 4.6 (2H, m), 4.3 (1H, m), 4.2 (3H, s), 2.6 (2H, m).

*Compound* **(*14*)** *was prepared by the method described in the following scheme:*

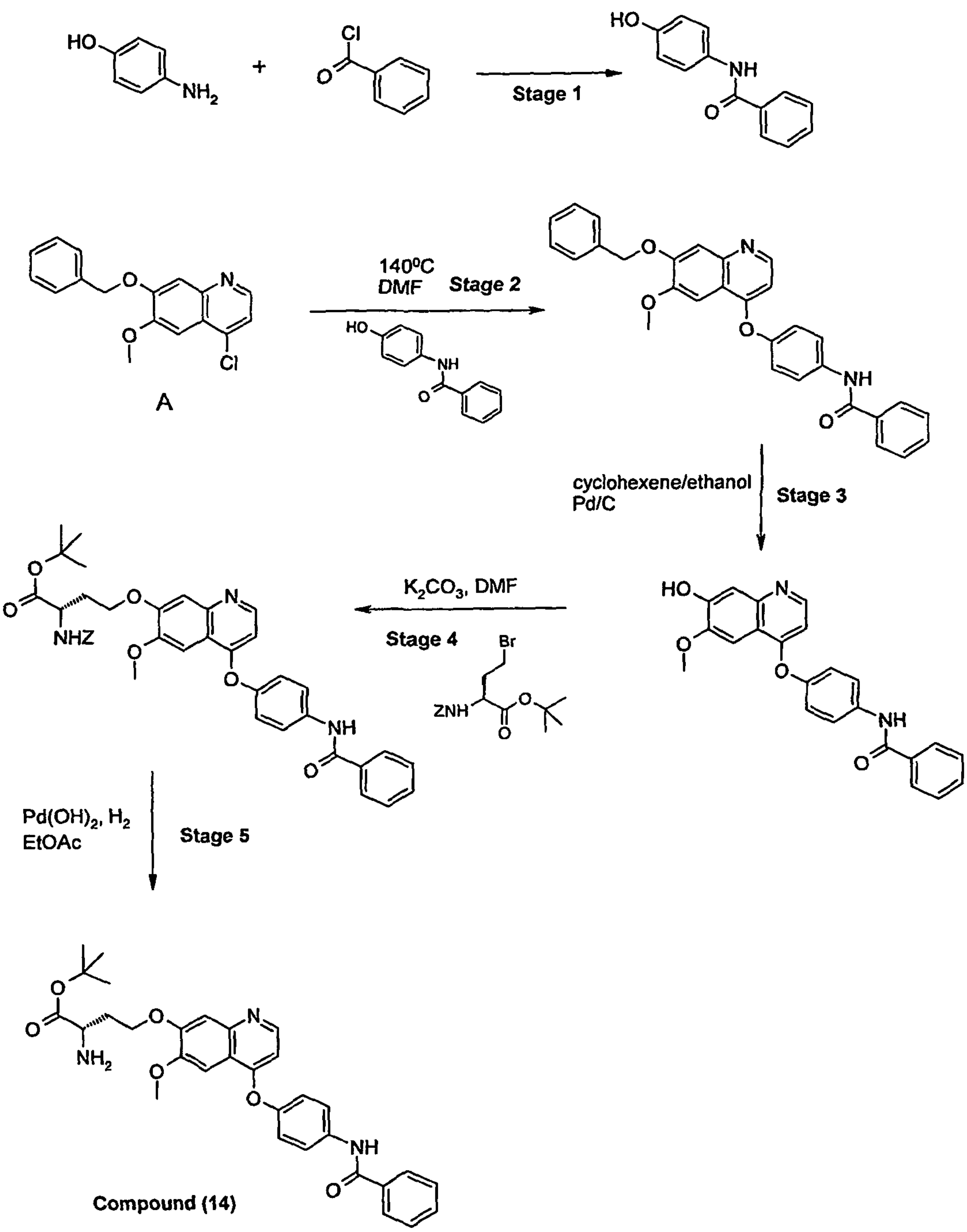


Compound (14)

Stage 1, 2 and 3 are the same as described above for the synthesis of compound **(12).**

Stage 4 - Synthesis of (S)-4-[4-(4-Benzoylamino-phenoxy)-6-methoxy-quinolin-7-yloxy]-2-benzyloxycarbonylamino-butyric acid tert-butyl ester

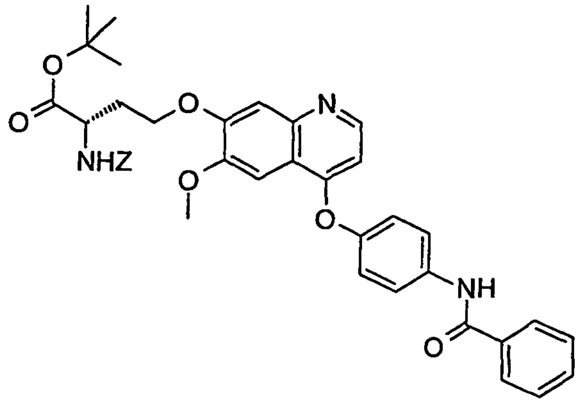

The Stage 3 product (0.15g, 0.39mmol), (S)-2-Benzyloxycarbonylamino-4-bromo-butyric acid tert-butyl ester (0.16g, 0.43mmol, 1.1 eq) and $K_2CO_3$ (0.11g, 0.78mmol, 2eq) were dissolved in anhydrous DMF (10ml) under an atmosphere of nitrogen. The reaction was stirred at 35°C overnight before the DMF was removed under reduced pressure. The residue was dissolved in DCM and washed with water followed by brine. The organic layer was dried over $MgSO_4$ and evaporated under reduced pressure. Column chromatography (eluting with 1% MeOH/ DCM) afforded the title compound (0.16g, 60%). m/z 678 $[M+H]^+$, $^1H$ NMR (300 MHz, CDCl3), δ: 8.49 (1H, d, J = 5.3Hz), 7.98 (1H, s), 7.92 (2H, dd, J = 8.2, 1.4Hz), 7.80-7.72 (2H, m), 7.63-7.48 (4H, m), 7.43-7.29 (4H, m), 7.24-7.17 (2H, m), 6.64 (1H, d, J = 8.9Hz), 6.49 (1H, d, J = 5.3Hz), 5.15 (2H, s), 4.66-4.57 (1H, m), 4.43-4.34 (1H, m), 3.85 (3H, s), 2.55-2.33 (2H, m), 1.41 (9H, m).

Stage 5 - Synthesis of -(S)-2-Amino-4-[4-(4-benzoylamino-phenoxy)-6-methoxy-quinolin-7-yloxy]-butyric acid tert-butyl ester **(14)**

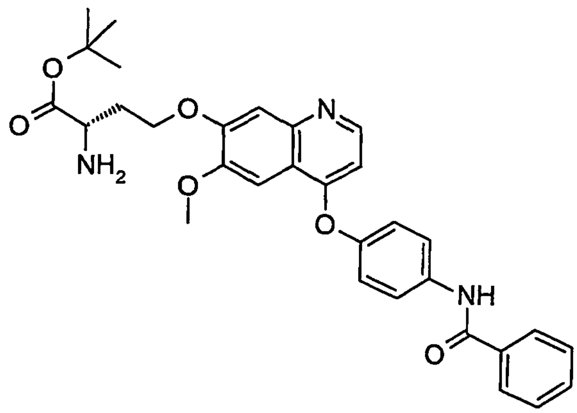

The Stage 4 product (0.045g, 0.066mmol), was dissolved in anhydrous EtOAc (5ml) and $Pd(OH)_2/C$ was added under an atmosphere of nitrogen. The reaction was degassed and stirred under an atmosphere of hydrogen at r. t. overnight. The catalyst was filtered off through a pad of celite and the solvent removed under reduced pressure. Compound **(14)** was purified by preparative HPLC. m/z 544 $[M+H]^+$, $^1H$ NMR (300 MHz, CD30D), δ: 8.67 (1H, d, J = 6.8Hz), 7.98 (4H,d, J = 8.7Hz), 7.90 (1H,s), 7.68-7.51 (4H, m), 7.42-7.36 (2H, m), 6.97 (1H, d, J = 6.6Hz), 4.52 (2H, t, J = 5.7Hz), 4.28 (1H, t, J = 6.5Hz), 4.13 (3H, s), 2.69-2.45 (2H,m), 1.53 (9H, s).

*Compound **(25)** was prepared by the method described in the following scheme:*

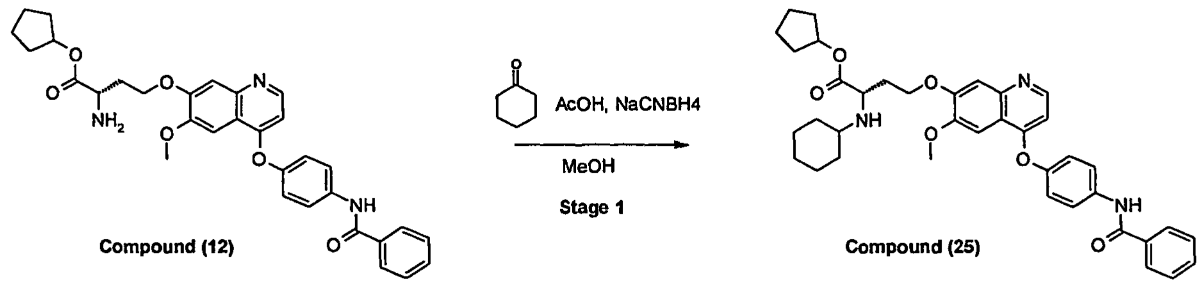


Compound (12)

Compound (25)

Stage 1 - Synthesis of (S)-4-[4-(4-Benzoylamino-phenoxy)-6-methoxy-quinolin-7-yloxy]-2-cyclohexylamino-butyric acid cyclopentyl ester **(25)**

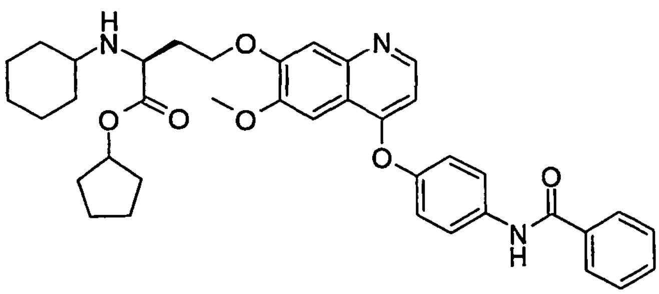

To compound **(12)** (37mg, 0.066mmol) in anhydrous MeOH (1ml) was added 100μL of a 1M solution of cyclohexanone in MeOH and 1 drop of acetic acid. The reaction mixture was stirred at r. t. for 3 h. Sodium cyanoborohydride (10.3mg, 0.165mmol) was then added and the reaction was left stirring 4 h at r. t., prior to concentration under vacuum. Purification by preparative HPLC afforded the title compound **(25)** as a di-TFA salt. m/z 638 $[M+H]^+$. $^1H$ NMR (300 MHz, $CD_3OD$) δ: 8.72 (1H, d, J = 6.8 Hz), 8.02-7.98 (4H, m), 7.93 (1H, s), 7.67 (1H, s), 7.66-7.53 (3H, m), 7.42 (2H, m), 6.99 (1H, d, J = 6.8 Hz), 5.38 (1H, m), 4.49 (3H, m), 4.14 (3H, s), 3.27 (11 H, m), 2.66 (2H, m), 2.20 (2H, m), 12.05-1.46 (16H, m).

## REFERENCE EXAMPLE 3

This example describes the modification of the known P38 kinase inhibitor 6-Amino-5-(2,4-difluoro-benzoyl)-1-(2,6-difluoro-phenyl)-1H-pyridin-2-one (compound **3258**) by the attachment of an amino acid ester motif at a point where no disruption of its binding mode occurs.

**Compound (15):** 6-Amino-5-(2,4-difluoro-benzoyl)-1-(2,6-difluoro-phenyl)-1H-pyridin-2-one

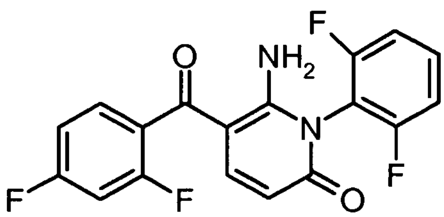

Compound **(15)** was prepared as described in WO03/076405.

Compounds based on compound **(15)** were prepared by the methods outlined below.

*Compounds (16) and **(17)** were prepared by the method described in the following scheme:*

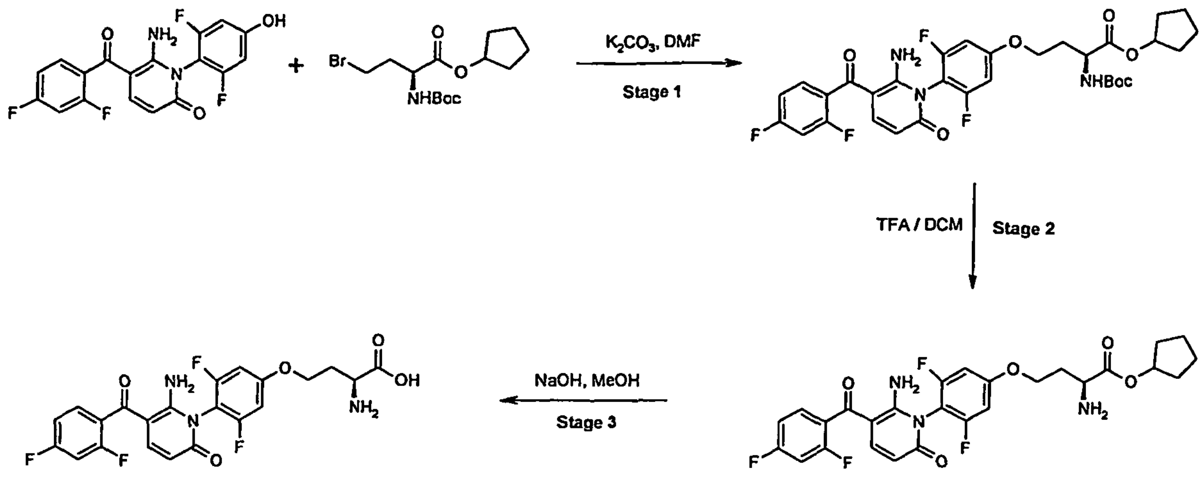


**Compound (17)** **Compound (16)**

Stage 1 - Synthesis of cyclopentyl (S)-4-{4-[6-Amino-5-(2,4-difluorobenzoyl)-2-oxo-2H-pyridin-1-yl]-3,5-difluorophenoxy}-2-tert-butoxycarbonylaminobutyrate

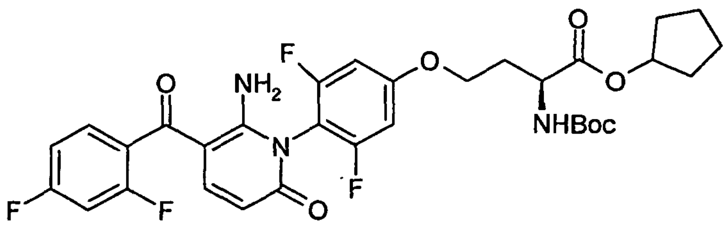

To a stirred mixture of 6-amino-5-(2,4-difluorobenzoyl)-1-(2,6-difluoro-4-hydroxy-phenyl)-1H-pyridin-2-one [prepared by methods described in WO03/076405] (100mg, 0.265mmol) and $K_2CO_3$ in DMF (1.5 ml) was added (L)-5-bromo-2-tert-butoxycarbonylaminopentanoic acid cyclopentyl ester (96mg, 0.265 mmol). The reaction mixture was stirred at 60°C for 2 h. The reaction mixture was diluted with EtOAc (15ml) and washed with sat aq $NaHCO_3$ (3ml) and water (10ml). The EtOAc layer was dried ($Na_2SO_4$), filtered and concentrated to dryness. Purification by flash chromatography (20% EtOAc / heptane) yielded the title compound as a white solid (50mg, 29%). LCMS purity 100%, m/z 648 $[M^++H]$, $^1H$ NMR (400 MHz, MeOD), δ:1.30 (9H, s), 1.40-1.65 (6H, m), 1.70-1.85 (2H, m), 1.95-2.30 (2H, m), 4.00-4.10 (2H, m), 4.15-4.20 (1H, m), 5.05-5.10 (1H, m), 5.65 (1H, d), 6.70-6.80 (2H, m), 6.95-7.05 (2H, m), 7.25-7.45 (2H, m).

Stage 2 - Synthesis of cyclopentyl (S)-2-Amino-4-{4-[6-amino-5-(2,4-difluorobenzoyl)-2-oxo-2H-pyridin-1-yl]-3,5-difluorophenoxy}butanoate trifluoroacetate **(16)**

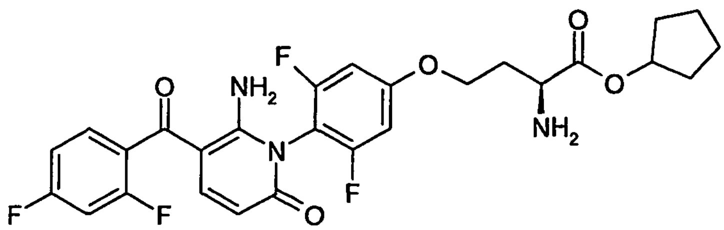

A mixture of Stage 1 product (10mg) and 20% TFA / DCM (0.5ml) was allowed to stand at r. T. For 3 h. The reaction mixture was concentrated to dryness by blowing under $N_2$. The residue was triturated with $Et_2O$ (0.3ml x 2) to give compound (16) as a white solid (9.3mg, 91%). LCMS purity 100%, m/z 548 $[M^++H]$, $^1H$ NMR (400 MHz, MeOD),

δ: 1.55-1.80 (6H, m), 1.85-2.00 (2H, m), 2.30-2.50 (2H, m), 4.15-4.30 (3H, m), 5.25-5.35 (1H, m), 5.75 (1H, d), 6.85-6.95 (2H, m), 7.05-7.15 (2H, m), 7.40-7.55 (2H, m).

Stage 3 - Synthesis of (S)-2-Amino-4-{4-[6-amino-5-(2,4-difluorobenzoyl)-2-oxo-2H-pyridin-1-yl]-3,5-difluorophenoxy} butanoic acid **(17)**

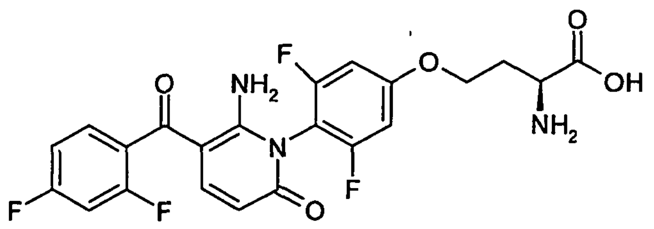

To a solution of compound (16) (20mg, 0.0317mmol) in a mixture of MeOH (0.3ml) and THF (0.3ml) was added 2M aq NaOH (0.3ml). The reaction mixture was allowed to stand at RT for 3h. Upon completion the reaction mixture was evaporated to dryness by blowing under a flow of $N_2$, acidified to pH 1-2 by dropwise addition of 2M aq HCl. The resulting white solid formed was collected by filtration. Yield= 9mg, 48%., LCMS purity 97%, m/z 480 [$M^+$+H], $^1$H NMR (400 MHz, MeOD), δ: 2.35-2.55 (2H, m, $CH_2$), 4.15-4.20 (1H, m, CH), 4.25-4.35 (2H, m, $CH_2$), 5.75 (1H, d, CH), 6.85-7.00 (2H, m, Ar), 7.05-7.20 (2H, m, Ar), 7.40-7.55 (2H, m, Ar).

*Compound* ***(18)*** *was prepared by the method described in the following scheme:*

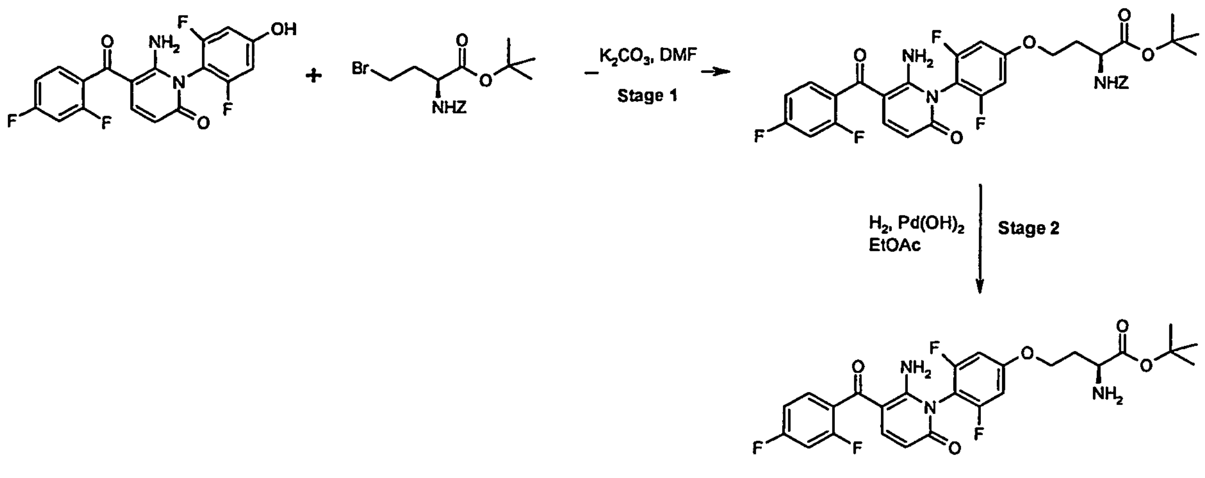


**Compound (18)**

Stage 1 - Synthesis of (S)-4-{4-[6-Amino-5-(2,4-difluoro-benzoyl)-2-oxo-2H-pyridin-1-yl]-3,5-difluoro-phenoxy}-2-benzyloxycarbonylamino-butyric acid tert-butyl ester

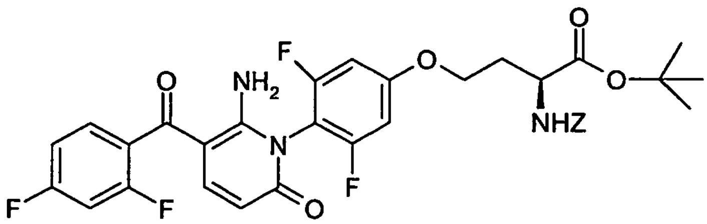

To a solution of 6-Amino-5-(2,4-difluorobenzoyl)-1-(2,6-difluoro-4-hydroxyphenyl)-1H-pyridin-2-one (100mg, 0.26mmol) and (S)-2-benzyloxycarbonylamino-4-bromo-butyric acid t-butyl ester (108mg, 0.29mmol) in acetone (2mL) was added sodium iodide (79mg, 0.53mmol) and potassium carbonate (146mg, 1.06mmol). The reaction was heated at reflux for 12h, cooled and partitioned between water (20mL) and ethyl acetate (20mL). The aqueous layer was re-extracted with ethyl acetate (2x10mL) and the combined organic extracts washed with brine (20mL), dried ($MgSO_4$) and concentrated under reduced pressure to give a yellow oil. This residue was subjected to column chromatography [silica gel, 40% ethyl acetate-heptane] to give the desired product (186mg, 79%) as a colourless solid, m/z 670 [M+H].

Stage 2 - Synthesis of (S)-2-Amino-4-{4-[6-amino-5-(2,4-difluoro-benzoyl)-2-oxo-2H-pyridin-1-yl]-3,5-difluoro-phenoxy}-butyric acid tert-butyl ester

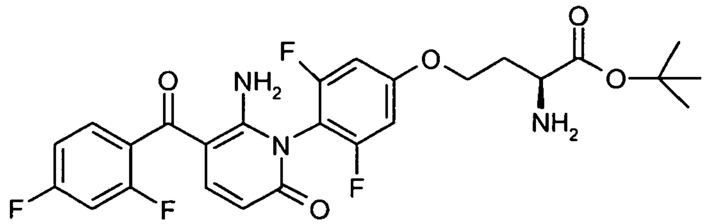

(S)-4-{4-[6-Amino-5-(2,4-difluoro-benzoyl)-2-oxo-2H-pyridin-1-yl]-3,5-difluoro-phenoxy}-2-benzyloxycarbonylamino-butyric acid tert-butyl ester

(140mg, 0.2mmol) was dissolved in ethyl acetate (15mL) containing 10% palladium hydroxide on carbon (20mg) and stirred under a hydrogen atmosphere (1atm) for 1 h. The reaction mixture was purged with N2, and filtered through Celite® washing with additional ethyl acetate. The filtrate was concentrated under reduced pressure to give a solid which was subjected to column chromatography [silica gel: 5%MeOH in dichloromethane]. This gave the desired product (60mg, 54%) as a grey solid: LCMS purity 98%, m/z 536 $[M+H]^+$, 1H NMR (300 MHz, CDCl3) 7.65-7.44 (1H, m), 7.39-7.29 (2H, m), 6.96-6.82 (2H, m), 6.66 (2H, br d, J=8.1 Hz), 5.82 (1H, d, J=9.9 Hz), 4.20-4.07 (3H, m), 3.48 (1H, dd, J=4.8, 8.7 Hz), 2.22-2.15 (1H, m), 1.91-1.84 (1H, m), 1.62 (2H, br s), 1.43 (9H, s).

## REFERENCE EXAMPLE 4

This example describes the modification of the known DHFR inhibitor 5-Methyl-6-((3,4,5-trimethoxyphenylamino)methyl)pyrido[2,3-*d*]pyrimidine-2,4-diamine (compound **(2 G=N))** by the attachment of an amino acid ester motif at a point where no disruption of its binding mode occurs.

**Compound (2 G=N):** 5-Methyl-6-((3,4,5-trimethoxyphenylamino)methyl)pyrido[2,3-*d*]pyrimidine-2,4-diamine

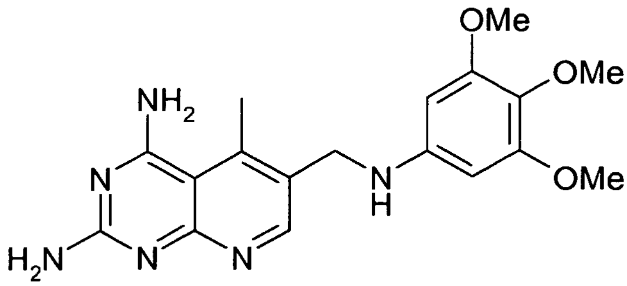

Compound **(2 G=N)** was prepared by a modification of the method described in J. Med. Chem. 1993, 36, 3437-3443.

2,4-Diamino-5-methylpyrido[2,3-*d*]pyrimidine-6-carbonitrile (0.10g, 0.5mmol), 3,4,5-trimethoxyaniline (0.10g, 0.55mmol) and Raney nickel (0.7g, damp) in acetic acid (20ml) were stirred at r. t. under an atmosphere of hydrogen. After 2 h the reaction mixture was filtered through celite and the solvent evaporated under reduced pressure. The crude residue was purified by reverse phase HPLC to afford compound **(2 G=N)** as a solid (22mg, 16%). LCMS purity 94%, m/z 371.1 $[M+H]^+$, 1H NMR (400 MHz, DMSO), δ: 8.5 (1H, s), 7.0 (2H, bs), 6.2 (2H, bs), 6.0 (2H, s), 5.7 (1H, m), 4.2 (2H, d), 3.7 (6H, s), 3.5 (3H, s), 2.7 (3H, s).

Compounds based on compound **(2 G=N)** were prepared by the methods outlined below.

*Compounds **(6)** and **(19)** were prepared by the method described in the following scheme:*

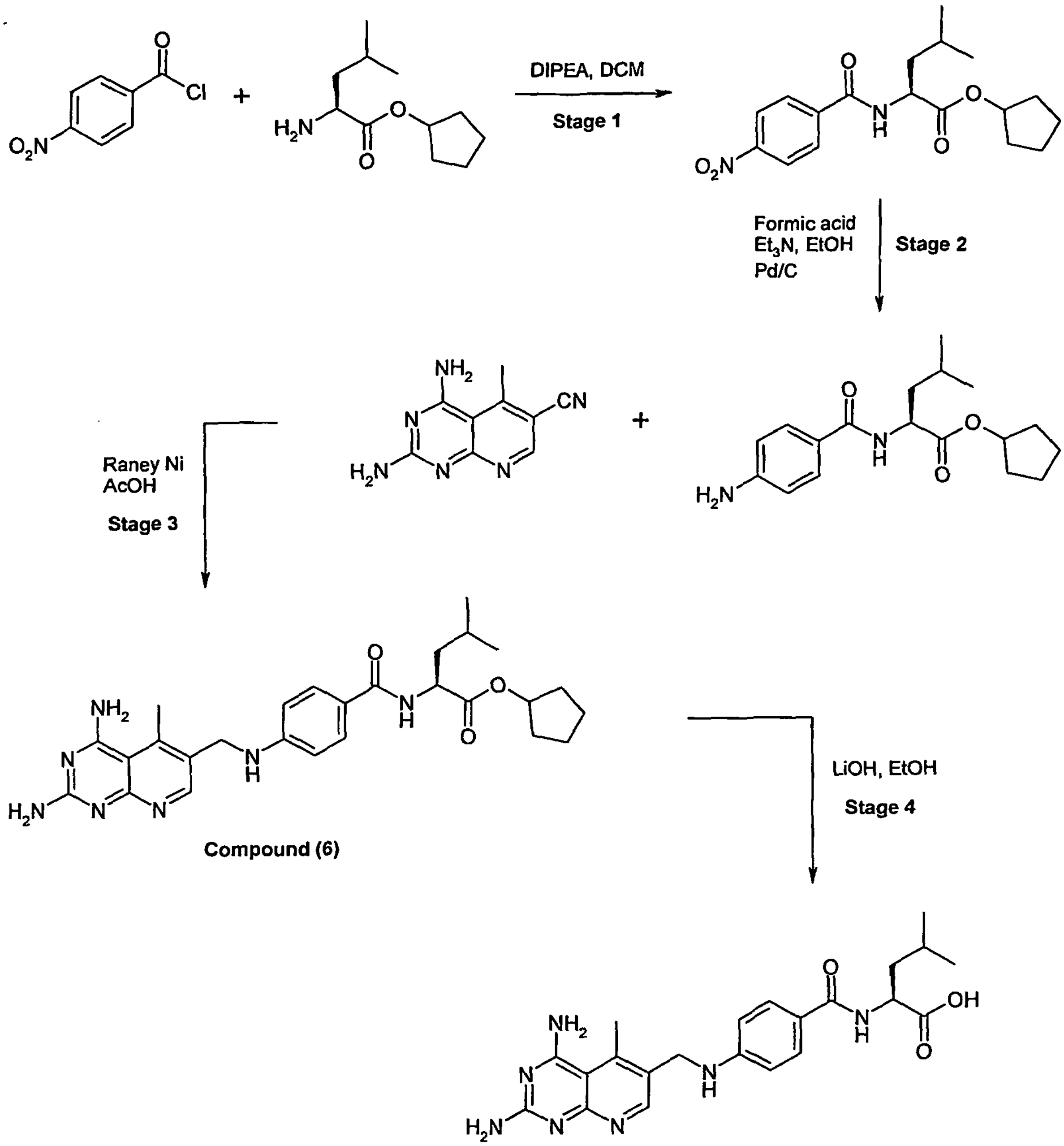


Compound (6)

Compound (19)

Stage 1 - Synthesis of (*S*)-cyclopentyl 4-methyl-2-(4-nitrobenzamido)pentanoate

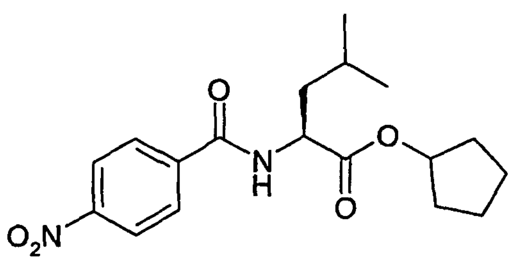

4-Nitrobenzoyl chloride (0.60g, 3.9mmol) in DCM (2ml) was added dropwise over 10 min to a solution of

(*S*)-cyclopentyl 2-amino-4-methylpentanoate (0.70g, 3.5 mmol) and diisopropylethylamine (0.94ml, 5.3mmol) in DCM (10ml) at -5°C under an atmosphere of nitrogen. On completion of the addition, the reaction mixture was allowed to warm to r. t. and stirred for a further 30 min. The reaction mixture was poured on to sat. aq. $NaHCO_3$ and the aqueous layer was extracted with DCM. The organic extracts were combined, washed with brine, dried over $MgSO_4$ and evaporated under reduced pressure afford the title compound as an oily solid in quantitative yield. LCMS purity 92%, m/z 347.1 $[M+H]^+$.

Stage 2 - Synthesis of (*S*)-cyclopentyl 2-(4-aminobenzamido)-4-methylpentanoate

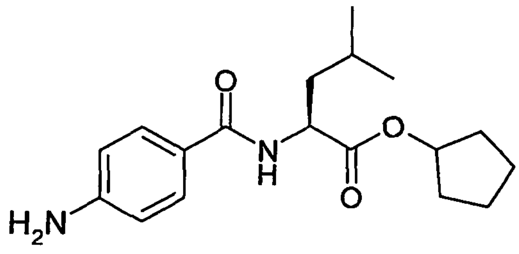

Triethylamine (1.09g, 10.8mmol) and formic acid (0.50g, 10.8mmol) were dissolved in EtOH (10ml) and added to a solution of Stage 1 product (1.2g, 3.4mmol) in EtOH (10ml). 10% Pd/C (approximately 10 mol%) was added and the mixture was heated to reflux. After 1 h the hot reaction mixture was filtered through celite and the residue was washed with MeOH. The filtrate and washings were combined and evaporated and the residue was partitioned between DCM and sat. aq. $NaHCO_3$. The organic layer was washed with brine, dried over $MgSO_4$ and evaporated under reduced pressure to furnish the title compound as a white solid (0.80g, 73%). LCMS purity 97%, m/z 319.2 $[M+H]^+$, $^1H$ NMR (400 MHz, $CDCl_3$), δ: 7.6 (2H, dd), 6.6 (2H, dd), 5.2 (1H, m), 6.4 (1H, d) 4.7 (1H, m), 4.0 (2H, s), 1.9 (2H, m), 1.7 (5H, m), 1.6 (4H, m), 0.9 (6H, dd).

Stage 3 - Synthesis of (S)-2-{4-[(2,4-Diamino-5-methyl-pyrido[2,3-d]pyrimidin-6-ylmethyl)-amino]-benzoylamino}-4-methyl-pentanoic acid cyclopentyl ester **(6)**

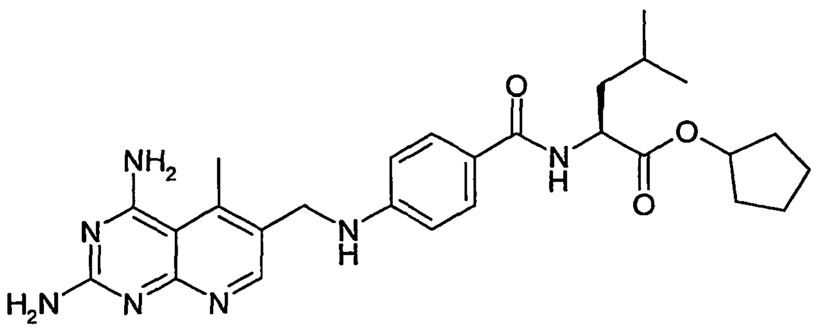

2,4-Diamino-5-methylpyrido[2,3-*d*]pyrimidine-6-carbonitrile (0.47g, 2.4mmol), Stage 2 product (300mg, 0.94mmol) and Raney nickel (1g, damp) in acetic acid (40ml) were stirred at r. t. under an atmosphere of hydrogen. After 48 h the reaction mixture was filtered through celite and the solvent evaporated under reduced pressure. The material was loaded in MeOH onto an SCX column and eluted off with a 1 % ammonia solution in MeOH. The crude product was then adsorbed onto silica and purified by column chromatography (10% MeOH/DCM) to afford compound **(6)** (60mg,13%). LCMS purity 95 %, m/z 506.1 $[M+H]^+$, $^1H$ NMR (400 MHz, DMSO), δ: 8.5 (1H, s), 8.2 (1H, d), 7.7 (2H, d), 7.0 (2H, bs), 6.7 (2H, d), 6.5 (1H, m), 6.2 (2H, bs), 5.1 (1H, m), 4.4 (1H, m), 4.3 (2H, d), 2.7 (3H, s), 1.7 (11H, m), 0.9 (6H, dd).

Stage 4 - Synthesis of (*S*)-2-(4-((2,4-diamino-5-methylpyrido[2,3-*d*]pyrimidin-6-yl)methylamino)benzamido)-4-methylpentanoic acid **(19)**

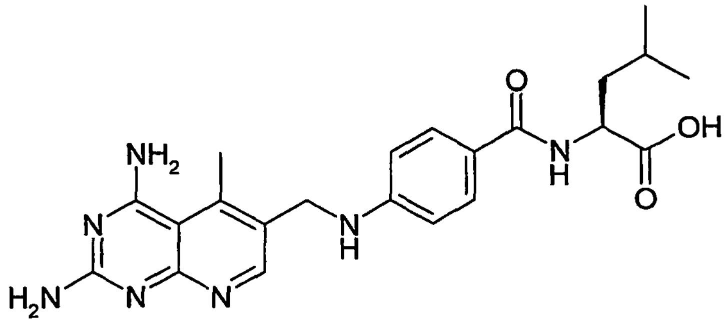

Stage 3 product (39μM) was suspended in EtOH (1.0ml). A solution of 1M lithium hydroxide (156μl) was added to the above and the suspension allowed to stir for 48 h. The EtOH was subsequently removed under reduced pressure, the residual diluted with water and taken down to pH 4 with dilute acetic acid. The solution was washed with DCM, evaporated and subjected to SCX purification to afford compound **(19).** LCMS purity 92%, m/z 438 $[M+H]^+$; $^1H$ NMR (400 MHz, DMSO) δ: 8.5 (1H, s), 8.1 (1H, d), 7.7 (2H, d), 7.2 (2H, br s), 6.7 (2H, d), 6.5 (1H, t), 6.4 (2H, br s), 4.4 (1H, m), 4.3 (2H, d), 2.7 (3H, s), 1.8 - 1.6 (2H, m), 1.6 - 1.5 (1H, m), 0.9 (6H, dd).

*Compounds* ***(5)*** *and the corresponding acid were prepared by the method described in the following scheme:*

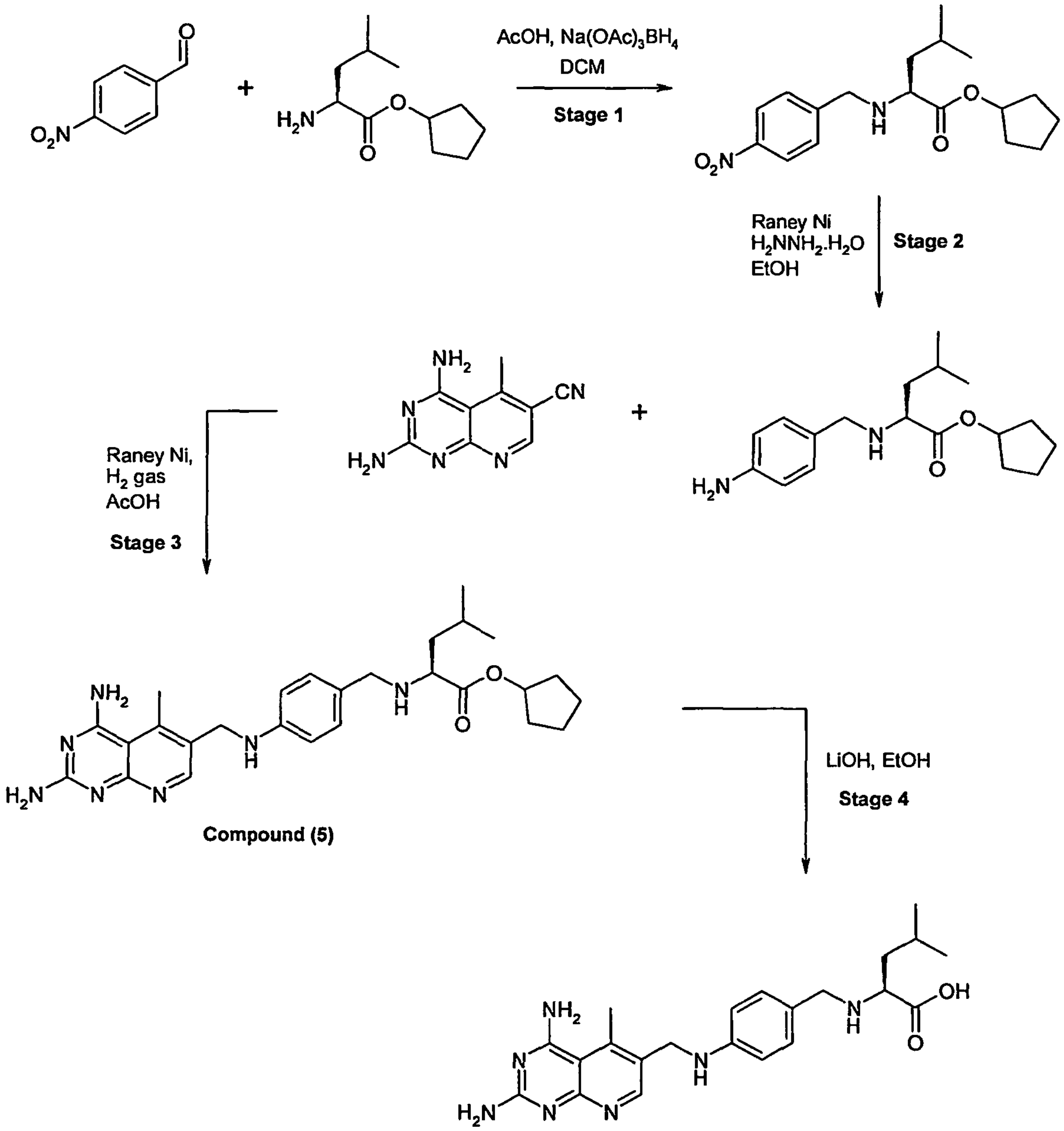


Compound (5)

Stage 1 - Synthesis of (*S*)-Cyclopentyl 4-methyl-2-(4-nitrobenzylamino)pentanoate

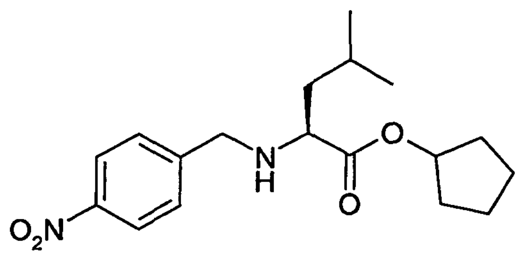

To a solution of (*S*)-cyclopentyl 2-amino-4-methylpentanoate (2.00g, 10.0mmol) and 4-nitrobenzaldehyde (3.04g, 20.0mmol) in DCM (40ml) was added glacial acetic acid (2 drops). The solution was allowed to stir at r. t. for 1 h whereupon sodium triacetoxyborohydride (6.40g, 30.2mmol) was added in a single a portion. After stirring for 3 h, the

solution was poured on to aq. 1 M HCl, allowed to stir for 30 min, neutralised with aq. 1 M NaOH and extracted with DCM. The combined organics were washed with water and brine, dried over $MgSO_4$, and evaporated under reduced pressure. The crude material was purified by chromatography (5% EtOAc / isohexane) to furnish the title compound as an oil (1.51 g). This was used without further purification for the following step. LCMS purity 71%, m/z 335.1 $[M-H]^+$.

Stage 2 - Synthesis of (S)-2-(4-Amino-benzylamino)-4-methyl-pentanoic acid cyclopentyl ester

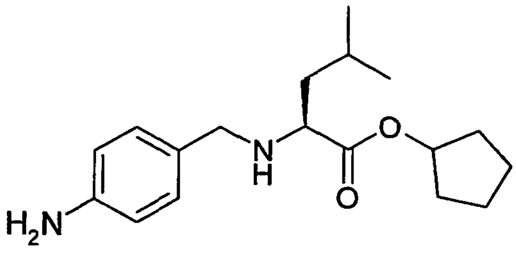

Stage 1 product (0.90g, 2.7mmol) was dissolved in EtOH (5ml) and added to a suspension of Raney nickel (~ 0.5g) and hydrazine monohydrate (0.38ml, 8.1mmol) in EtOH (5ml). After heating under reflux for 1 h the hot reaction mixture was filtered through celite and the residue was washed with MeOH. The filtrate and washings were combined and evaporated and the residue was partitioned between DCM and sat. aq. Sodium hydrogen carbonate. The organic layer was washed with brine, dried over $MgSO_4$ and evaporated under reduced pressure. The crude material was purified by chromatography (20% EtOAc / isohexane) to furnish the title compound as an oil (0.50g, 61 %). LCMS purity 99%, m/z 305.2 $[M+H]^+$, $^1H$ NMR (400 MHz, $CDCl_3$), δ: 7.1 (2H, d), 6.6 (2H, d), 5.2 (1H, m), 3.7 (1H, d), 3.5 (1H, d), 3.2 (1H, t), 1.9 (2H, m), 1.7 (5H, m), 1.6 (4H, m), 0.9 (6H, dd).

Stage 3 - Synthesis of (S)-2-{4-[(2,4-Diamino-5-methyl-pyrido[2,3-d]pyrimidin-6-ylmethyl)-amino]-benzylamino}-4-methyl-pentanoic acid cyclopentyl ester (5)

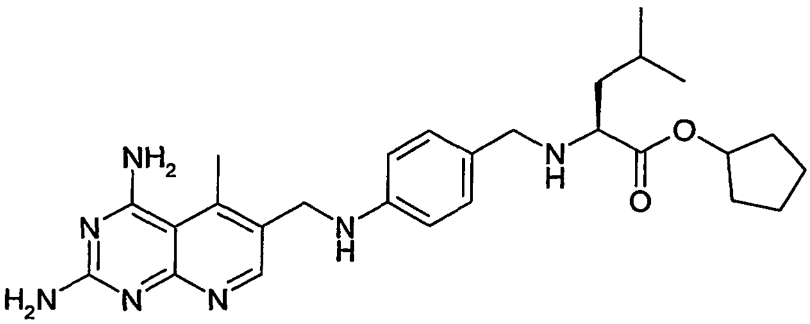

2,4-Diamino-5-methylpyrido[2,3-*d*]pyrimidine-6-carbonitrile (0.16g, 0.83mmol), Stage 2 product (100mg, 0.33mmol) and Raney nickel (1g, damp) in acetic acid (10ml) were stirred at r. t. under an atmosphere of hydrogen. After 5 h the reaction mixture was filtered through celite and the solvent evaporated under reduced pressure. The material was loaded in MeOH onto an SCX column and eluted with a 1% ammonia solution in MeOH. The crude product was then adsorbed onto silica and purified by column chromatography (10% MeOH/DCM) to afford the title compound (5) (30mg, 19%). LCMS purity 95 %, m/z 492.1 $[M+H]^+$, $^1H$ NMR (400 MHz, DMSO), δ: 8.5 (1H, s), 7.2 (2H, bs), 7.0 (2H, d), 6.6 (2H, d), 6.2 (2H, bs), 5.8 (1H, m), 5.1 (1H, m), 4.2 (2H, s), 3.6 (1H, m), 3.4 (1H, m), 3.1 (1H, m), 2.7 (3H, s), 1.5 (11 H, m), 0.8 (6H, dd).

Stage 4 - Synthesis of (S)-2-{4-[(2,4-Diamino-5-methyl-pyrido[2,3-d]pyrimidin-6-ylmethyl)-amino]-benzylamino}-4-methyl-pentanoic acid

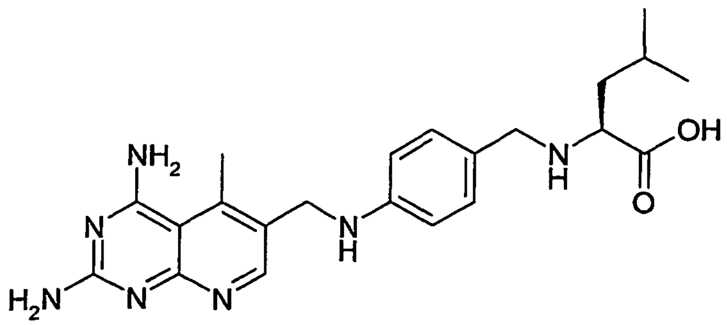

Stage 3 product (39$\mu$M) was suspended in EtOH (1.0ml). A solution of 1 M lithium hydroxide (156$\mu$l) was added to the above and the suspension allowed to stir for 48 h. The EtOH was subsequently removed under reduced pressure, the residual diluted with water and taken down to pH 4 with dilute acetic acid. The solution was washed with DCM, evaporated and subjected to SCX purification to afford the title compound LCMS : 95% purity at $R_t$ 0.52 and 1.91 min, m/z (ES$^+$) 424 [M+H]$^+$; $^1$H NMR (400 MHz, DMSO) $\delta$: 8.5 (1H, s), 7.1 (2H, d), 7.0 (2H, br s), 6.6 (2H, d), 6.2 (2H, br s), 5.7 (1H, t), 4.3 (2H, d), 3.6 (1H, m), 3.3 (2H, obscured by water), 2.7 (3H, s), 1.8 (1H, m), 1.3 (1H, m), 1.2 (1H, m).

*Compound **(3)** was prepared by the method described in the following scheme:*

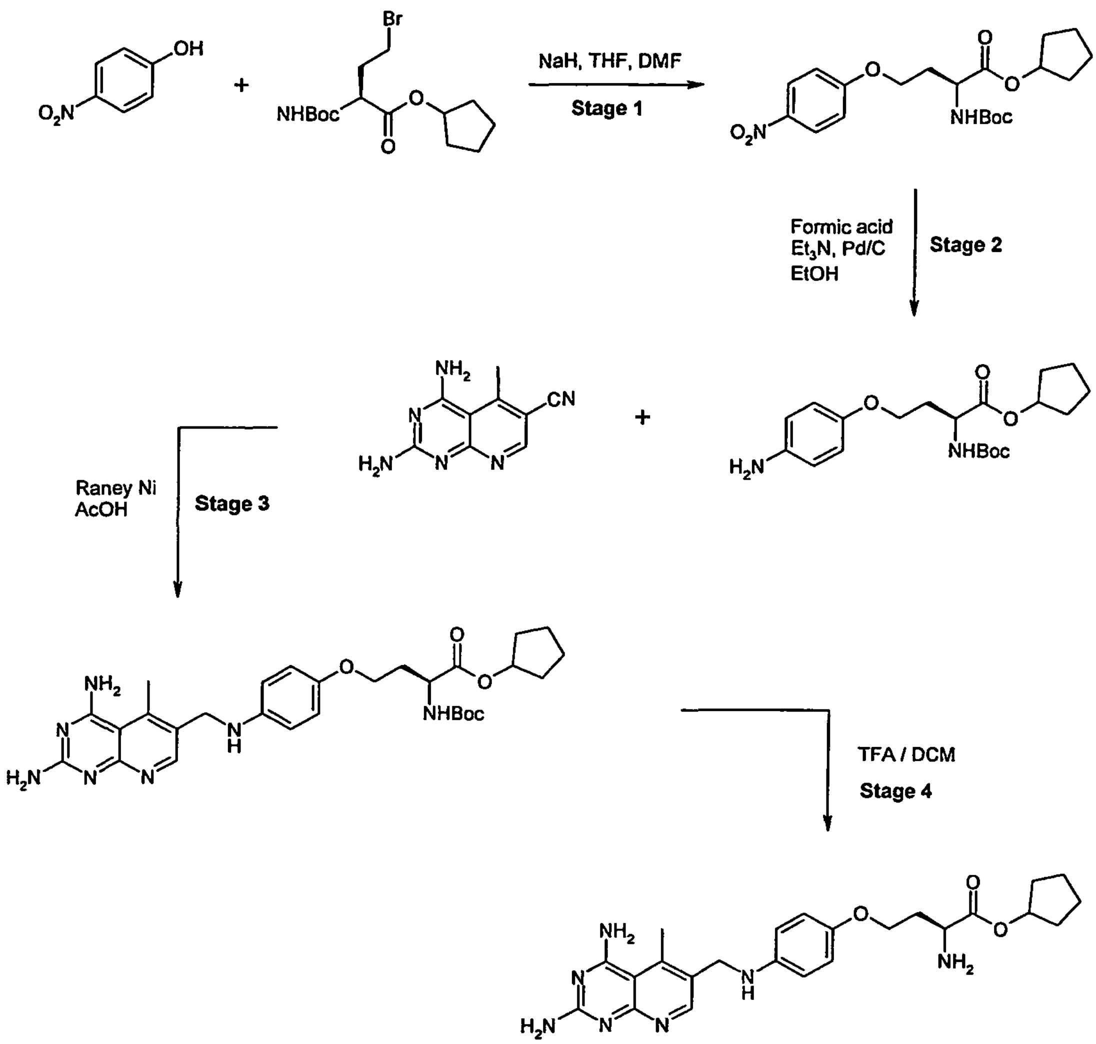


**Compound (3)**

Stage 1 - Synthesis of (*S*)-Cyclopentyl-2-(*tert*-butoxycarbonylamino)-4-(4-nitrophenoxy)butanoate

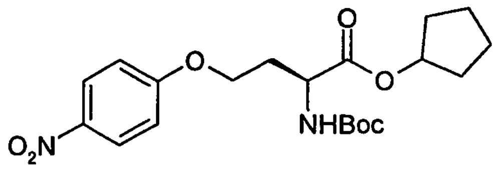

To a solution of 4-nitrophenol (2.18g, 15.7mmol) in tetrahydrofuran (100ml) at 0°C under nitrogen was added sodium hydride (0.63g, 15.7mmol). After warming to r. t. and stirring for 10 min, a solution of (*S*)-cyclopentyl-4-bromo-2-(*tert*-butoxycarbonylamino)butanoate (5.0g, 14.3mmol) in DMF (20ml) was added. The reaction was heated to 60°C for 10 h, after which the reaction was cooled to r. t. and poured onto ether / sodium carbonate. The organic layer was collected and washed with 2M aq. Sodium carbonate solution, 1 M HCl and brine before being dried over $MgSO_4$ and concentrated under reduced pressure to afford an oil which solidified upon standing to yield the title compound (4.0g, 69%). LCMS purity 97%, m/z 407.1 $[M+H]^+$, $^1H$ NMR (400 MHz, $CDCl_3$), δ: 8.2 (2H, d), 7.4 (1H, d), 7.1 (2H, d), 5.1 (1H, m), 4.1 (3H, m), 2.1 (2H, m), 1.8 (2H, m), 1.6 (6H, m), 1.4 (9H, s).

Stage 2 - Synthesis of (*S*)-Cyclopentyl-4-(4-aminophenoxy)-2-(tertbutoxycarbonylamino)butanoate

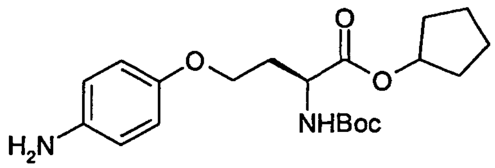

Triethylamine (0.77ml, 5.2 mmol) and formic acid (0.19ml, 5.2 mmol) were dissolved in EtOH (4ml) and added to a solution of Stage 1 product (0.7g, 1.7mmol) in EtOH (4ml). 10% Pd/C (approximately 10 mol%) was added and the mixture was heated to reflux. After 2 h the hot reaction mixture was filtered through celite and the residue was washed with MeOH. The filtrate and washings were combined and evaporated and the residue was partitioned between DCM and sat. aq. Sodium hydrogen carbonate. The organic layer was washed with brine, dried over $MgSO_4$ and concentrated under reduced pressure. The residue was purified by column chromatography (gradient elution, 10-40% EtOAc in hexane) to afford the title compound (0.3g, 46%). LCMS purity 93%, m/z 379.1 $[M+H]^+$, $^1H$ NMR (400 MHz, $CDCl_3$), δ: 6.9 (2H, d), 6.8 (2H, d), 5.3 (2H, m), 4.4 (1H, m) 4.0 (2H, m), 2.3 (1H, m), 2.2 (1H, m), 1.9 (2H, m), 1.7 (4H, m), 1.6 (2H, m), 1.4 (9H, s).

Stage 3 - Synthesis of (*S*)-cyclopentyl-2-(*tert*-butoxycarbonylamino)-4-(4-((2,4-diamino-5-methylpyrido[2,3-*d*]pyrimidin-6-yl)methylamino)phenoxy)butanoate

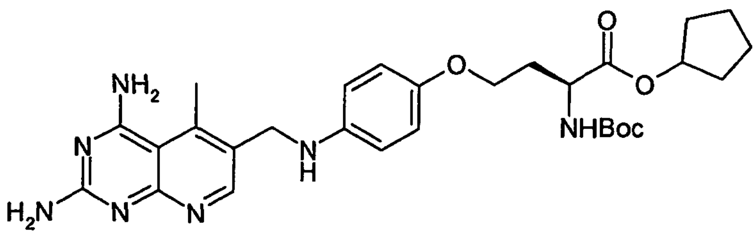

2,4-Diamino-5-methylpyrido[2,3-*d*]pyrimidine-6-carbonitrile (0.50g, 2.5mmol), Stage 2 product (0.38g, 1.0mmol) and Raney nickel (3g, damp) in acetic acid (40ml) were stirred at r. T. Under an atmosphere of hydrogen. After 16 h the reaction mixture was filtered through celite and the solvent evaporated under reduced pressure. The material was loaded in MeOH onto an SCX column and eluted with a 1% ammonia solution in MeOH. The crude product

was then adsorbed onto silica and purified by column chromatography (5% MeOH/DCM) to afford the title compound (145mg, 26%). LCMS purity 95 %, m/z 566.2 [M+H]$^+$, $^1$H NMR (400 MHz, DMSO), δ: 8.5 (1H, s), 7.3 (2H, m), 7.0 (2H, m), 6.7 (2H, d), 6.6 (2H, d), 6.2 (2H, bs), 5.5 (1H, bs), 5.1 (1H, m), 4.1 (3H, m), 3.9 (2H, m), 2.6 (3H, s), 2.0 (1H, m), 1.8 (3H, m), 1.6 (6H, m), 1.4 (9H, s).

Stage 4 - Synthesis of (S)-2-Amino-4-{4-[(2,4-diamino-5-methyl-pyrido[2,3-d]pyrimidin-6-ylmethyl)-amino]-phenoxy}-butyric acid cyclopentyl ester (3)

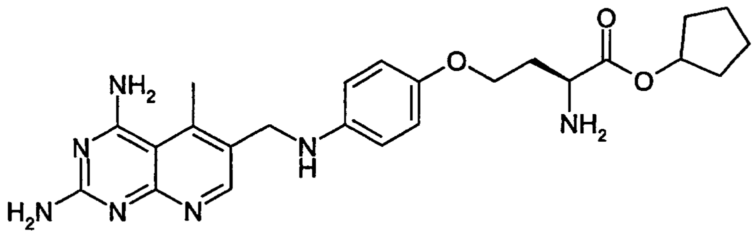

To a solution of Stage 3 product (145mg, 0.26mmol) in DCM (3ml) was added trifluoroacetic acid (3ml) and the reaction stirred for 30 min at r. t.. The solvent was evaporated under reduced pressure and the crude residue purified by loading in MeOH onto an SCX column and eluting with a 1 % ammonia solution in MeOH to afford compound (3) (39mg, 33%). LCMS purity 94 %, m/z 466.1 [M+H]$^+$, $^1$H NMR (400 MHz, DMSO), δ: 8.5 (1H, s), 7.0 (2H, bs), 6.7 (2H, d), 6.6 (2H, d), 6.2 (2H, bs), 5.5 (1H, bs), 5.1 (1H, m), 4.1 (2H, s), 3.9 (2H, m), 3.4 (1H, m), 2.7 (3H, s), 2.0 (2H, m), 1.8 (3H, m), 1.6 (6H, m).

*Compound **(4)** was prepared by the method described in the following scheme:*

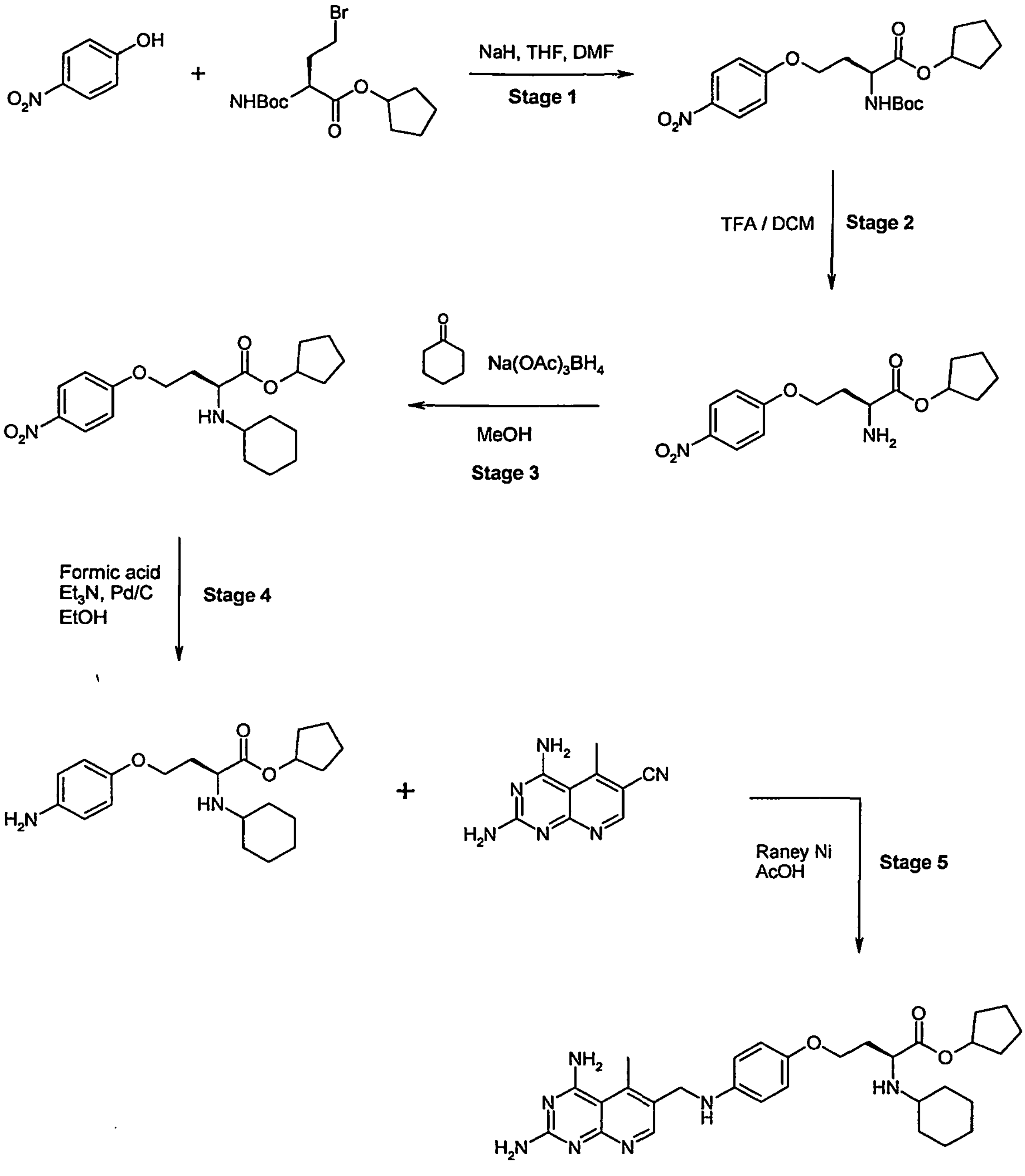


**Compound (4)**

Stage 1 is the same as described for compound (3).

Stage 2 - Synthesis of (*S*)-Cyclopentyl 2-amino-4-(4-nitrophenoxy)butanoate

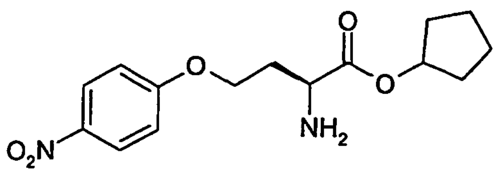

To a solution of Stage 1 product (4.0g, 9.8mmol) in DCM (12ml) was added trifluoroacetic acid (12ml). After stirring at r. t. for 1 h the reaction was diluted with DCM, cooled in ice and neutralised by the addition of aq. Ammonia. The organic layer was collected and washed with water, aq. Sodium hydrogen carbonate and brine, then dried over $MgSO_4$ and concentrated under reduced pressure to afford the title compound as a yellow oil (3.0g, 100%). LCMS purity 97%, m/z 309.1 $[M+H]^+$, $^1H$ NMR (400 MHz, $CDCl_3$), δ: 8.2 (2H, d), 7.0 (2H, d), 5.2 (1H, m), 4.2 (2H, m), 3.6 (1H, dd), 1.7-1.5 (10H, m).

Stage 3 - Synthesis of (*S*)-Cyclopentyl 2-(cyclohexylamino)-4-(4-nitrophenoxy)butanoate

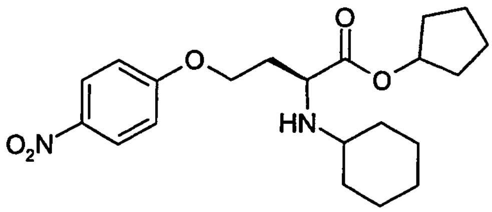

To a flask containing Stage 2 product (1.0g, 3.3mmol) and cyclohexanone (0.34ml, 3.3mmol) under nitrogen was added anhydrous MeOH (10ml). After stirring for 12 h at r. t. sodium triacetoxyborohydride (2.07g, 9.75mmol) was added. After 4 h the reaction was poured slowly onto a mixture of DCM / aq. HCl (1M). After stirring for 10 min the organic layer was collected and washed with sodium hydrogen carbonate and brine, then dried over $MgSO_4$ and concentrated under reduced pressure to afford the title compound as a yellow oily solid (1.21g, 95%). LCMS purity 92%, m/z 391.1 $[M+H]^+$.

Stage 4 - Synthesis of (*S*)-Cyclopentyl 4-(4-aminophenoxy)-2-(cyclohexylamino)butanoate

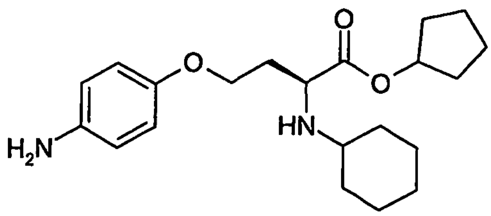

Triethylamine (1.29ml, 9.3mmol) and formic acid (348μl, 9.3mmol) were dissolved in EtOH (10ml) and added to a solution of Stage 3 product (1.2g, 3.1mmol) in EtOH (10ml). 10% Pd/C (approximately 10 mol%) was added and the mixture was heated to reflux. After 30 min the hot reaction mixture was filtered through celite and the residue was washed with MeOH. The filtrate and washings were combined and evaporated and the residue was partitioned between DCM and sat. aq. $NaHCO_3$. The organic layer was washed with brine, dried over $MgSO_4$ and concentrated under reduced pressure to afford the title compound (1.01g, 92%). LCMS purity 94%, m/z 361.1 $[M+H]^+$, $^1H$ NMR (400 MHz, $CDCl_3$), δ: 6.7 (2H, d), 6.6 (2H, d), 5.2 (1H, m), 4.0 (1H, m), 3.9 (1H, m), 3.5 (1H, dd), 2.3 (1H, m), 2.1 (1H, m), 1.9 (4H, m), 1.7 (7H, m), 1.6 (3H, m), 1.3-0.9 (5H, m).

Stage 5 - Synthesis of (S)-2-Cyclohexylamino-4-{4-[(2,4-diamino-5-methyl-pyrido[2,3-d]pyrimidin-6-ylmethyl)-amino]-phenoxy}-butyric acid cyclopentyl ester (4)

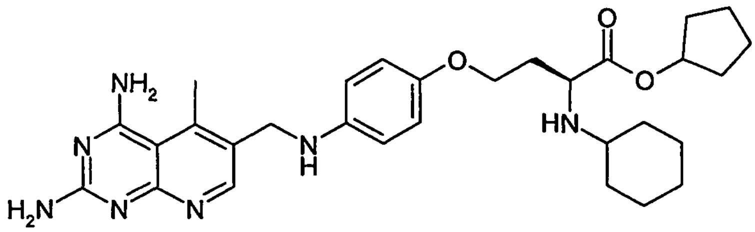

2,4-Diamino-5-methylpyrido[2,3-*d*]pyrimidine-6-carbonitrile (0.50g, 2.5mmol), Stage 4 product (0.36g, 1.0mmol) and Raney nickel (3g, damp) in acetic acid (40ml) were stirred at r. t. under an atmosphere of hydrogen. After 48 h the reaction mixture was filtered through celite and the solvent evaporated under reduced pressure. The material was loaded in MeOH onto an SCX column and eluted with a 1% ammonia solution in MeOH. The crude product was then adsorbed onto silica and purified by column chromatography (10% MeOH/DCM) to afford the title compound (76 mg, 14%). LCMS purity 90 %, m/z 548.2 $[M+H]^+$, $^1H$ NMR (400 MHz, DMSO), $\delta$: 8.5 (1H, s), 7.0 (2H, bs), 6.7 (2H, d), 6.6 (2H, d), 6.2 (2H, bs), 5.5 (1H, m), 5.1 (1H, m), 4.1 (2H, s), 3.9 (2H, m), 3.4 (1H, m), 2.7 (3H, s), 2.3 (1H, m), 1.9 (1H, m), 1.8 (4H, m), 1.6 (11 H, m), 1.1 (5H, m).

REFERENCE EXAMPLE 5

This example describes the modification of the known PI3 kinase inhibitor N-[5-(4-Chloro-3-methanesulfonyl-phenyl)-4-methyl-thiazol-2-yl]-acetamide (compound (20)) by the attachment of an amino acid ester motif at a point where no disruption of its binding mode occurs.

Compound (20): N-[5-(4-Chloro-3-methanesulfonyl-phenyl)-4-methyl-thiazol-2-yl]-acetamide

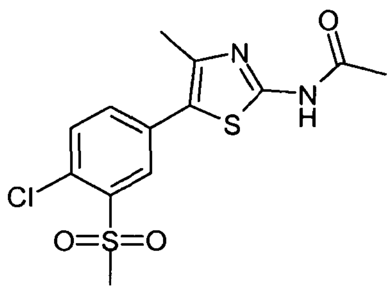

Compound (20) was prepared as described in WO03072552

Compounds based on compound (20) were prepared by the methods outlined below.

*Compounds* (21) *and* (22) *were prepared by the method described in the following scheme:*

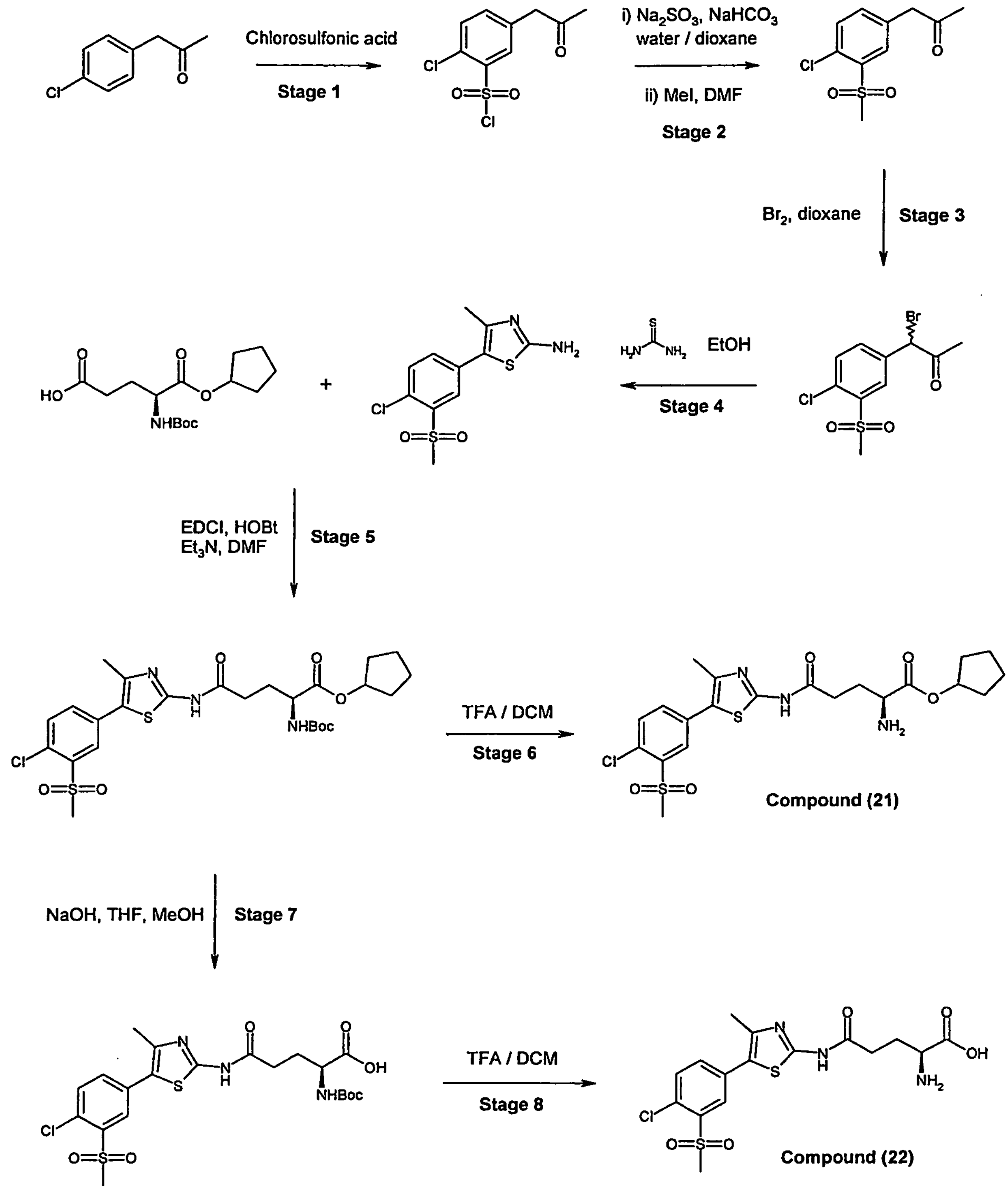


Stage 1 - Synthesis of 2-Chloro-5-(2-oxo-propyl)-benzenesulfonyl chloride

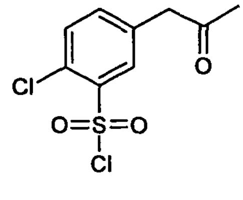

4-Chlorophenyl acetone (4g, 0.023mol) was added dropwise to chlorosulfonic acid (30ml, 0.45mol) at -10 °C under $N_2$ with gentle stirring. The reaction mixture was allowed to warm to r. t. and stirring was continued for 18 h. The reaction mixture was carefully quenched by adding dropwise to crushed ice (500ml). The aq. Solution was extracted with EtOAc (3 x 250ml). EtOAc layers combined, dried ($Na_2SO_4$), filtered and concentrated to dryness *in vacuo* to give the crude title compound (6.3g, 65%) which was used in the next step without further purification. LCMS purity 92%. $^1H$ NMR (400 MHz, $CDCl_3$), δ: 2.30 (3H, s), 3.85 (2H, s), 7.50 (1H, d), 7.65 (1H, d), 7.95 (1H, s).

Stage 2 - Synthesis of 1-(4-Chloro-3-methanesulfonyl-phenyl)-propan-2-one

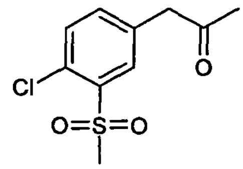

A mixture of $Na_2SO_3$ (3.79g, 0.030mol) and $NaHCO_3$ (2.53g, 0.030mol) in water (90ml) was stirred at 70°C. To this solution was added a solution of Stage 1 product (4.65g, 0.015mol) in dioxane (190ml). Stirring was continued at 70°C for 1 h. Upon cooling to r. t. the reaction mixture was concentrated to dryness *in vacuo* giving a brown solid. DMF (190ml) was added followed by MeI (1.88ml, 0.030mol). The reaction mixture was stirred at 40°C for 1 h. After completion the reaction mixture was poured into water (90ml) and extracted with EtOAc (500ml). The EtOAc was dried ($Na_2SO_4$), filtered and concentrated *in vacuo* to give the title compound as a brown solid (2.49g, 67%) which was used in the next step without further purification. LCMS purity 81 %, m/z 247 [$M^+$+H]; $^1H$ NMR (400 MHz, $CDCl_3$), δ: 2.15 (3H, s), 3.20 (3H, s), 3.75 (2H, s), 7.35 (1H, d), 7.45 (1H, d), 7.85 (1H, s).

Stage 3 - Synthesis of 1-Bromo-1-(4-chloro-3-methanesulfonyl-phenyl)-propan-2-one

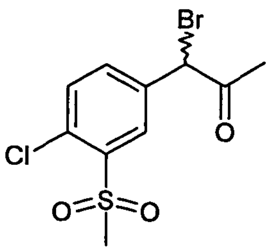

To a stirred solution of Stage 2 product (1.88g, 7.60mmol) in 1,4-dioxane (120ml) bromine (0.292ml, 5.72mmol) was added dropwise at r. t. giving a dark orange solution. Stirring was continued for 18 h. The reaction mixture was evaporated to dryness *in vacuo* avoiding heating above 30°C during evaporation. The residue was re-dissolved in EtOAc (100ml) and washed with sat aq $NaHCO_3$ (20ml) followed by water (20ml). The EtOAc layer was dried ($Na_2SO_4$), filtered and concentrated *in vacuo.* Purification by flash chromatography (50% EtOAc / heptane) gave the title compound as a yellow oil (2.0g, 80%). LCMS purity 74%, m/z 325/327 [$M^+$+H]; $^1H$ NMR (400 MHz, $CDCl_3$), δ: 2.35 (3H, s), 3.25 (3H, s), 5.35 (1H, s), 7.50 (1H, d), 7.65 (1H, dd), 8.05 (1H, s).

Stage 4 - Synthesis of 5-(4-Chloro-3-methanesulfonyl-phenyl)-4-methyl-thiazol-2-ylamine

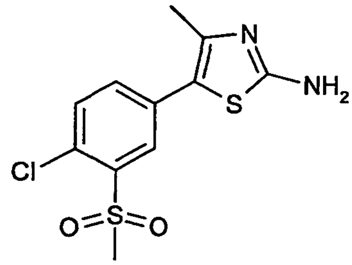

A mixture of Stage 3 product (2g, 6.15mmol) and thiourea (468mg, 6.15mmol) in EtOH (65ml) was stirred at 70°C for 1.5 h. The reaction was then cooled to r. t. and precipitation occurred. The cream solid was collected by filtration to afford the title compound (1.2g, 64%). LCMS purity 91%, m/z 303 [$M^+$+H], $^1$H NMR (400 MHz, MeOD), δ: 2.35 (3H, s), 3.35 (3H, s), 7.75-7.85 (2H, m), 8.15 (1H, s).

Stage 5 - Synthesis of (S)-2-tert-Butoxycarbonylamino-4-[5-(4-chloro-3-methanesulfonylphenyl)-4-methyl-thiazol-2-ylcarbamoyl]-butyric acid cyclopentyl ester

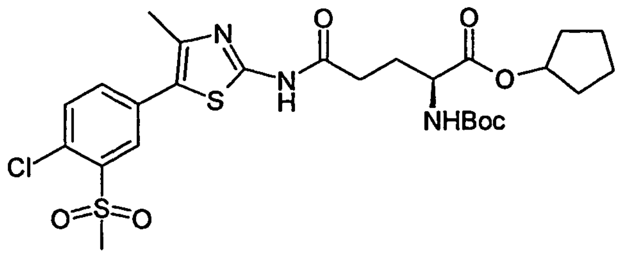

To a stirred mixture of 2-tert-butoxycarbonylamino-pentanedioic acid 1-cyclopentyl ester (208mg, 0.66mmol), EDCI (190mg, 0.99mmol) and HOBt (107mg, 0.79mmol) in DMF (1.5ml) was added dropwise a solution of Stage 4 product (200mg, 0.66mmol) in DMF (1.5ml) at r. t. Triethylamine (0.138ml,0.99mmol) was added and stirring was continued for 18 h. The reaction mixture was diluted with water (10ml) and extracted with EtOAc (15ml). The EtOAc layer was washed with water (10ml), dried ($Na_2SO_4$), filtered and concentrated *in* vacuo. Purification by preparative TLC (70% EtOAc/ heptane, $R_f$ 0.5) afforded the title compound (160mg, 40%). LCMS purity 91%, m/z 600/602[$M^+$+H], $^1$H NMR (400 MHz, DMSO), δ: 1.45-1.55 (9H, s), 1.65-2.15 (10H, m), 2.45 (3H, s), 2.70 (2H, m), 3.45 (3H, s), 4.10-4.25 (1H, m), 5.25 (1H, m), 7.75-7.90 (2H, m), 8.25 (1H, s).

Stage 6 - Synthesis of (S)-2-Amino-4-[5-(4-chloro-3-methanesulfonyl-phenyl)-4-methyl-thiazol-2-ylcarbamoyl]-butyric acid cyclopentyl ester **(21)**

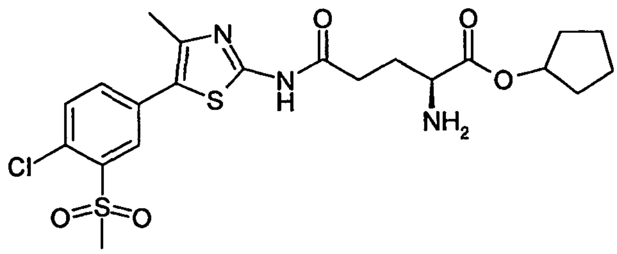

A solution of Stage 5 product (140mg, 0.233mmol) in 20% TFA / DCM (2ml) was allowed to stand at r. t. for 3 h. After completion the reaction mixture was concentrated *in vacuo* to give compound **(21)** (143mg, 100%). LCMS purity 97%, m/z 500/502 [$M^+$+H], $^1$H NMR (400 MHz, MeOD), δ: 1.35-1.85 (8H, m), 2.00-2.20 (2H, m), 2.25 (3H, s), 2.60 (2H, m), 3.20 (3H, s), 3.85-4.00 (1H, m), 5.10 (1H, m), 7.50-7.65 (2H, m), 7.95 (1H, s).

Stage 7 - Synthesis of (S)-2-tert-Butoxycarbonylamino-4-[5-(4-chloro-3-methanesulfonylphenyl)-4-methyl-thiazol-2-yl-carbamoyl]-butyric acid

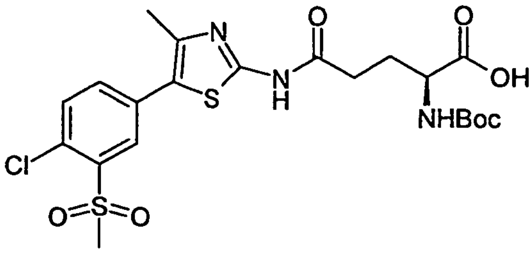

To a solution of Stage 5 product (20mg, 0.033mmol) in a mixture of THF (0.5ml) and MeOH (0.5ml) was added 2M aq. NaOH (0.5ml). The mixture was allowed to stand at r. t. for 3 h. Upon completion the reaction mixture was concentrated to near dryness and 1 M HCl added dropwise until pH 1-2. The resultant precipitate was collected by filtration under slight pressure. The solid was washed with water (0.5ml) and thoroughly dried *in vacuo* to yield the title compound (12mg, 68%). LCMS purity 94%, m/z 532/534 $[M^{+}+H]$, $^{1}H$ NMR (400 MHz, $CDCl_3$), δ: 1.55-1.70 (9H, s), 2.15-2.55 (2H, m), 2.60 (3H, s), 2.75-2.90 (2H, m), 3.55 (3H, s), 4.25-4.45 (1H, m), 7.85-8.00 (2H, m), 8.35 (1H, s).

Stage 8 - Synthesis of (S)-2-Amino-4-[5-(4-chloro-3-methanesulfonyl-phenyl)-4-methyl-thiazol-2-ylcarbamoyl]-butyric acid **(22)**

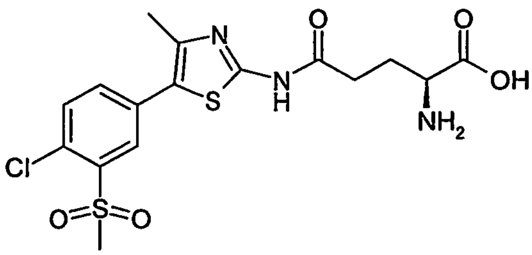

A solution of Stage 7 product (12mg, 0.0225mmol) in 20% TFA / DCM (0.3ml) was allowed to stand at r. t. for 3 h. After completion the reaction mixture was concentrated *in vacuo* to give the title compound **(22)** (12mg, 100%). LCMS purity 94%, m/z 432/434 $[M^{+}+H]$, $^{1}H$ NMR (400 MHz, MeOD), δ: 2.10-2.25 (2H, m), 2.30 (3H, s), 2.65-2.75 (2H, m), 3.25 (3H, s), 3.95-4.05 (1H, m), 7.60-7.80 (2H, m), 8.05 (1H, s).

*Compound **(23)** was prepared by the method described in the following scheme:*

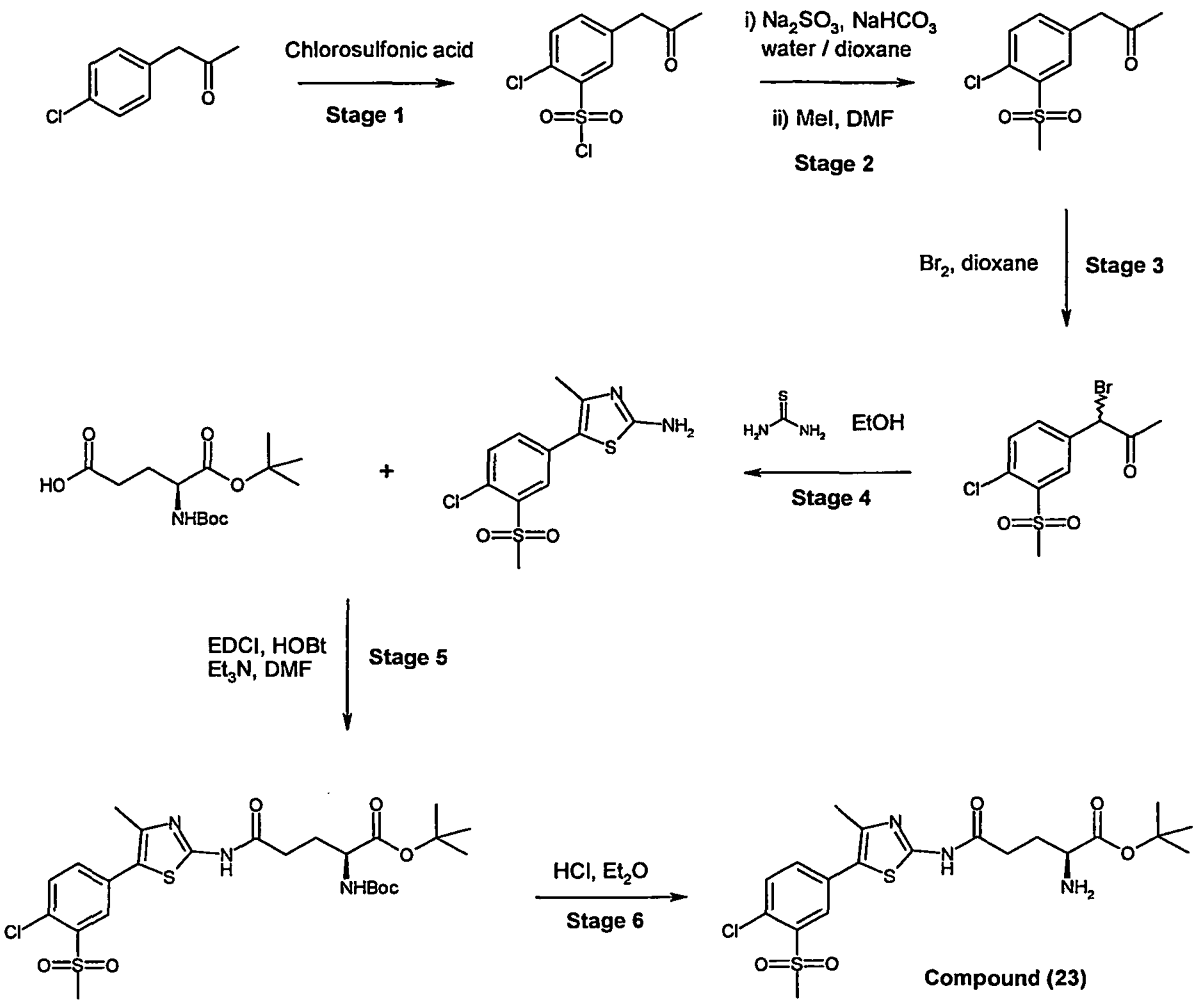


**Compound (23)**

Stages 1, 2, 3 and 4 are the same as described for the preparation of compounds **(21)** and **(22)**

Stage 5 - Synthesis of (S)-2-Amino-4-[5-(4-chloro-3-methanesulfonyl-phenyl)-4-methyl-thiazol-2-ylcarbamoyl]-butyric acid tert-butyl ester

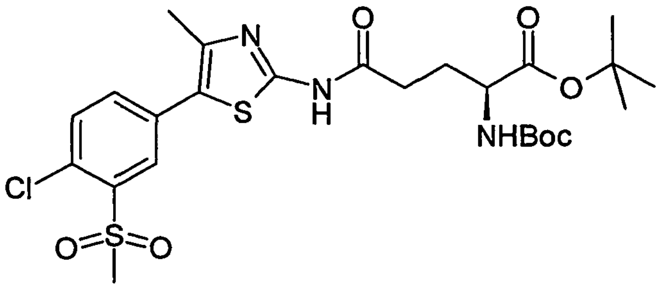

This compound was prepared from 2-tert-butoxycarbonylamino-pentanedioic acid 1-tert-butyl ester and 5-(4-chloro-3-methanesulfonyl-phenyl)-4-methyl-thiazol-2-ylamine (Stage 4 product) following the procedure described for the synthesis of compound (21).

Stage 6 - Synthesis of (S)-2-Amino-4-[5-(4-chloro-3-methanesulfonyl-phenyl)-4-methyl-thiazol-2-ylcarbamoyl]-butyric acid tert-butyl ester **(23)**

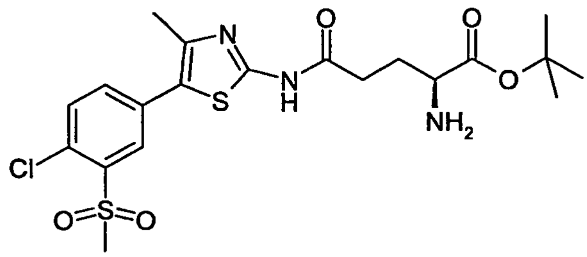

To a solution of Stage 5 product (50mg, 0.085mmol) in EtOAc (0.25ml) was added 2M HCl / ether solution (0.25ml) at r. t. The reaction mixture was vigorously stirred for 4 h. The reaction was re-treated with a mixture of EtOAc (0.25ml) and 2M HCl / ether (0.25ml). Stirring was continued for 1 h. The precipitate formed was collected by filtration under gravity, partitioned between EtOAc (3ml) and sat. aq. $NaHCO_3$ (0.5ml). The EtOAc layer was washed with water (1ml), dried ($Na_2SO_4$), filtered and concentrated *in vacuo* to give compound (23) (6.5mg, 16%). LCMS purity 95%, m/z 488/490 [$M^+$+H], $^1$H NMR (400 MHz, MeOD), $\delta$: 1.35-1.40 (9H, s), 1.80-2.05 (2H, m), 2.30 (3H, s), 2.45-2.55 (2H, m), 3.25 (3H, s), 3.30-3.35 (1H, m), 7.60-7.70 (2H, m), 8.05 (1H, s).

**Biological Assays**

**Histone deacetylase inhibitory activity assay**

The ability of compounds to inhibit histone deacetylase activities was measured using the commercially available HDAC fluorescent activity assay from Biomol. In brief, the *Fluor de Lys*™substrate, a lysine with an epsilon-amino acetylation, is incubated with the source of histone deacetylase activity (HeLa cell nuclear extract) in the presence or absence of inhibitor. Deacetylation of the substrate sensitises the substrate to *Fluor de Lys*™ developer, which generates a fluorophore. Thus, incubation of the substrate with a source of HDAC activity results in an increase in signal that is diminished in the presence of an HDAC inhibitor.

Data are expressed as a percentage of the control, measured in the absence of inhibitor, with background signal being subtracted from all samples, as follows:-

$$\% \text{ activity} = ((S^i - B) / (S^o - B)) \times 100$$

where $S^i$ is the signal in the presence of substrate, enzyme and inhibitor, $S^o$ is the signal in the presence of substrate, enzyme and the vehicle in which the inhibitor is dissolved, and B is the background signal measured in the absence of enzyme.

IC50 values were determined by non-linear regression analysis, after fitting the results of eight data points to the equation for sigmoidal dose response with variable slope (% activity against log concentration of compound), using Graphpad Prism software.

Histone deacetylase activity from crude nuclear extract derived from HeLa cells was used for screening. The preparation, purchased from 4C (Seneffe, Belgium), was prepared from HeLa cells harvested whilst in exponential growth phase. The nuclear extract was prepared according to Dignam JD 1983 Nucl. Acid. Res. 11, 1475-1489, snap frozen in liquid nitrogen and stored at -80°C. The final buffer composition was 20 mM Hepes, 100 mM KCl, 0.2 mM EDTA, 0.5 mM DTT, 0.2 mM PMSF and 20 % (v/v) glycerol.

**Aurora Kinase A Inhibitory Activity Assay**

The ability of compounds to inhibit aurora kinase A activity was measured using a microplate assay. In brief, 96-well Flashplates ® (PerkinElmer Life Sciences) were precoated with myelin basic protein (MBP). MBP (100ul of 100 mg/ml in PBS) was incubated at 37°C for 1 h, followed by overnight incubation at 4°C. Plates were then washed with PBS and allowed to air dry.

To determine the activity of aurora kinase A, 40 ng enzyme (ProQuinase: recombinant, full length human aurora kinase A, N-terminally fused to GST and expressed by baculovirus in Sf21 insect cells) was incubated in assay buffer (50 mM Tris (pH7.5), 10 mM NaCl, 2.5 mM $MgCl_2$, 1 mM DTT, 0.4 % DMSO), 10 μM ATP (Km of the enzyme) and 0.5 μCi [γ-$^{33}$P]-ATP and with varying concentrations of inhibitor. Wells lacking inhibitor were used as vehicle controls and wells containing no enzyme were used to measure the 'background' signal. Plates were incubated overnight at 30°C. After incubation the contents of the wells were removed and the plates washed three times with PBS containing 10 mM tetra sodium pyrophosphate prior to scintillation counting using a Wallac MicroBeta TriLux.
Dose response curves were generated from 10 concentrations (top final concentration 10 μM, with 3-fold dilutions), using triplicate wells.
IC50 values were determined by non-linear regression analysis, after fitting the data point results to the equation for sigmoidal dose response with variable slope (% activity against log concentration of compound), using Xlfit software.

## Dihydrofolate Reductase (DHFR) Inhibitory Activity Assay

The ability of compounds to inhibit DHFR activity was measured in an assay based on the ability of DHFR to catalyse the reversible NADPH-dependent reduction of dihydrofolic acid to tetrahydrofolic acid using a Sigma kit (Catalogue number CS0340). This uses proprietary assay buffer and recombinant human DHFR at 7.5 x$10^{-4}$ Unit per reaction, NADPH at 60 μM and dihydrofolic acid at 50 μM. The reaction was followed by monitoring the decrease in absorbance at 340 nm, for a 2 minute period, at room temperature, and the enzyme activity was calculated as the rate of decrease in absorbance. Enzyme activity, in the presence of inhibitor, was expressed as a percentage of inhibitor-free activity and the inhibitor IC50 was determined from a sigmoidal dose response curve using Xlfit software (% activity against log concentration of compound). Each sample was run in triplicate and each dose response curve was composed of 10 dilutions of the inhibitor.

## P38 MAP Kinase α Inhibitory Activity Assay

The ability of compounds to inhibit p38 MAP kinase α (full length human enzyme expressed in E coli as an N-terminally GST-tagged protein) activity was measured in an assay performed by Upstate (Dundee UK). In a final reaction volume of 25 μl, p38 MAP kinase α (5-10 mU) was incubated with 25 mM Tris pH 7.5, 0.02 mM EGTA, 0.33 mg/ml myelin basic protein, 10 mM magnesium acetate, ATP 90 μM (Km 97 μM) and [γ$^{33}$P]-ATP (specific activity approx. 500 cpm/pmol). The reaction was initiated by the addition of the MgATP mix. After incubation for 40 minutes at room temperature, the reaction was stopped by the addition of 5 μl of a 3 % phosphoric acid solution. 10 μl of the reaction was then spotted onto a P30 filtermat and washed three times for 5 minutes in 75 mM phosphoric acid and once in methanol, prior to drying and scintillation counting.
Dose response curves were generated from a ½ log dilution series of a stock inhibitor solution in DMSO. Nine dilutions steps were made from a top, final concentration of 10 μM, and a 'no compound' blank was included. Samples were run in duplicate. Data from scintillation counts were collected and subjected to free-fit analysis by Graphpad Prism software. From the curve generated, the concentration giving 50 % inhibition was determined.

## PI 3-Kinase γ Inhibition Assay

The measurement of PI 3-kinase γ activity is dependent upon the specific and high affinity binding of the GRP1 pleckstrin homology (PH) domain to PIP3, the product of PI 3-kinase activity. A complex is formed between europium-labelled anti-GST monoclonal antibody, a GST-tagged GRP1 PH domain, biotinylated PIP3 and streptavidin-allophycocyanin(APC). This complex generates a stable time-resolved fluorescence resonance energy transfer (FRET) signal, which is diminished by competition of PIP3, generated in the PI 3-kinase assay, with the biotinylated PIP3.
The assay was performed at Upstate (Dundee, UK) as follows: in a final reaction volume of 20 μl, PI 3-kinase γ (recombinant N-terminally His6-tagged, full length human enzyme, expressed by baculovirus in Sf21 insect cells) was incubated in assay buffer containing 10 μM phosphatidylinositol-4,5-bisphosphate and 100 μM MgATP (Km of the enzyme 117 μM). The reaction was initiated by the addition of the MgATP mix. After incubation for 30 minutes at room temperature, the reaction was stopped by the addition of 5 μl of stop solution containing EDTA and biotinylated phosphatidylinositol-3,4,5-trisphosphate. Finally, 5 μl of detection buffer was added, which contained europium-labelled anti-GST monoclonal antibody, GST-tagged GRP1 PH domain and streptavidin-APC. The plate was then read in time-resolved fluorescence mode and the homogenous time-resolved fluorescence (HTRF) signal was determined according to the formula HTRF =10000 x (Em665nm/Em620nm).
Duplicate data points were generated from a ½ log dilution series of a stock solution of compound in DMSO. Nine dilutions steps were made from a top final concentration of 10 μM, and a 'no compound' blank was included. HTRF ratio data were transformed into % activity of controls and analysed with a four parameter sigmoidal dose-response

(variable slope) application. The concentration giving 50 % inhibition (IC50) was determined.

**Cell Proliferation inhibition Assay**

Cancer cell lines (U937and HCT 116) growing in log phase were harvested and seeded at 1000 - 2000 cells/well (100 μl final volume) into 96-well tissue culture plates. Following 24 h of growth cells were treated with compound. Plates were then re-incubated for a further 72 - 96 h before a WST-1 cell viability assay was conducted according to the suppliers (Roche Applied Science) instructions.

Data were expressed as a percentage inhibition of the control, measured in the absence of inhibitor, as follows:-

$$\% \text{ inhibition} = 100-((S^i/S^o)x100)$$

where $S^i$ is the signal in the presence of inhibitor and $S^o$ is the signal in the presence of DMSO.

Dose response curves were generated from 8 concentrations (top final concentration 10 μM, with 3-fold dilutions), using 6 replicates.

IC50 values were determined by non-linear regression analysis, after fitting the results to the equation for sigmoidal dose response with variable slope (% activity against log concentration of compound), using Graphpad Prism software.

**LPS-stimulation of human whole blood**

Whole blood was taken by venous puncture using heparinised vacutainers (Becton Dickinson) and diluted in an equal volume of RPMI1640 tissue culture media. 100 μl was plated in V-bottomed 96 well tissue culture plates. Inhibitor was added in 100 μl of RPMI1640 media, and 2 h later the blood was stimulated with LPS (*E coli* strain 005: B5, Sigma) at a final concentration of 100 ng/ml and incubated at 37°C in 5 % $CO_2$ for 6 h. TNF-α levels were measured from cell-free supernatants by sandwich ELISA (R&D Systems #QTA00B).

**Broken Cell Carboxylesterase Assay**

*Preparation of cell extract*

U937 or HCT 116 tumour cells (- $10^9$) were washed in 4 volumes of Dulbeccos PBS (~ 1 litre) and pelleted at 525 g for 10 min at 4°C. This was repeated twice and the final cell pellet was resuspended in 35 ml of cold homogenising buffer (Trizma 10 mM, NaCl 130 mM, $CaCl_2$ 0.5 mM pH 7.0 at 25°C). Homogenates were prepared by nitrogen cavitation (700 psi for 50 min at 4°C). The homogenate was kept on ice and supplemented with a cocktail of inhibitors at final concentrations of:

Leupeptin 1 μM
Aprotinin 0.1 μM
E64 8 μM
Pepstatin 1.5 μM
Bestatin 162 μM
Chymostatin 33 μM

After clarification of the cell homogenate by centrifugation at 525 g for 10 min, the resulting supernatant was used as a source of esterase activity and was stored at -80°C until required.

***Measurement* of *ester cleavage***

Hydrolysis of esters to the corresponding carboxylic acids can be measured using the cell extract, prepared as above. To this effect cell extract (-30 μg / total assay volume of 0.5 ml) was incubated at 37°C in a Tris- HCl 25 mM, 125 mM NaCl buffer, pH 7.5 at 25°C. At zero time the ester (substrate) was then added at a final concentration of 2.5 μM and the samples were incubated at 37°C for the appropriate time (usually 0 or 80 min). Reactions were stopped by the addition of 3 x volumes of acetonitrile. For zero time samples the acetonitrile was added prior to the ester compound. After centrifugation at 12000 g for 5 min, samples were analysed for the ester and its corresponding carboxylic acid at room temperature by LCMS (Sciex API 3000, HP1100 binary pump, CTC PAL). Chromatography was based on an

AceCN (75*2.1mm) column and a mobile phase of 5-95 % acetonitrile in water /0.1 % formic acid.

**Quantification of hCE-1, hCE-2 and hCE-3 expression in monocytic and non-monocytic cell lines**

Gene-specific primers were used to PCR-amplify hCE-1, -2 and -3 from human cDNA. PCR products were cloned into a plasmid vector and sequence-verified. They were then serially diluted for use as standard curves in real-time PCR reactions. Total RNA was extracted from various human cell lines and cDNA prepared. To quantitate absolute levels of hCE's in the cell lines, gene expression levels were compared to the cloned PCR product standards in a real-time SYBR Green PCR assay. Figure 1 shows that hCE-1 is only expressed to a significant amount in a monocytic cell line.

**Biological Results**

The compounds referred to in Examples 1-5 above were investigated in the enzyme inhibition, cell proliferation and ester cleavage assays described above and the results are shown in Tables 3 and 4.

**Potency**

**Table 3**

| **HDAC** | Enzyme inhibition IC50 nM (HDAC - Hela cell nuclear extract) | Cell proliferation IC50 nM (U937 cells) | Ratio IC50 cell/enzyme |
|---|---|---|---|
| Unmodified Modulator **Compound (7)** (SAHA) | 100 | 400 | 4 |
| Modified Modulator **Compound (8)** (cyclopentyl ester) | 100 | 50 | 0.5 |
| Acid resulting from ester cleavage of Modified Modulator **Compound (9)** | 70 | Inactive | NA |
| Modified Modulator **Compound (10)** (t-butyl ester) | 130 | 1300 | 10 |

The above results show that:

(i) the amino acid ester modified compounds (Compounds 8 and 10) and the acid (Compound 9) which would result from cleavage of the ester motif, have IC50s in the enzyme assay comparable to the value for the unmodified HDAC inhibitor (SAHA - Compound 7) indicating that the alpha amino acid ester motif was attached to SAHA at a point which did not disrupt its binding mode.

(ii) even though the esters (Compounds 8 and 10) and acid (Compound 9) have comparable activities to the unmodified inhibitor (SAHA - Compound 7) there is a significant increase in the cellular potency of the esterase cleavable cyclopentyl ester (Compound 8) over the unmodified inhibitor (Compound 7) but a substantial decrease in cellular potency in the case of the esterase stable t-butyl ester (Compound 10), indicating that the latter did not accumulate the acid in cells to generate increased cellular potency.

(iii) the greater activity in the cell proliferation assay for Compound 8 over the unmodified counterpart, Compound 7 (or the non-hydrolysable ester derivative, Compound 10), indicates that the ester is hydrolysed to the parent acid Compound 9 in the cell where it accumulates and exerts a greater inhibitory effect.

**Table 3 continued**

| **Aurora kinase** | Enzyme inhibition IC50 nM (Aurora kinase A) | Cell proliferation IC50 nM (U937 cells) | Ratio IC50 cell/enzyme |
|---|---|---|---|
| Unmodified modulator **Compound(11)** | 350 | 430 | 1.3 |
| Modified Modulator **Compound (12)** (cyclopentyl ester) | 2300 | 3.5 | 0.0015 |
| Acid resulting from ester cleavage of Modified Modulator **Compound (13)** | 500 | >5000 | NA |
| Modified Modulator **Compound (14)** (t-butyl ester) | 3000 | 75 | 0.025 |

The above results show that:

(i) the alpha amino acid modified inhibitor, Compound 13, which would result from cleavage of the ester motif in Compound 12, has an IC50 value in the enzyme assay comparable to that of the unmodified aurora kinase inhibitor (Compound 11) indicating that it is possible to attach the alpha amino acid ester motif at a point which does not disrupt the binding to aurora kinase A.

(ii) even though the acid (Compound 13) has a comparable enzyme activity to the unmodified inhibitor (Compound 11) and the ester (Compound 12) is a weaker inhibitor, there is a significant increase in the cellular potency of Compound 12 over the unmodified inhibitor (Compound 11). The less readily cleaved t-butyl ester (Compound 14) has a comparable enzyme activity to the cleavable cyclopentyl ester (Compound 12) but is some 20 fold less active in the cell assay

(iii) the greater activity in the cell proliferation assay of Compound 12 over both the unmodified counterpart (Compound 11) and the less readily cleaved t-butyl ester (compound 14) indicates that the cyclopentyl ester is hydrolysed to the parent acid in the cell, where it accumulates, and exerts greater inhibitory effect.

**Table 3 continued**

| **P38 MAP kinase** | Enzyme inhibition IC50 nM (P38 MAP kinase) | Inhibition of TNF$\alpha$ production in human whole blood IC50 nM | Ratio IC50 WB/enzyme |
|---|---|---|---|
| Unmodified Modulator **Compound (15)** | 50 | 300 | 6 |
| Modified Modulator **Compound (16)** (cyclopentyl ester) | 25 | 20 | 0.8 |
| Acid resulting from ester cleavage of Modified Modulator Compound **(17)** | 30 | not tested | NA |
| Modified Modulator **Compound (18)** (t-butyl ester) | 40 | 750 | 18 |

The above results show that:

(i) the alpha amino acid modified inhibitor Compound 16, and the acid Compound 17 which would result from cleavage of the ester motif in Compound 16, have IC50 values in the enzyme assay comparable to the value for the unmodified P38 MAP kinase inhibitor (Compound 15) indicating that it is possible to attach the alpha amino acid

ester motif at a point which does not disrupt the binding to P38 MAP kinase.
(ii) the acid, Compound 17, has comparable activity against the enzyme to the unmodified inhibitor (Compound 15) and to the t-butyl ester (Compound 18). However there is a significant increase in the ability of the cyclopentyl ester (Compound 16) to inhibit TNF production inside monocytic cells present in whole blood compared to the unmodified inhibitor (Compound 15) and the less readily cleaved t-butyl ester (Compound 18).
(iii) the greater activity in the whole blood assay for Compound 16 over the unmodified counterpart Compound 15 and the less readily cleaved t-butyl ester Compound 18 indicates that the cyclopentyl ester is hydrolysed to the parent acid in the cell, where it accumulates, and exerts a greater inhibitory effect.

**Table 3 continued**

| **DHFR** | Enzyme inhibition IC50 nM (DHFR) | Cell proliferation IC50 nM (U937 cells) | Ratio IC50 cell/enzyme |
|---|---|---|---|
| Unmodified Modulator **Compound (2 G=N)** | 10 | 2200 | 220 |
| Modified Modulator **Compound (6)** (cyclopentyl ester) | 1700 | 23 | 0.013 |
| Acid resulting from ester cleavage of Modified Modulator **Compound (19)** | 10 | not tested | Not applicable |

The above results show that:

(i) the alpha amino acid modified inhibitor, Compound 19, which would result from cleavage of the ester motif in Compound 6, has an IC50 value in the enzyme assay comparable to that of the unmodified DHFR inhibitor (Compound 2 (G=N)) indicating that it is possible to attach the alpha amino acid ester motif at a point which does not disrupt the binding to DHFR.
(ii) even though the acid (Compound 19) has a comparable enzyme inhibitory activity to the unmodified inhibitor (Compound 2 (G=N)), the ester (Compound 6) is significantly more potent in inhibiting cell proliferation than the unmodified inhibitor (Compound 2 (G=N)).
(iii) the greater activity in the cell proliferation assay of Compound 6 over the unmodified counterpart (Compound 2 (G=N)) indicates that the cyclopentyl ester is hydrolysed to the parent acid in the cell, where it accumulates, and exerts greater inhibitory effect.

**Table 3 continued**

| **PI 3-Kinase** | Enzyme inhibition IC50 nM (PI3-Kinase) | Inhibition of TNF$\alpha$ production in human whole blood IC50 nM | Ratio IC50 WB/enzyme |
|---|---|---|---|
| Unmodified Modulator **Compound (20)** | 500 | 8500 | 17 |
| Modified Modulator **Compound (21)** (cyclopentyl ester) | 2700 | 400 | 0.15 |
| Acid resulting from ester cleavage of Modified Modulator **(22)** | 3600 | Not tested | not applicable |
| Modified Modulator **Compound (23)** (t-butyl ester) | 7100 | 5200 | 0.75 |

The above results show that:

(i) the alpha amino acid ester modified inhibitor, Compound 21, and the acid, Compound 22, which would result from cleavage of the ester motif in Compound 21, have IC50 values in the enzyme assay within a factor of 10 of the value for the unmodified PI 3-kinase inhibitor (Compound 20), indicating that it is possible to attach the alpha amino acid ester motif at a point which still retains reasonable binding to PI 3-kinase.

(ii) although the acid, Compound 22, has comparable activity to the unmodified inhibitor (Compound 20) and the ester (Compound 21), there is a significant increase in the potency of the ester to inhibit TNF production in monocytic cells present in whole blood compared to the unmodified inhibitor (Compound 20) and the less readily cleaved t-butyl ester (Compound 23).

(iii) the greater activity in the whole blood assay for Compound 21 over the unmodified counterpart Compound 20 and the less readily cleaved t-butyl ester Compound 23 indicates that the cyclopentyl ester is hydrolysed to the parent acid in the cell, where it accumulates, and exerts a greater inhibitory effect.

**Selectivity**

**Table 4: Comparison of cell proliferation and ester cleavage for a monocytic and a non monocytic cell line.**

| **HDAC** | **U937 (Monocytic cell line)** | | | **HCT116 (non-monocytic cell line)** | | |
|---|---|---|---|---|---|---|
| Compound | Cell proliferation IC50 nM | | Acid produced[1] ng/ml | Cell proliferation IC50 nM | | Acid produced[1] ng/ml |
| Unmodified Modulator **Compound (7)** | 400 | | Not applicable | 700 | | Not applicable |
| Modified Modulator **Compound (24)** | 60 | | 110 | 2100 | | 1 |
| [1] The amount of acid produced after incubation of the modified compound (Compound 24) for 80 min in the broken cell carboxylesterase assay described above. | | | | | | |

The above results show:

i) that the unmodified compound (compound 7) shows no selectivity between a monocytic and non-monocytic cell line whereas this can be achieved by attaching an appropriate ester motif, as in Compound 24.

ii) this selectivity correlates with the improved cleavage of the ester to the acid by the monocytic cell line.

iii) the improved cellular activity is only seen in the cell line where acid is produced indicating that this improvement in cellular potency is due to accumulation of the acid.

**Table 4 continued**

| **Aurora Kinase A** | **U937 (Monocytic cell line)** | | | **HCT116 (non-monocytic cell line)** | | |
|---|---|---|---|---|---|---|
| Compound | Cell proliferation IC50 nM | | Acid produced[1] ng/ml | Cell proliferation IC50 nM | | Acid produced[1] ng/ml |
| Unmodified Modulator **Compound (10)** | 430 | | NA | 560 | | NA |
| Modified Modulator **Compound (25)** | 1900 | | 50 | 6100 | | 0 |
| [1] The amount of acid produced after incubation of the compound (25) for 80 minutes in the broken cell carboxylesterase assay described above. | | | | | | |

The above results show:

i) that the unmodified compound (compound (10) shows no selectivity between a monocytic and a non-monocytic cell line whereas this is achieved by attaching an appropriate ester motif, as in Compound 25.

ii) this selectivity correlates with the improved cleavage of the ester to the acid by the monocytic cell line.

iii) the improved cellular activity is only seen in the cell line where acid is produced indicating that this improvement in cellular potency is due to accumulation of the acid

**Table 4 continued**

| **DHFR** | **U937 (Monocytic cell line)** | | | **HCT116 (non-monocytic cell line)** | | |
|---|---|---|---|---|---|---|
| Compound | Cell proliferation IC50 nM | | Acid produced[1] ng/ml | Cell proliferation IC50 nM | | Acid produced[1] ng/ml |
| Unmodified Modulator **Compound (2 G=N)** | 2200 | | Not applicable | 1700 | | NA |
| Modified Modulator **Compound (5)** | 310 | | 210 | 6700 | | 2 |
| [1] The amount of acid produced after incubation of compound (5) for 80 min in the broken cell carboxylesterase assay described above. | | | | | | |

The above results show that:

(i) the unmodified compound (compound 2 G = N) shows no selectivity between a monocytic and non-monocytic cell lines whereas this can be achieved by attaching an appropriate ester motif as in compound 5.

(ii) this selectivity correlates with the improved cleavage of the ester to the acid by the monocytic cell line.

(iii) the improved cellular activity is only seen in the cell line where acid is produced indicating that this improvement in cellular potency is due to accumulation of the acid

## Claims

1. A covalent conjugate of an alpha amino acid ester and an inhibitor of the activity of the target intracellular enzyme histone deacetylase for use in a method of treatment of the human or animal body by therapy, wherein:

   the ester group of the conjugate is hydrolysable by one or more intracellular carboxylesterase enzymes to the corresponding acid;
   the nitrogen of the amino group of the amino acid ester is not linked directly to a carbonyl moiety, or is left unsubstituted; and
   the alpha amino acid ester is conjugated to the inhibitor at a position remote from the binding interface between the inhibitor and the histone deacetylase enzyme, wherein the position of conjugation is remote when the conjugate has a potency in a cellular activity assay at least as high as that of the unconjugated inhibitor in the same assay, which cellular activity assay is a cell proliferation inhibition assay carried out in U937 cancer cells.

2. A covalent conjugate of an alpha amino acid ester and an inhibitor of the activity of the target intracellular enzyme histone deacetylase for use in a method of treatment of the human or animal body by therapy, wherein:

   the ester group of the conjugate is hydrolysable by one or more intracellular carboxylesterase enzymes to the corresponding acid;
   the nitrogen of the amino group of the amino acid ester is not linked directly to a carbonyl moiety, or is left unsubstituted; and
   the alpha amino acid ester is conjugated to the inhibitor such that the binding mode of the conjugated inhibitor and the said corresponding acid to the histone deacetylase enzyme is the same as that of the unconjugated inhibitor, wherein the binding mode is the same when the conjugate has a potency in a cellular activity assay at least as high as that of the unconjugated inhibitor in the same assay, which cellular activity assay is a cell proliferation inhibition assay carried out in U937 cancer cells.

3. A covalent conjugate for use according to claim 1 or claim 2, which conjugate is for use in the treatment of a proliferative disorder.

4. A conjugate for use as claimed in claim 1 or claim 2 wherein the covalently conjugated alpha amino acid ester is not the C-terminal element of a dipeptide motif of the conjugated inhibitor.

5. A conjugate for use as claimed in any of the preceding claims wherein the inhibitor is one which binds non-covalently to the histone deacetylase enzyme.

6. A conjugate for use as claimed in any of the preceding claims wherein the alpha amino acid ester is covalently conjugated to the inhibitor through a linker radical.

7. A conjugate for use as claimed in any of the preceding claims wherein the alpha amino acid ester is conjugated to the inhibitor via the amino group of the amino acid ester.

8. A conjugate for use as claimed in any of claims 1 to 7 wherein the alpha amino acid ester is conjugated to the inhibitor via the alpha carbon of the amino acid ester.

9. A conjugate for use as claimed in any of the preceding claims wherein the ester is hydrolysable by cells containing one or more of the intracellular carboxylesterase enzymes hCE-1, hCE-2 and hCE-3 to the corresponding alpha amino acid.

10. A conjugate for use as claimed in any of claims 1 to 7 wherein the ester is hydrolysable by cells containing the intracellular carboxylesterase enzyme hCE-1 to the corresponding alpha amino acid and not by cells containing hCE-2 or hCE-3.

11. A conjugate for use as claimed in claim 10 wherein the nitrogen of the amino group of the amino acid ester is substituted but not directly linked to a carbonyl group.

12. A pharmaceutical composition for use in a method of treatment of the human or animal body by therapy, the composition comprising a conjugate as claimed in any of claims 1 to 11 and a pharmaceutically acceptable carrier.

**13.** A pharmaceutical composition for use as claimed in claim 12 which is adapted for topical administration and wherein in the conjugate (a) when the alpha amino acid ester is linked to the inhibitor via its amino group, the carbon adjacent to the alpha carbon of the alpha amino acid ester is monosubstituted, or (b) when the alpha amino acid ester is linked to the inhibitor via a carbon atom of the amino acid, the adjacent carbon atom to that carbon atom of the amino acid is unsubstituted.

**14.** An in vitro method of selectively increasing or prolonging the intracellular potency and/or residence time of an inhibitor of the activity of the target intracellular enzyme histone deacetylase in macrophages and/or monocytes relative to other cell types, comprising treating a mixed population of cells with a conjugate of the inhibitor as claimed in claim 11.

**15.** A method of identifying a covalent conjugate of an alpha amino acid ester of a given inhibitor of the activity of the target intracellular enzyme histone deacetylase, said conjugate having increased or prolonged cellular potency relative to the given inhibitor, which method comprises:

(i) identifying a position or positions on a given inhibitor of the activity of the target intracellular enzyme histone deacetylase, or on a plurality of given inhibitors of the activity of the target intracellular enzyme histone deacetylase sharing the same binding mode for the target histone deacetylase enzyme, said position or positions being remote from the binding interface between the inhibitor(s) and the target histone deacetylase enzyme;
(ii) covalently modifying the inhibitor(s) by attachment of an alpha amino acid ester radical, or a range of different alpha amino acid ester radicals at one or more of the positions identified in (i);
(iii) testing the alpha amino acid-conjugated inhibitor(s) prepared in (ii) to determine their activity against the target histone deacetylase enzyme; and
(iv) from data acquired in (iii), selecting one or more of the tested alpha amino acid ester-conjugated versions of the given inhibitor(s) which cause modulation of histone deacetylase activity inside cells, are converted to and accumulate as the corresponding carboxylic acid inside cells, and which show increased or prolonged cellular potency

## Patentansprüche

**1.** Kovalentes Konjugat eines alpha-Aminosäureesters und eines Inhibitors der Aktivität des intrazellulären Zielenzyms Histon-Deacetylase zur Verwendung in einem Behandlungsverfahren des menschlichen oder tierischen Körpers durch Therapie, wobei:

die Estergruppe des Konjugates durch ein intrazelluläres oder mehrere intrazelluläre Carboxylesterase-Enzym (e) zu der korrespondierenden Säure hydrolysierbar ist;
der Stickstoff der Aminogruppe des Aminosäureesters nicht direkt mit einer Carbonylgruppe verknüpft ist oder unsubstituiert gelassen ist; und
der alpha-Aminosäureester an den Inhibitor an einer Position, die von der Bindungs-Kontaktfläche zwischen dem Inhibitor und dem Histon-Deacetylase-Enzym entfernt ist, konjugiert ist, wobei die Konjugationsposition entfernt ist, wenn das Konjugat in einem zellulären Aktivitätsassay eine Wirksamkeit hat, die mindestens so hoch ist wie die des unkonjugierten Inhibitors im gleichen Assay, wobei der zelluläre Aktivitätsassay ein Zellproliferations-Inhibitionsassay, der in U937 Krebszellen durchgeführt wird, ist.

**2.** Kovalentes Konjugat eines alpha-Aminosäureesters und eines Inhibitors der Aktivität des intrazellulären Zielenzyms Histon-Deacetylase zur Verwendung in einem Behandlungsverfahren des menschlichen oder tierischen Körpers durch Therapie, wobei:

die Estergruppe des Konjugates durch ein intrazelluläres oder mehrere intrazelluläre Carboxylesterase-Enzym (e) zu der korrespondierenden Säure hydrolysierbar ist;
der Stickstoff der Aminogruppe des Aminosäureesters nicht direkt mit einer Carbonylgruppe verknüpft ist oder unsubstituiert gelassen ist; und
der alpha-Aminosäureester so an den Inhibitor konjugiert ist, dass die Bindungsweise des konjugierten Inhibitors und der besagten korrespondierenden Säure an das Histon-Deacetylase-Enzym die selbe ist wie die des unkonjugierten Inhibitors, wobei die Bindungsweise die gleiche ist, wenn das Konjugat in einem zellulären Aktivitätsassay eine Wirksamkeit hat, die mindestens so hoch ist wie die des unkonjugierten Inhibitors im gleichen Assay, wobei der zelluläre Aktivitätsassay ein Zellproliferations-Inhibitionsassay, der in U937 Krebszellen ausgeführt wird, ist.

**3.** Kovalentes Konjugat zur Verwendung gemäß Anspruch 1 oder Anspruch 2, wobei das Konjugat zur Verwendung bei der Behandlung einer proliferativen Erkrankung ist.

**4.** Konjugat zur Verwendung, wie es in Anspruch 1 oder Anspruch 2 beansprucht wird, wobei der kovalent konjugierte alpha-Aminosäureester nicht das C-terminale Element eines Dipeptid-Motivs des konjugierten Inhibitors ist.

**5.** Konjugat zur Verwendung, wie es in einem der vorherigen Ansprüche beansprucht wird, wobei der Inhibitor einer ist, der nicht-kovalent an das Histon-Deacetylase-Enzym bindet.

**6.** Konjugat zur Verwendung, wie es in einem der vorherigen Ansprüche beansprucht wird, wobei der alpha-Aminosäureester kovalent an den Inhibitor über eine Linkergruppe konjugiert ist.

**7.** Konjugat zur Verwendung, wie es in einem der vorherigen Ansprüche beansprucht wird, wobei der alpha-Aminosäureester an den Inhibitor über die Aminogruppe des Aminosäureesters konjugiert ist.

**8.** Konjugat zur Verwendung, wie es in einem der Ansprüche 1 bis 7 beansprucht wird, wobei der alpha-Aminosäureester an den Inhibitor über den alpha-Kohlenstoff des Aminosäureesters konjugiert ist.

**9.** Konjugat zur Verwendung, wie es in einem der vorherigen Ansprüche beansprucht wird, wobei der Ester durch Zellen, die eines oder mehrere der intrazellulären Carboxylesterase-Enzyme hCE-1, hCE-2 und hCE-3 enthält/enthalten, zu der korrespondierenden alpha-Aminosäure hydrolysierbar ist.

**10.** Konjugat zur Verwendung, wie es in einem der Ansprüche 1 bis 7 beansprucht wird, wobei der Ester durch Zellen, die das intrazelluläre Carboxylesterase-Enzym hCE-1 enthalten, zu der korrespondierenden alpha-Aminosäure hydrolysierbar ist und nicht durch Zellen, die hCE-2 oder hCE-3 enthalten.

**11.** Konjugat zur Verwendung, wie es in Anspruch 10 beansprucht wird, wobei der Stickstoff der Aminogruppe des Aminosäureesters substituiert aber nicht direkt mit einer Carbonylgruppe verknüpft ist.

**12.** Pharmazeutische Zusammensetzung zur Verwendung in einem Behandlungsverfahren des menschlichen oder tierischen Körpers durch Therapie, wobei die Zusammensetzung ein Konjugat, wie es in einem der Ansprüche 1 bis 11 beansprucht wird, und einen pharmazeutisch verträglichen Träger umfasst.

**13.** Pharmazeutische Zusammensetzung zur Verwendung, wie es in Anspruch 12 beansprucht wird, welche zur topischen Verabreichung geeignet ist und wobei in dem Konjugat (a) wenn der alpha-Aminosäureester mit dem Inhibitor über seine Aminogruppe verknüpft ist, der zu dem alpha-Kohlenstoff des alpha-Aminosäureesters benachbarte Kohlenstoff monosubstituiert ist, oder (b) wenn der alpha-Aminosäureester mit dem Inhibitor über ein Kohlenstoffatom der Aminosäure verknüpft ist, das Kohlenstoffatom benachbart zu dem Kohlenstoffatom der Aminosäure unsubstituiert ist.

**14.** *In* vitro-Verfahren zur selektiven Erhöhung oder Verlängerung der intrazellulären Wirksamkeit und/oder Verweilzeit eines Inhibitors der Aktivität des intrazellulären Zielenzyms Histon-Deacetylase in Makrophagen und/oder Monozyten relativ zu anderen Zelltypen, umfassend Behandeln einer gemischten Zellpopulation mit einem Konjugat des Inhibitors, wie es in Anspruch 11 beansprucht wird.

**15.** Verfahren zum Identifizieren eines kovalenten Konjugates eines alpha-Aminosäureesters eines bestimmten Inhibitors der Aktivität des intrazellulären Zielenzyms Histon-Deacetylase, wobei besagtes Konjugat erhöhte oder verlängerte zelluläre Wirksamkeit relativ zu dem bestimmten Inhibitor hat, wobei das Verfahren umfasst:

(i) Identifizieren einer Position oder Positionen auf einem bestimmten Inhibitor der Aktivität des intrazellulären Zielenzyms Histon-Deacetylase oder auf einer Mehrzahl bestimmter Inhibitoren der Aktivität des intrazellulären Zielenzyms Histon-Deacetylase, welche/r die gleiche Bindungsweise an das Histon-Deacetylase-Zielenzym teilt/teilen, wobei besagte Position oder Positionen von der Bindungs-Kontaktfläche zwischen dem/den Inhibitor/en und dem Histon-Deacetylase-Zielenzym entfernt ist/sind;
(ii) kovalentes Modifizieren des/der Inhibitors/en durch Bindung einer alpha-Aminosäureester-Gruppe oder einer Reihe verschiedener alpha-Aminosäureester-Gruppen an eine oder mehrere der Positionen, welche in (i) identifiziert wurde/wurden;
(iii) Testen des/der in (ii) hergestellten alpha-Aminosäurekonjugierten Inhibitors/en, um seine/ihre Aktivität gegen

das Histon-Deacetylase-Zielenzym zu bestimmen; und
(iv) aus den Daten, welche in (iii) erfasst wurden, Auswählen einer oder mehrerer der getesteten alpha-Aminosäureester-konjugierten Version/en des/der bestimmten Inhibitors/en, welche eine Modulation der Histon-Deacetylase-Aktivität in Zellen erzeugt/erzeugen, in Zellen zu der korrespondierenden Carbonylsäure ungewandelt wird/werden und als diese akkumuliert/akkumulieren und welche eine erhöhte oder verlängerte zelluläre Wirksamkeit zeigt/zeigen.

**Revendications**

1. Conjugué covalent d'un ester d'acide alpha-aminé et d'un inhibiteur de l'activité de l'enzyme intracellulaire cible histone désacétylase, pour utilisation dans un procédé de traitement thérapeutique du corps d'un humain ou d'un animal, dans lequel conjugué :

- le groupe ester du conjugué peut être hydrolysé, par une ou plusieurs enzyme(s) carboxylesterase(s) intracellulaire(s), pour donner l'acide correspondant ;
- l'atome d'azote du groupe amino de l'ester d'acide aminé n'est pas lié directement à un groupe carbonyle, ou reste sans substituant ;
- et l'ester d'acide alpha-aminé est conjugué à l'inhibiteur en une position éloignée de l'interface de liaison entre l'inhibiteur et l'enzyme histone désacétylase, la position de conjugaison étant éloignée quand le conjugué présente, dans un test d'activité cellulaire, une efficacité au moins aussi élevée que celle de l'inhibiteur non-conjugué dans le même test, lequel test d'activité cellulaire est un test d'inhibition de la prolifération cellulaire mené sur des cellules tumorales U937.

2. Conjugué covalent d'un ester d'acide alpha-aminé et d'un inhibiteur de l'activité de l'enzyme intracellulaire cible histone désacétylase, pour utilisation dans un procédé de traitement thérapeutique du corps d'un humain ou d'un animal, dans lequel conjugué :

- le groupe ester du conjugué peut être hydrolysé, par une ou plusieurs enzyme(s) carboxylesterase(s) intracellulaire(s), pour donner l'acide correspondant ;
- l'atome d'azote du groupe amino de l'ester d'acide aminé n'est pas relié directement à un groupe carbonyle, ou reste sans substituant ;
- et l'ester d'acide alpha-aminé est conjugué à l'inhibiteur de telle sorte que le mode de liaison à l'enzyme histone désacétylase de l'inhibiteur conjugué et dudit acide correspondant est le même que celui de l'inhibiteur non-conjugué, le mode de liaison étant le même quand le conjugué présente, dans un test d'activité cellulaire, une efficacité au moins aussi élevée que celle de l'inhibiteur non-conjugué dans le même test, lequel test d'activité cellulaire est un test d'inhibition de la prolifération cellulaire mené sur des cellules tumorales U937.

3. Conjugué covalent pour utilisation conforme à la revendication 1 ou 2, lequel conjugué est destiné à être utilisé dans le traitement d'une maladie proliférative.

4. Conjugué pour utilisation conforme à la revendication 1 ou 2, dans lequel l'ester d'acide alpha-aminé conjugué de manière covalente n'est pas l'élément C-terminal d'un motif dipeptide de l'inhibiteur conjugué.

5. Conjugué pour utilisation conforme à l'une des revendications précédentes, dans lequel l'inhibiteur est un inhibiteur qui se lie de manière non-covalente à l'enzyme histone désacétylase.

6. Conjugué pour utilisation conforme à l'une des revendications précédentes, dans lequel l'ester d'acide alpha-aminé est conjugué de manière covalente à l'inhibiteur, par l'intermédiaire d'un groupe de raccord.

7. Conjugué pour utilisation conforme à l'une des revendications précédentes, dans lequel l'ester d'acide alpha-aminé est conjugué à l'inhibiteur par l'intermédiaire du groupe amino de l'ester d'acide aminé.

8. Conjugué pour utilisation conforme à l'une des revendications 1 à 7, dans lequel l'ester d'acide alpha-aminé est conjugué à l'inhibiteur par l'intermédiaire de l'atome de carbone alpha de l'ester d'acide aminé.

9. Conjugué pour utilisation conforme à l'une des revendications précédentes, dans lequel l'ester peut être hydrolysé, par des cellules contenant l'une ou plusieurs des enzymes carboxylesterases intracellulaires hCE-1, hCE-2 et hCE-3, en l'acide alpha-aminé correspondant.

**10.** Conjugué pour utilisation conforme à l'une des revendications 1 à 7, dans lequel l'ester peut être hydrolysé en l'acide alpha-aminé correspondant par des cellules contenant l'enzyme carboxylesterase intracellulaire hCE-1, mais ne peut pas l'être par des cellules contenant l'enzyme hCE-2 ou hCE-3.

**11.** Conjugué pour utilisation conforme à la revendication 10, dans lequel l'atome d'azote du groupe amino de l'ester d'acide aminé porte un ou des substituant(s), mais n'est pas lié directement à un groupe carbonyle.

**12.** Composition pharmaceutique pour utilisation dans un procédé de traitement thérapeutique du corps d'un humain ou d'un animal, laquelle composition comprend un conjugué conforme à l'une des revendications 1 à 11 et un véhicule pharmacologiquement admissible.

**13.** Composition pharmaceutique pour utilisation conforme à la revendication 12, qui est adaptée pour être administrée en topique et dans laquelle, dans le conjugué,

a) si l'ester d'acide alpha-aminé est raccordé à l'inhibiteur par l'intermédiaire de son groupe amino, l'atome de carbone adjacent à l'atome de carbone alpha de l'ester d'acide alpha-aminé porte un et un seul substituant ;
b) ou bien, si l'ester d'acide alpha-aminé est raccordé à l'inhibiteur par l'intermédiaire d'un atome de carbone de l'acide aminé, l'atome de carbone adjacent à cet atome de carbone de l'acide aminé ne porte aucun substituant.

**14.** Procédé *in vitro* permettant d'accroître sélectivement l'activité intracellulaire et/ou de prolonger sélectivement le temps de séjour d'un inhibiteur de l'activité de l'enzyme intracellulaire cible histone désacétylase au sein de macrophages et/ou de monocytes, par rapport à des cellules d'autres types, lequel procédé comporte le fait de traiter une population mixte de cellules avec un conjugué d'inhibiteur conforme à la revendication 11.

**15.** Procédé permettant d'identifier un conjugué covalent d'un ester d'acide alpha-aminé et d'un inhibiteur donné de l'activité de l'enzyme intracellulaire cible histone désacétylase, lequel conjugué est doté d'une activité cellulaire accrue ou prolongée par rapport à celle de l'inhibiteur donné, lequel procédé comporte les étapes suivantes :

i) identifier une position ou des positions sur un inhibiteur donné de l'activité de l'enzyme intracellulaire cible histone désacétylase, ou sur plusieurs inhibiteurs donnés de l'activité de l'enzyme intracellulaire cible histone désacétylase qui partagent le même mode de liaison à l'enzyme cible histone désacétylase, ladite position ou lesdites positions étant éloignée(s) de l'interface de liaison entre l'inhibiteur ou les inhibiteurs et l'enzyme cible histone désacétylase ;
ii) modifier de manière covalente l'inhibiteur ou les inhibiteurs en lui ou leur attachant un groupe ester d'acide alpha-aminé ou une série de différents groupes ester d'acide alpha-aminé, en une ou plusieurs des positions identifiées dans l'étape (i) ;
iii) soumettre l'inhibiteur ou les inhibiteurs conjugué(s) avec un ou des ester(s) d'acide(s) alpha-aminé(s), préparé(s) dans l'étape (ii), à des tests pour déterminer leur activité contre l'enzyme cible histone désacétylase ;
iv) et sélectionner en partant des données acquises dans l'étape (iii), parmi les versions testées de l'inhibiteur ou des inhibiteurs donné(s) conjugué(s) avec un ou des ester(s) d'acide(s) alpha-aminé(s), une ou plusieurs de ces versions qui engendre(nt) une modulation de l'activité d'histone désacétylase à l'intérieur des cellules, qui est ou sont convertie(s) en le ou les acide(s) carboxylique(s) correspondant(s) à l'intérieur des cellules et s'y accumule(nt) sous cette forme, et qui fait ou font preuve d'une activité cellulaire accrue ou prolongée.

**Figure 1 showing the expression of human carboxylesterases in different cell lines.**

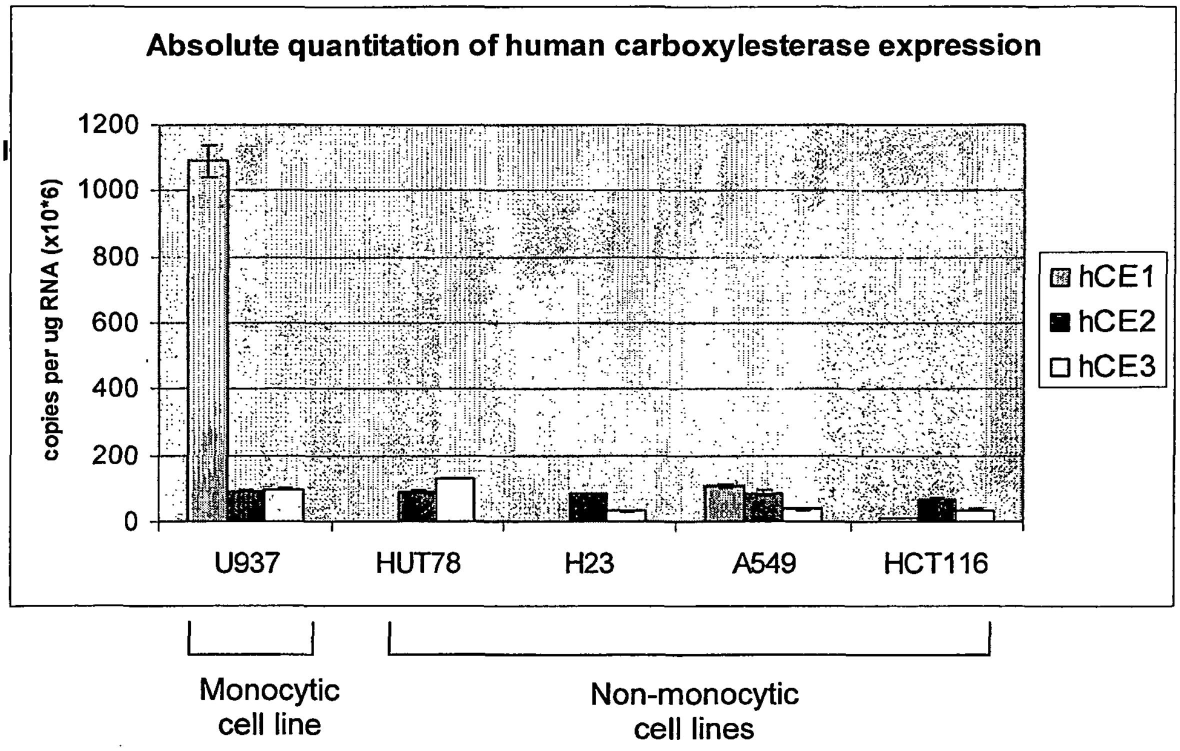

**Figure 2 showing (2 G = N) docked into DHFR and point of attachment of esterase motif**

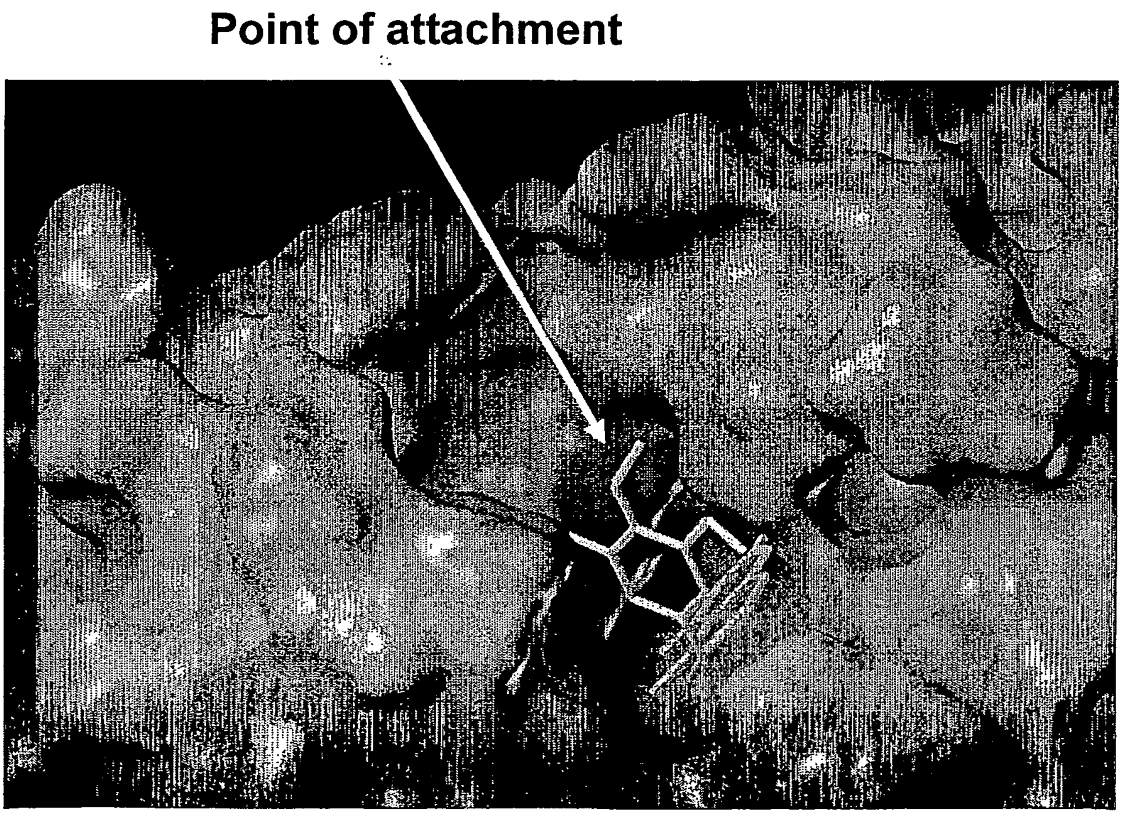

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 1345502 A
- US 6143764 A
- US 006143764 A
- WO 03076405 A
- WO 03072552 A


### Non-patent literature cited in the description


- *Drug Disc. Today,* 2005, vol. 10, 313-325
- **VAN ROON et al.** *Arthritis and Rheumatism,* 2003, 1229-1238
- **CONELL.** *N.Eng J. Med.,* 2004, vol. 350, 2591-2602
- **NALDINI ; CARRARO.** *Curr Drug Targets Inflamm Allergy,* 2005, 3-8
- **NAM et al.** *J Exp Med,* 2005, vol. 201, 441
- **ZHU et al.** *Structure,* 1999, vol. 7, 651
- **JIN et al.** *J Biol Chem,* 2004, vol. 279, 42818
- **HUAI et al.** *Biochemistry,* 2003, vol. 42, 13220
- **SCAPIN et al.** *Biochemistry,* 2004, vol. 43, 6091
- **INTCHAK et al.** *Cell,* 1996, vol. 85, 921
- **WANG et al.** *Structure,* 1998, vol. 6, 1117
- **KIEFER et al.** *J Biol Chem,* 2003, vol. 278, 45763
- **SCHUMACHER et al.** *J Mol Biol,* 2000, vol. 298, 875
- **CHANDRA et al.** *Biochemistry,* 2002, vol. B, 10914
- **ESSEN et al.** *Biochemistry,* 1997, vol. 36, 1704
- **LEIROS et al.** *J Mol Biol,* 2004, vol. 339, 805
- **ROSENFELD et al.** *Biochemistry,* 2002, vol. 41, 13915
- **RUDBERG et al.** *J Biol Chem,* 2004, vol. 279, 27376
- **OKAMATO et al.** *Chem Pharm Bull,* 1999, vol. 47, 11
- **BERTRAND et al.** *J Mol Biol,* 2003, vol. 333, 393
- **XU et al.** *JBC,* 2004, vol. 279, 50401
- **RUF et al.** *PNAS (USA,* 1996, vol. 93, 7481
- **SHEPPARD et al.** *Bioorg Med Chem Lett,* 2004, vol. 14, 865
- **BLEDSOE et al.** *Cell,* 2002, vol. 110, 93
- **WALKER et al.** *Mol Cell Biol,* 2000, vol. 6, 909
- **WAN et al.** *Cell,* 2004, vol. 116, 855
- **YANG et al.** *Nat Struct Biol,* 2002, vol. 9, 940
- **FINNIN et al.** *Nature,* 1999, vol. 401, 188
- **NAGAR et al.** *Cancer Res,* 2002, vol. 62, 4236
- **MUNSHI et al.** *Acta Crystallogr Sect D,* 2003, vol. 59, 1725
- **STOUT et al.** *Structure,* 1998, vol. 6, 839
- **KLEIN et al.** *J Mol Biol,* 1995, vol. 249, 153
- **KOELNER et al.** *J Mol Biol,* 1998, vol. 280, 153
- **HERNANDEZ-GUZMAN et al.** *J Biol Chem,* 2003, vol. 278, 22989
- **STAMOS et al.** *J Biol Chem,* 2002, vol. 277, 46265
- **LAMERS et al.** *J Mol Biol,* 1999, vol. 285, 713
- **MCTIGUE et al.** *Structure,* 1999, vol. 7, 319
- **HOUGH et al.** *J Mol Biol,* 1999, vol. 287, 579
- **ALMRUD et al.** *J Mol Biol,* 2000, vol. 295, 7
- **CLASSEN et al.** *PNAS (USA,* 2003, vol. 100, 10629
- **STAKER et al.** *PNAS (USA,* 2002, vol. 99, 15387
- **MATIAS et al.** *J Biol Chem,* 2000, vol. 275, 26164
- **HEO et al.** *EMBO J,* 2004, vol. 23, 2185
- **CURTIN et al.** *Bioorg Med Chem Lett,* 2003, vol. 13, 1367
- **DAVIS et al.** *Science,* 2001, vol. 291, 134
- **GARGARO et al.** *J Mol Biol,* 1998, vol. 277, 119
- **GRIFFITH et al.** *Mol Cell,* 2004, vol. 13, 169
- **STAMS et al.** *Protein Sci,* 1998, vol. 7, 556
- **NORMAN et al.** *Structure,* 2004, vol. 12, 75
- **DOBRITZSCH et al.** *JBC,* 2002, vol. 277, 13155
- **DEN ELSEN et al.** *EMBO J,* 2001, vol. 20, 3008
- **RANGANATHAN et al.** *Cell,* 1997, vol. 89, 875
- **EGEA et al.** *EMBO J,* 2000, vol. 19, 2592
- **JAIN et al.** *Nat Struct Biol,* 1996, vol. 3, 375
- **OAKLEY et al.** *J Mol Biol,* 1999, vol. 291, 913
- **JEZ et al.** *Chem Biol,* 2003, vol. 10, 361
- **CHANDRA et al.** *Biochemistry,* 2002, vol. 41, 10914

- **ANDERSEN et al.** *J Biol Chem,* 2000, vol. 275, 7101
- **KOMANDER et al.** *Biochem J,* 2003, vol. 375, 255
- **ISTVAN ; DEISENHOFER.** *Science,* 2001, vol. 292, 1160
- **LENHART et al.** *Chem Biol,* 2002, vol. 9, 639
- **MATTEVI et al.** *Science,* 1992, vol. 255, 1544
- **ZHANG et al.** *Nature,* 1997, vol. 386, 247
- **EBDURP et al.** *J Med Chem,* 2003, vol. 46, 1306
- **BAHNSON et al.** *PNAS USA,* 1997, vol. 94, 12797
- **WEI et al.** *Nat Struct Biol,* 1999, vol. 6, 340
- **MATHEWS et al.** *Biochemistry,* 1998, vol. 37, 15607
- **URZHMSEE.** *Structure,* 1997, vol. 5, 601
- **TOCCHINI-VATENTINI et al.** *PNAS USA,* 2001, vol. 98, 5491
- **LOUIS et al.** *Biochemistry,* 1998, vol. 37, 2105
- **BRESSANELLI et al.** *PNAS USA,* 1999, vol. 96, 13034
- **TAYLOR et al.** *J Med Chem,* 1998, vol. 41, 798
- **DAS et al.** *J Mol Biol,* 1996, vol. 264, 1085
- **KHAYAT et al.** *Biochemistry,* 2003, vol. 42, 885
- **CHAMPNESS et al.** *Proteins,* 1998, vol. 32, 350
- **MOLTENI et al.** *Acta Crystallogr Sect D,* 2001, vol. 57, 536
- **BERTINO J.** *J.Cin. Oncol.,* 1993, vol. 11, 5-14
- **CRONSTEIN N.** *Pharmacol. Rev.,* vol. 57, 163-1723
- **SALTER A.** *Rev. Infect. Dis.,* 1982, vol. 4, 196-236
- **PLOWE C.** *BMJ,* 2004, vol. 328, 545-548
- **GANGJEE et al.** *J.Med.Chem.,* 1993, vol. 36, 3437-3443
- **POLSHAKOV, V.I. et al.** *Protein Sci.,* 1999, vol. 8, 467-481
- *Org. Synth. Col.,* 1955, vol. 3, 272
- *J. Med. Chem.,* 1993, vol. 36, 3437-3443
- **DIGNAM JD.** *Nucl. Acid. Res.,* 1983, vol. 11, 1475-1489